# EUROPEAN PATENT SPECIFICATION

(11) **EP 2 994 542 B1**
(45) Date of publication and mention of the grant of the patent: **05.07.2023**
(21) Application number: 14794184.3
(22) Date of filing: 08.05.2014
(51) Int. Cl.: C12Q 1/68, G01N 33/573, G01N 33/566, C40B 30/04, G01N 33/574

(54) **COMPOSITIONS FOR OVARIAN CANCER ASSESSMENT HAVING IMPROVED SPECIFICITY**
ZUSAMMENSETZUNGEN ZUR OVARIALKARZINOMBEURTEILUNG MIT VERBESSERTER SPEZIFITÄT
COMPOSITIONS PRÉSENTANT UNE GRANDE SPÉCIFICITÉ POUR L'ÉVALUATION DU CANCER DE L'OVAIRE

(30) Priority: 10.05.2013 US 201361822197 P
(43) Date of publication of application: 16.03.2016
(73) Proprietor: Johns Hopkins University, Baltimore, MD 21218 (US); Aspira Women's Health Inc., Austin, TX 78738 (US)
(72) Inventor: MUNROE, Donald, Austin, TX 78738 (US); CHAN, Daniel, W., Clarksville, MD 21029 (US); ZHANG, Zhen, Baltimore, MD 21218 (US)
(74) Representative: Zwicker, Jörk
(86) International application number: PCT/US2014/037295
(87) International publication number: WO 2014/182896

(56) References cited:
- WO-A2-2012/054824
- WO-A2-2012/112685
- WO-A2-2012/115820
- US-A1- 2010 216 137
- Anonymous: "GeneChip Human Genome Arrays", , 23 November 2004 (2004-11-23), pages 1-10, XP055327123, Retrieved from the Internet: URL:http://www.osa.sunysb.edu/udmf/ArraySh eets/human_datasheet.pdf [retrieved on 2016-12-07]
- MACUKS RONALDS ET AL: "An ovarian cancer malignancy risk index composed of HE4, CA125, ultrasonographic score, and menopausal status: use in differentiation of ovarian cancers and benign lesions", TUMOR BIOLOGY, KARGER, BASEL, CH, vol. 33, no. 5, 14 June 2012 (2012-06-14), pages 1811-1817, XP035966971, ISSN: 1010-4283, DOI: 10.1007/S13277-012-0440-1 [retrieved on 2012-06-14]
- BRANDON J. D. REIN ET AL: "Potential Markers for Detection and Monitoring of Ovarian Cancer", JOURNAL OF ONCOLOGY, vol. 10, no. 2, 1 January 2011 (2011-01-01), pages 1897-17, XP055283396, US ISSN: 1687-8450, DOI: 10.1111/j.1471-0528.2007.01499.x
- NOSOV V ET AL: "Validation of serum biomarkers for detection of early-stage ovarian cancer", AMERICAN JOURNAL OF OBSTETRICS & GYNECOLOGY, MOSBY, ST LOUIS, MO, US, vol. 200, no. 6, 1 June 2009 (2009-06-01), pages 639.e1-639.e5, XP026142509, ISSN: 0002-9378, DOI: 10.1016/J.AJOG.2008.12.042 [retrieved on 2009-03-14]

## Description

### BACKGROUND OF THE INVENTION

Ovarian cancer is among the most lethal gynecologic malignancies in developed countries. Annually in the United States alone, approximately 23,000 women are diagnosed with the disease and almost 14,000 women die from it. Despite progress in cancer therapy, ovarian cancer mortality has remained virtually unchanged over the past two decades. Given the steep survival gradient relative to the stage at which the disease is diagnosed, early detection remains the most important factor in improving long-term survival of ovarian cancer patients. A second important factor is whether or not women with ovarian cancer are treated by a surgeon that specializes in gynecological oncology.

The importance of identifying women who should be treated by a gynecological oncologist is highlighted in a consensus statement issued by the National Institutes of Health (NIH). In 1994, the NIH indicated that women identified preoperatively as having a significant risk of ovarian cancer should have the option of having their surgery performed by a gynecologic oncologist. To ensure that no woman who has ovarian cancer is overlooked, current diagnostic methods optimize sensitivity at the expense of specificity.

Present diagnostic methods have an unacceptably high false positive rate. In human terms, this means that fifty percent of women go into surgery believing that they have ovarian cancer when in fact they have a benign mass. There is an urgent need for improved diagnostic methods that not only have a high degree of sensitivity, but that also provide a high degree of specificity, which can be used to manage subject treatment more effectively and ensure that the appropriate patients are being promptly and properly referred to specialists.

WO 2012/112685 A2 discloses diagnostic assays for detecting ovarian cancer or for pre-operatively distinguishing a benign pelvic mass from ovarian cancer using a biomarker panel comprising Transthyretin, Apolipoprotein A-I, beta 2-Microglobulin, Transferrin, Cancer Antigen 125 (CA125) and/or HE4.

Ronalds et al. (Tumor Biology, 2012, Karger, Basel, CH, Vol. 33(5): 1811-1817) discloses an ovarian cancer malignancy risk index composed of CA125 and HE4. These factors, in combination with ultrasonic features and menopausal status are described to have high accuracy in ovarian tumor differentiation. Nosov et al. (AJOG, 2009, 200 639.e1-639.e5) describe a marker panel including apolipoprotein A1, transferrin, transthyretin and CA125 for detection of ovarian cancer.

As shown below, the specific combination of markers CA125, Prealbumin, Transferrin and HE4 of the present invention leads to an unexpectedly superior specificity over the prior art without sacrificing sensitivity.

### SUMMARY OF THE INVENTION

The present invention provides in vitro uses and methods that provide a high degree of sensitivity and a high degree of specificity for the preoperative assessment of ovarian tumors in a variety of subjects (e.g., pre- and post-menopausal women) having a variety of ovarian cancer types (e.g., clear cell/mucinous, low malignant potential, high malignant potential) and at a variety of disease states (e.g., early and late stage). In particular, the present invention provides the in vitro use of a panel for pre-operatively assessing the risk of a subject having an adnexal mass as having ovarian cancer, the panel consisting of markers Cancer Antigen 125 (CA125), Prealbumin, Transferrin, and Human Epididymis Protein 4 (HE4).

According to a further aspect, the present invention provides an in vitro method for reducing the rate of false positive pre-operative ovarian cancer assessment in a subject having an adnexal mass, the method comprising:
(a) measuring the level of a panel of markers consisting of CA125, Prealbumin, Transferrin, and HE4 in a biological sample derived from the subject; and
(b) comparing the level of said markers to a reference level.

According to a further aspect, the present invention provides an in vitro method for preoperatively assessing the risk of a subject having an adnexal mass as having ovarian cancer, the method comprising:
(a) measuring the level of a panel of markers consisting of CA125, Prealbumin, Transferrin, and HE4 in a biological sample derived from the subject; and
(b) comparing the level of said markers to a reference level.

According to one example, at a fixed sensitivity of 90%, the specificity using the panel was at least about 78% and 80%, respectively.

According to a further example, the specificity is about 75%, 80%, 90%, or 95% when the sensitivity is set at about 85%, 90%, 95% or more.

In another embodiment,
the method further involves assessing clinical markers of ovarian cancer risk comprising age, pre-menopausal status, post-menopausal status, family history, physical examination, imaging results, and history of smoking;
the comparing comprises using a linear model or a non- linear model;
the method further involves detecting follicle-stimulating hormone (FSH); and/or
the detecting step is by immunoassay or affinity capture.

According to yet another embodiment, the method is carried out in a plate, chip, beads, microfluidic platform, membrane, planar microarray, or suspension array.

The measurement of the panel of biomarkers set forth herein in subject samples provides information that is used to characterize an adnexal mass (e.g., an ovarian tumor) and determine whether the subject should be referred to a gynecologic oncologist. In various embodiments, the markers are identified and quantified by immunoassay (e.g., ELISA).

Specifically, biomarker panels described herein may further comprise one or more of insulin-like growth factor binding protein 2 (IGFBP2), Interleukin 6 (IL6), Follicle-stimulating hormone (FSH), and/or Human Epididymis Protein 4 (HE4) polypeptides and fragments thereof as set forth in Table 1. Biomarker panels may further comprise one or more of CA-125-II, Transthyretin/prealbumin, Apolipoprotein A-1, β2-microglobulin, and/or Transferrin. According to the invention, the following particular combination of biomarkers was shown to be surprisingly effective in assessing ovarian malignancy of an adnexal mass:
CA125, Prealbumin, Transferrin, and HE4;

Other marker panels which are not part of the invention include:
CA125, Prealbumin, and HE4;
Insulin-like growth factor binding protein 2 (IGFBP2), Interleukin 6 (IL6), follicle-stimulating hormone (FSH), CA-125-II, and Transthyretin/prealbumin;
Insulin-like growth factor binding protein 2 (IGFBP2), Interleukin 6 (IL6), Human Epididymis Protein 4 (HE4), CA-125-II, and Transthyretin/prealbumin;
CA-125-II, Transthyretin/prealbumin, Transferrin, and Human Epididymis Protein 4 (HE4);
CA-125-II, Transthyretin/prealbumin, and Human Epididymis Protein 4 (HE4);
CA 125, Transthyretin/prealbumin, Apolipoprotein A1, β-2-microglobulin, and Transferrin, and Human Epididymis Protein 4 (HE4);
CA-125, HE4, IGFBP2, IL6, and TAG72/ CA72-4;
CA-125, APOA1, Transthyretin/prealbumin, B2M, TRF, HE4, IGFBP2, IL6, FSH, CA724;
CA125, APOA1, Transthyretin/prealbumin, B2M, TRF, HE4, IGFBP2, IL6, FSH, TAG 72/CA72-4.
CA125, HE4, IGFBP2, IL6, and CA724
CA125, Prealb, TRF, HE4, and CA 72-4;
CA125, HE4, CA724;
CA125, Prealb, TRF, and CA724; and
CA125, Prealb, IGFBP2, IL6, and CA724.

Use of these panels described herein unexpectedly increased specificity, increased sensitivity, and/or reduced the rate of false positives identified by conventional panels of biomarkers.

As described in detail herein, any method known in the art can be used to measure a panel of biomarkers. In aspects of the invention, the panel of biomarkers are measured using any immunoassay well known in the art. The immunoassay can be, but is not limited to, ELISA, western blotting, and radioimmunoassay.

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

A "biomarker" or "marker" as used herein generally refers to a protein, nucleic acid molecule, clinical indicator, or other analyte that is associated with a disease. A marker of ovarian cancer can be differentially present in a biological sample obtained from a subject having or at risk of developing ovarian cancer relative to a reference. A marker is differentially present if the mean or median level of the biomarker present in the sample is statistically different from the level present in a reference. A reference level may be, for example, the level present in a sample obtained from a healthy control subject or the level obtained from the subject at an earlier timepoint, i.e., prior to treatment. Common tests for statistical significance include, among others, t-test, ANOVA, Kruskal-Wallis, Wilcoxon, Mann-Whitney and odds ratio. Biomarkers, alone or in combination, provide measures of relative likelihood that a subject belongs to a phenotypic status of interest. The differential presence of a marker disclosed herein in a subject sample can be useful in characterizing the subject as having or at risk of developing ovarian cancer, for determining the prognosis of the subject, for evaluating therapeutic efficacy, or for selecting a treatment regimen (e.g., selecting that the subject be evaluated and/or treated by a surgeon that specializes in gynecologic oncology).

Markers useful in the panels disclosed herein include, for example, IGFBP2, IL6, FSH, HE4, CA125, transthyretin, transferrin, ApoA1, β2 microglobulin and CA72-4 proteins, as well as the nucleic acid molecules encoding such proteins. Fragments useful in the methods disclosed herein are sufficient to bind an antibody that specifically recognizes the protein from which the fragment is derived. Also disclosed are markers that are substantially identical to the following sequences. Preferably, such a sequence is at least 85%, 90%, 95% or even 99% identical at the amino acid level or nucleic acid to the sequence used for comparison.

By "Insulin-like growth factor binding protein (IGFBP2) polypeptide" is meant a polypeptide or fragment thereof having at least about 85% amino acid identity to NCBI Accession No. NP_000588.

By "Interleukin 6 (IL6) polypeptide" is meant a polypeptide or fragment thereof having at least about 85% amino acid identity to NCBI Accession No. NP_000591.

By "Follicle-stimulating hormone (FSH) polypeptide" is meant a polypeptide or fragment thereof having at least about 85% amino acid identity to NCBI Accession No. NP_000501.

By "Human Epididymis Protein 4 (HE4) polypeptide" is meant a polypeptide or fragment thereof having at least about 85% amino acid identity to NCBI Accession No. NP_006094.

By "Cancer Antigen 125 (CA 125) polypeptide" is meant a polypeptide or fragment thereof having at least about 85% amino acid identity to Swiss-Prot Accession number Q8WXI7.

By "Transthyretin (Prealbumin) polypeptide" is meant a polypeptide or fragment thereof having at least about 85% amino acid identity to Swiss Prot Accession number P02766.

By "Transferrin polypeptide" is meant a polypeptide or fragment thereof having at least about 85% amino acid identity to UniProtKB/TrEMBL Accession number Q06AH7.

By "Apolipoprotein A1 (ApoA1) polypeptide" is meant a polypeptide or fragment thereof having at least about 85% amino acid identity to Swiss Prot Accession number P02647.

By "β-2 microglobulin polypeptide" is meant a polypeptide or fragment thereof having at least about 85% amino acid identity to SwissProt Accession No. P61769.

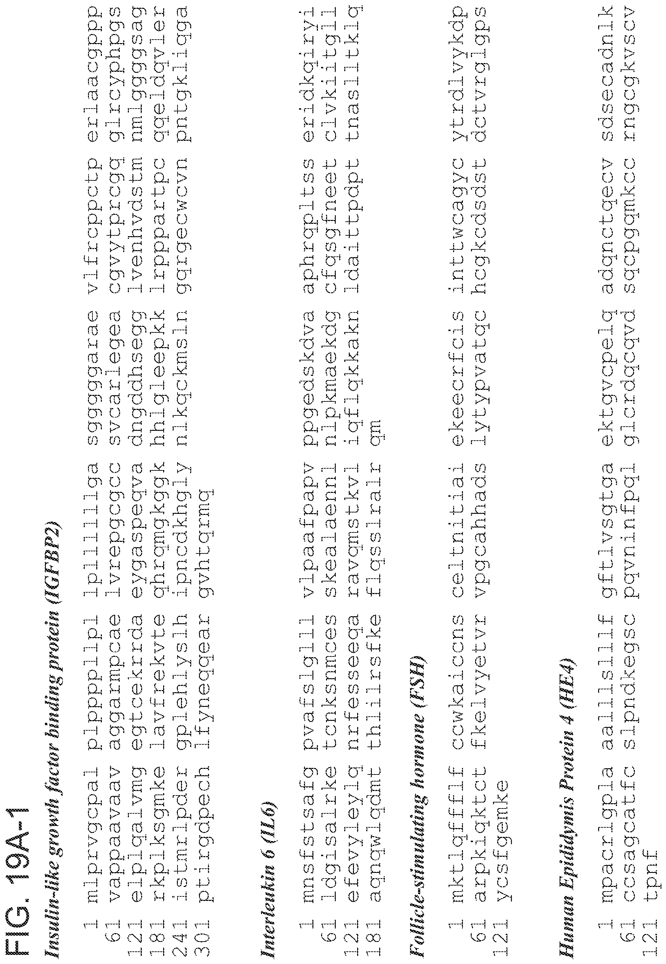

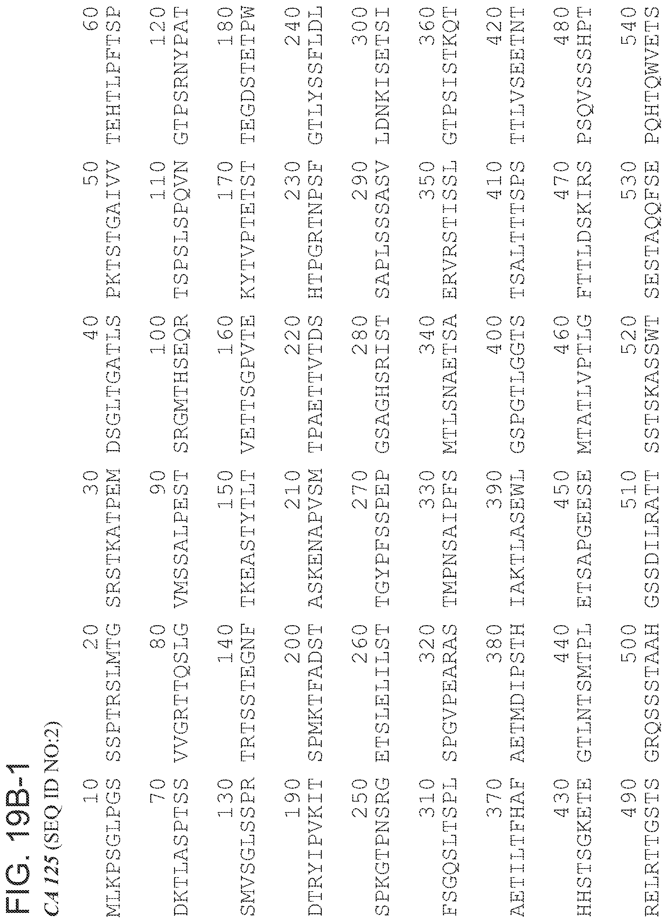

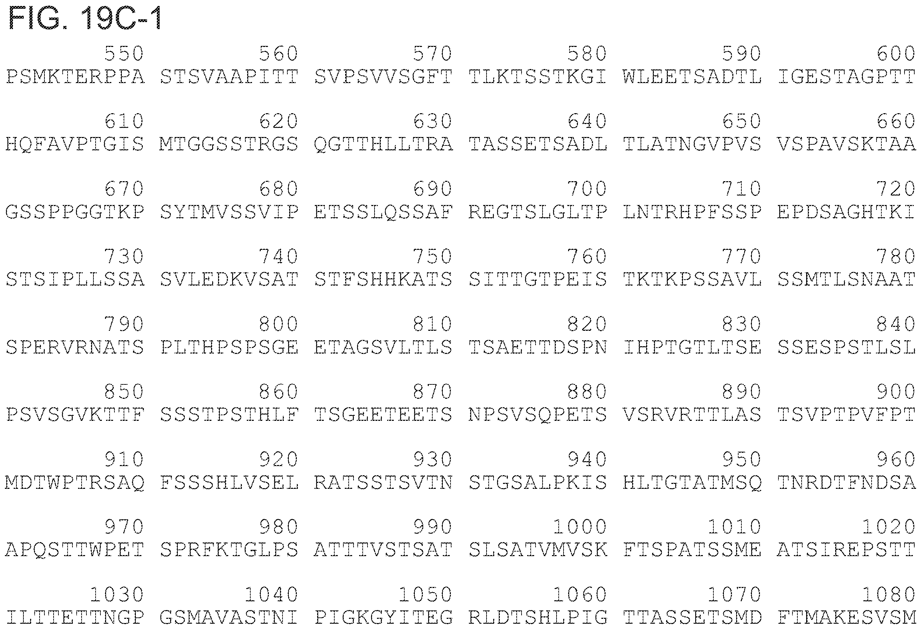

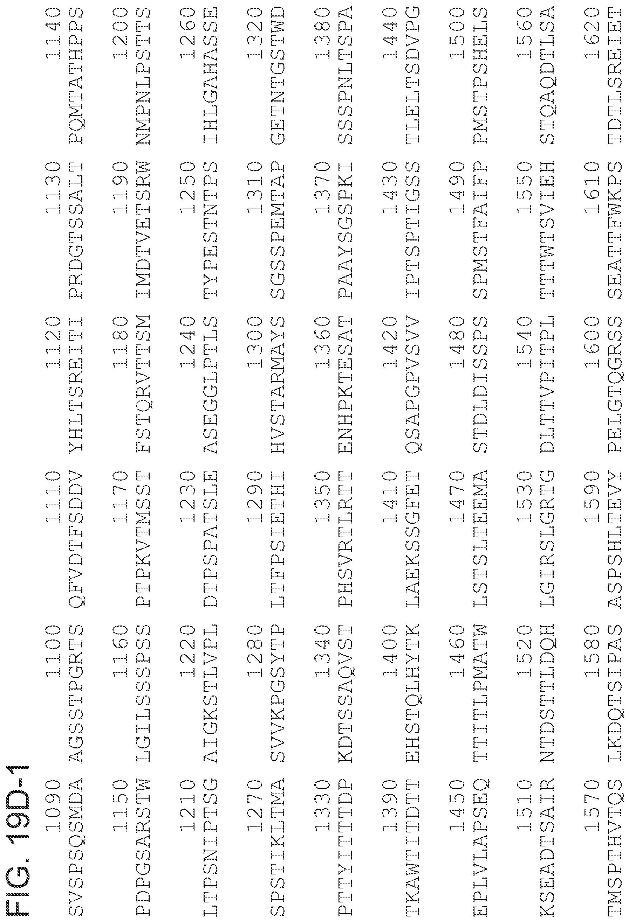

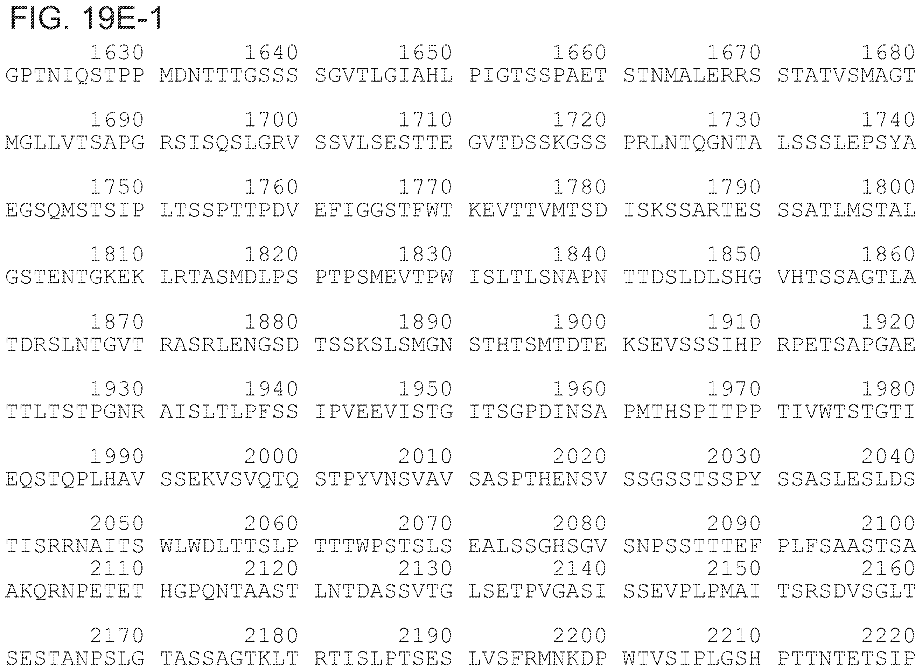

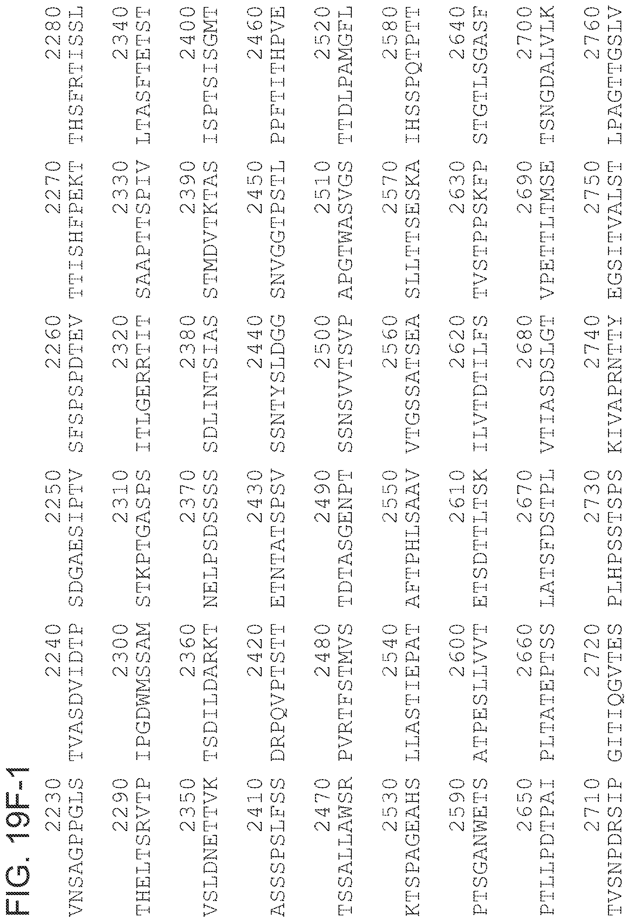

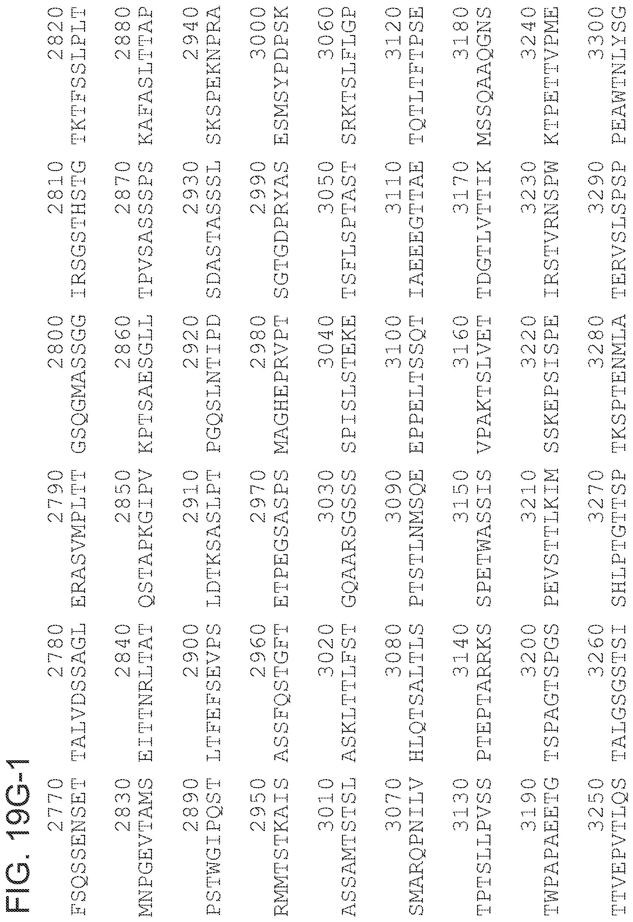

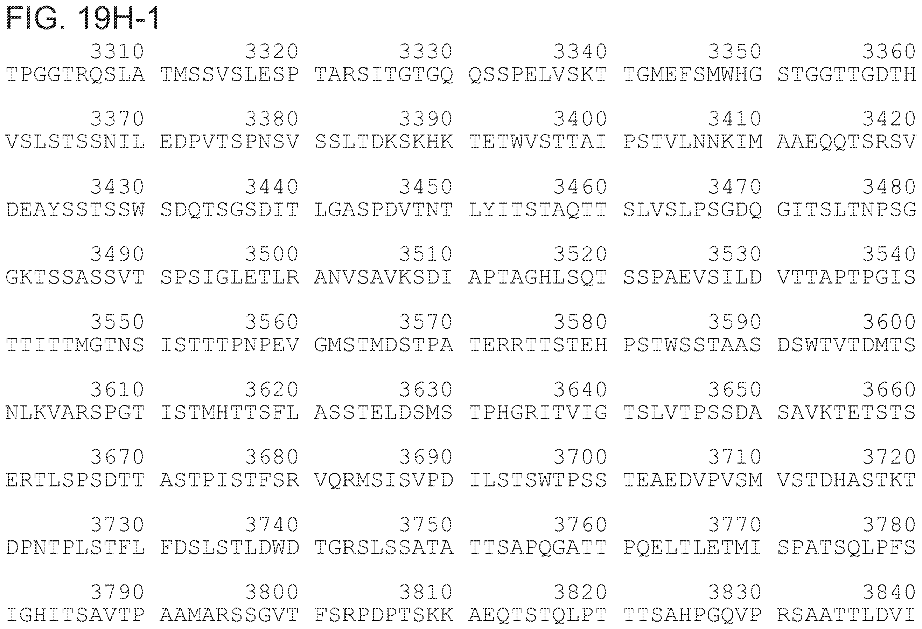

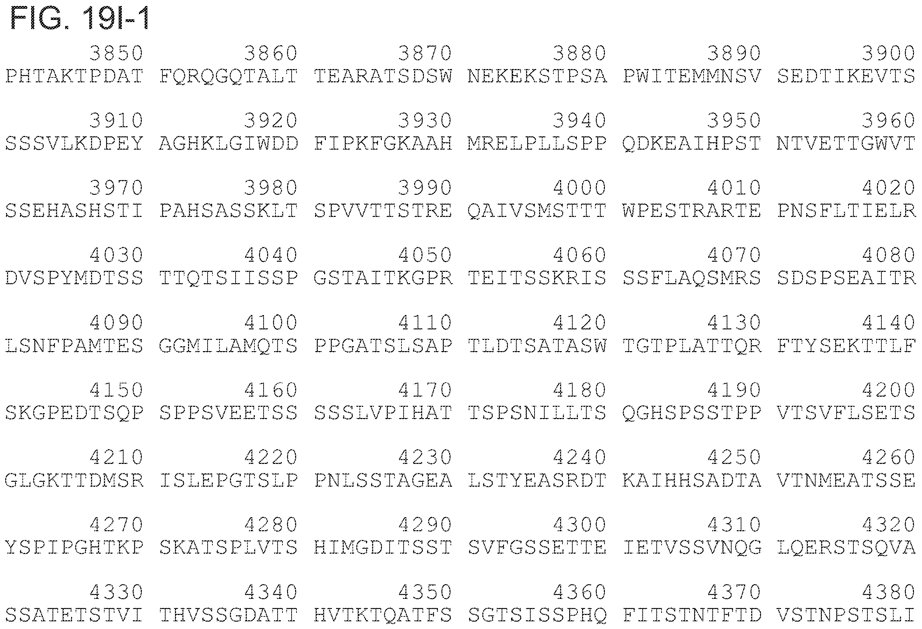

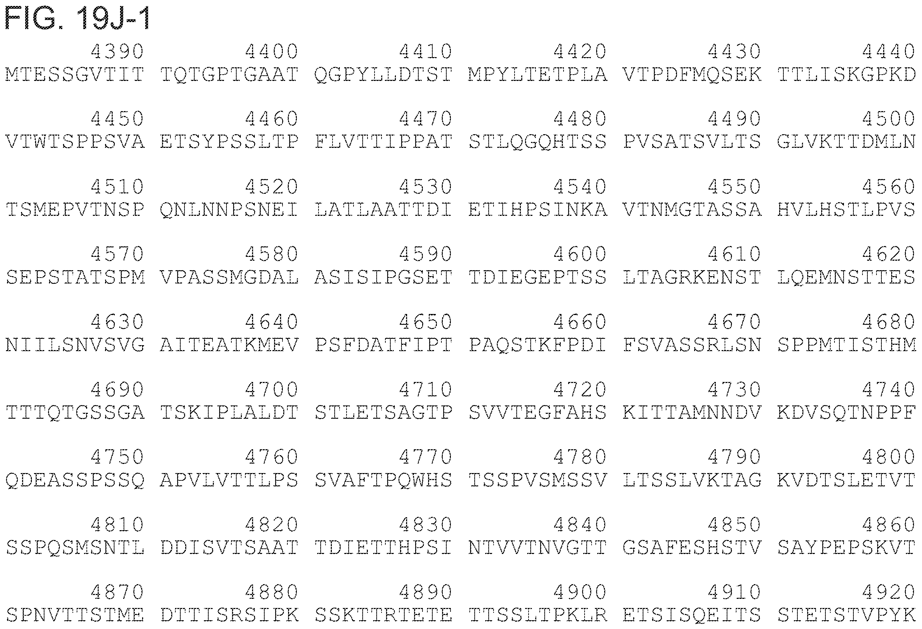

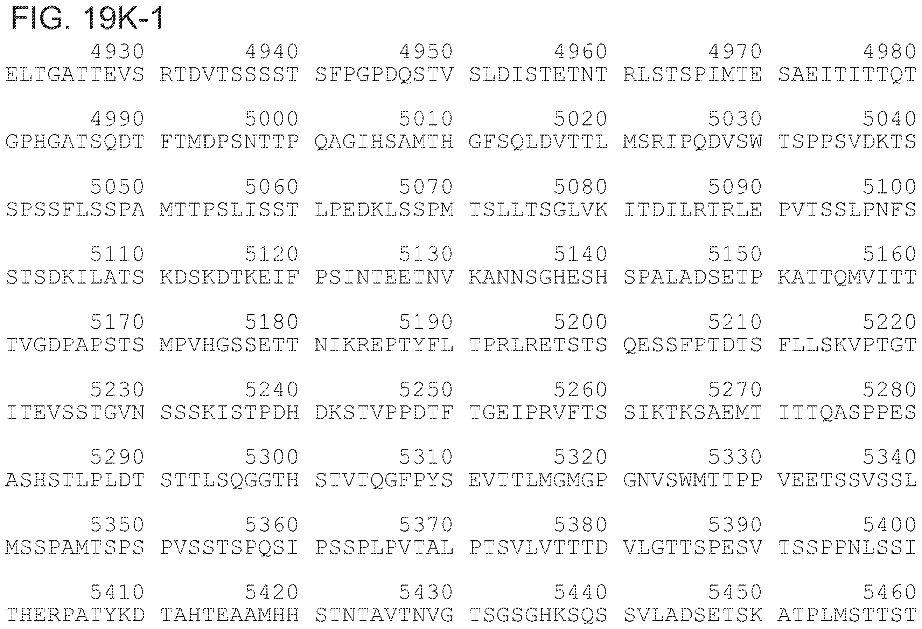

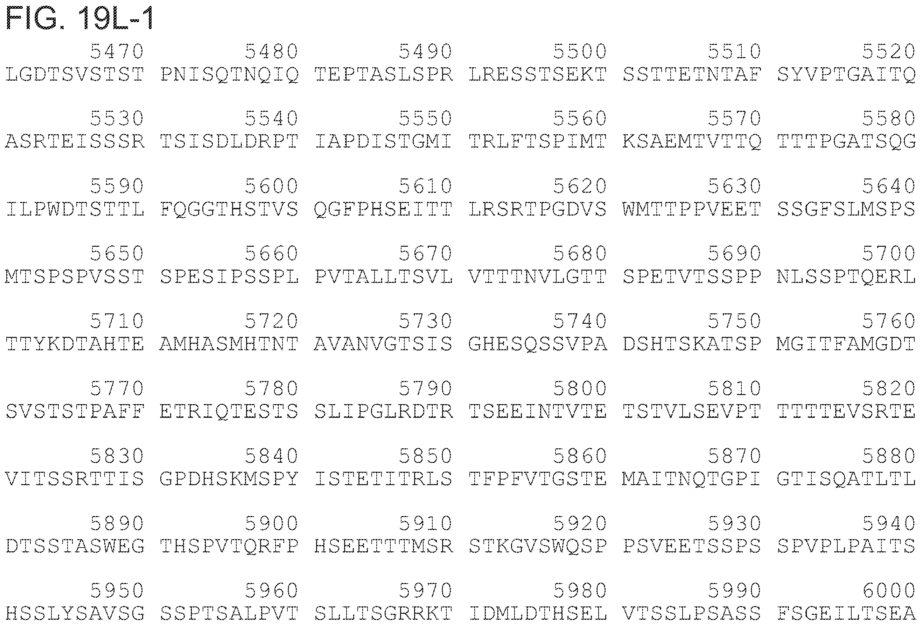

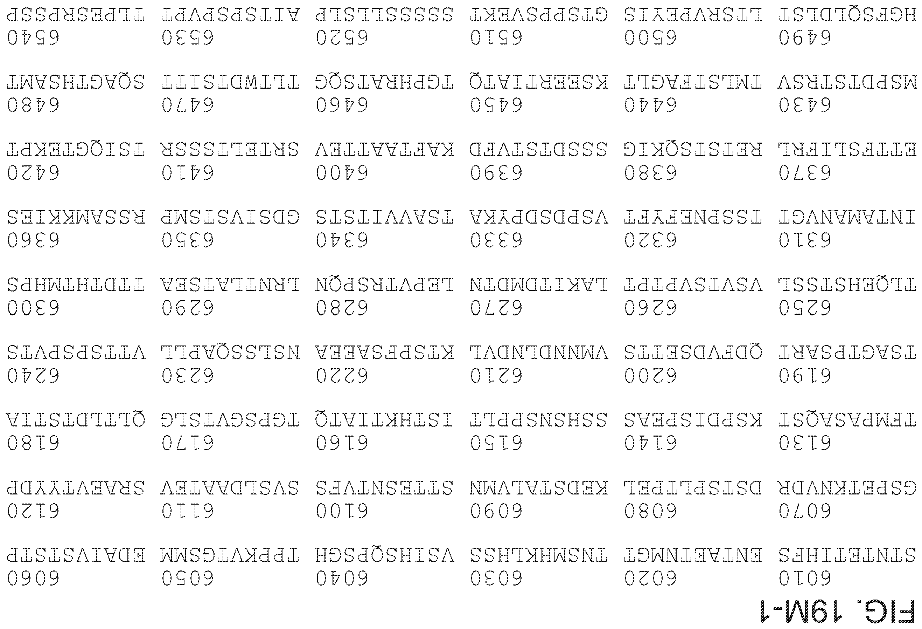

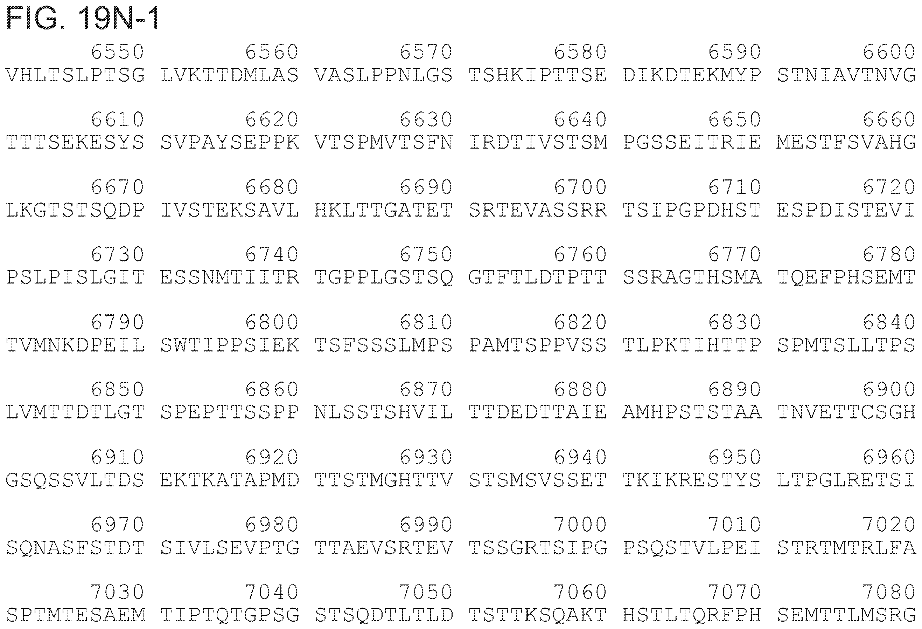

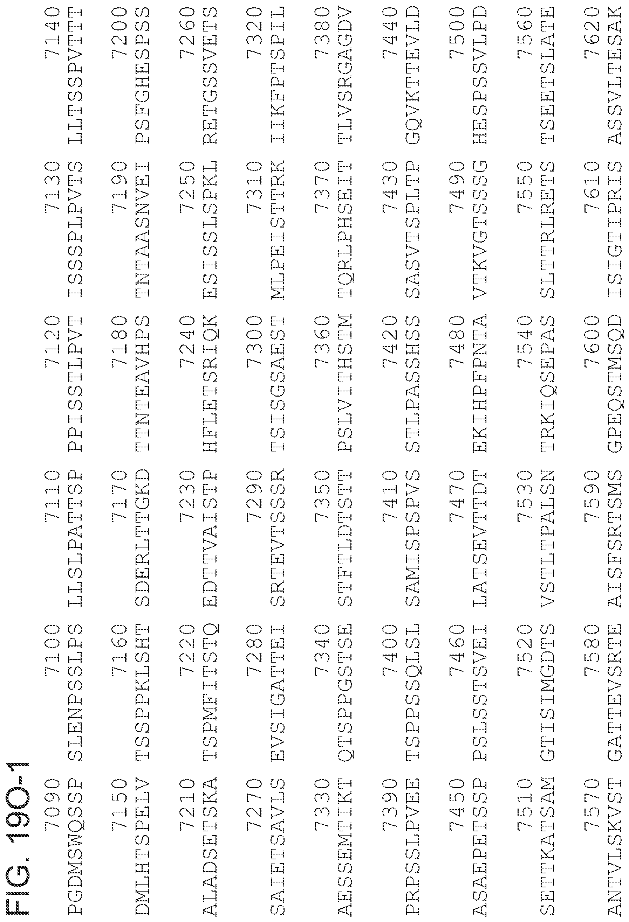

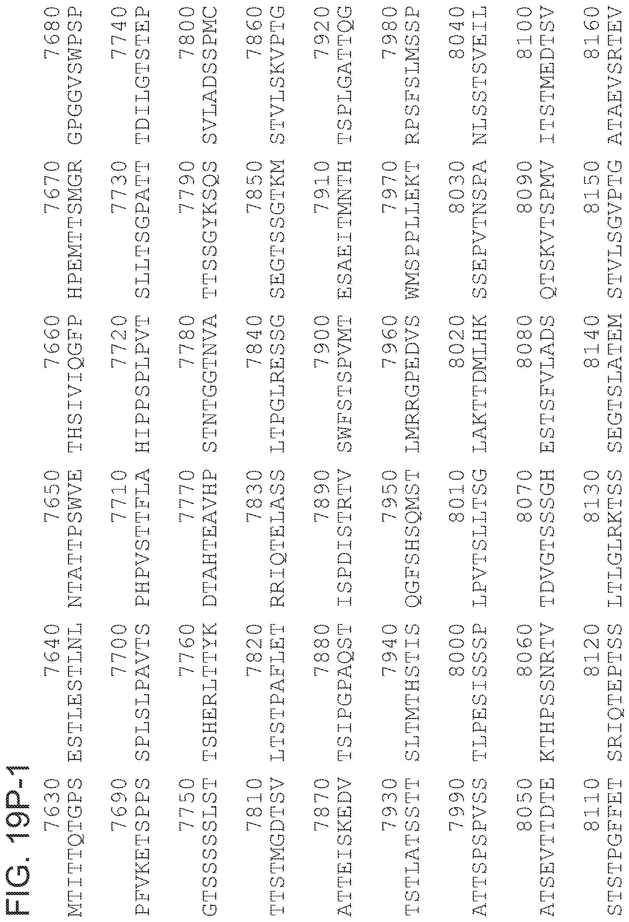

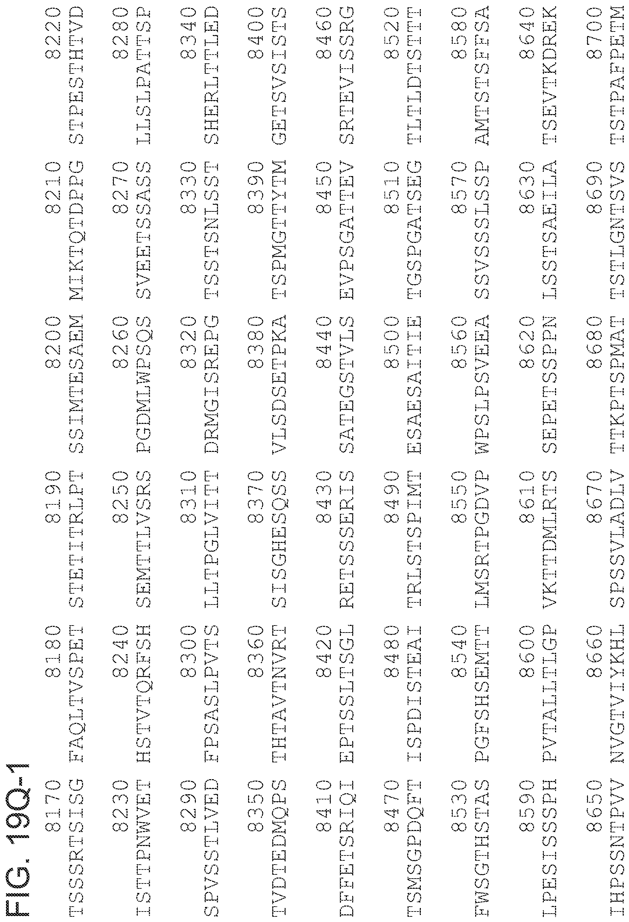

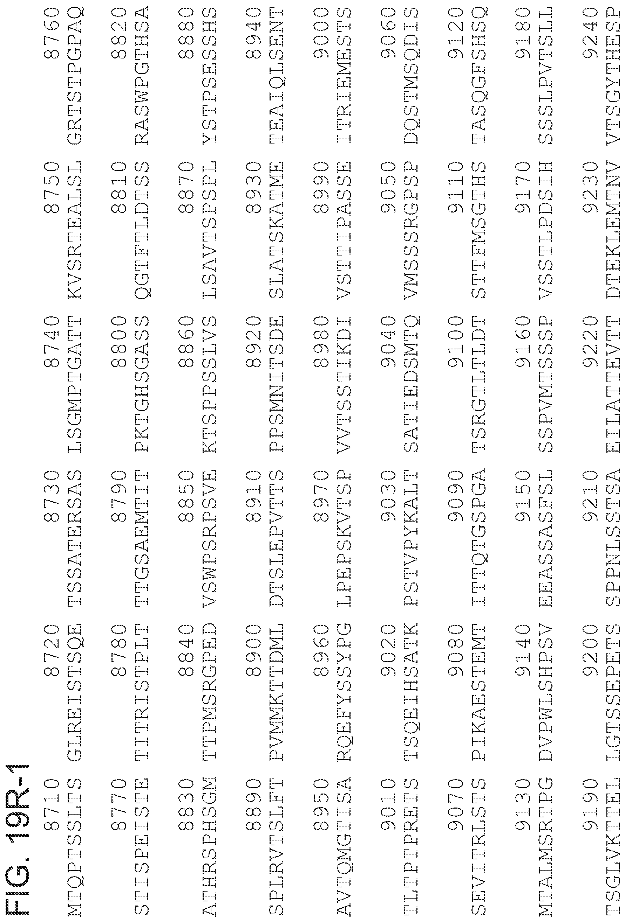

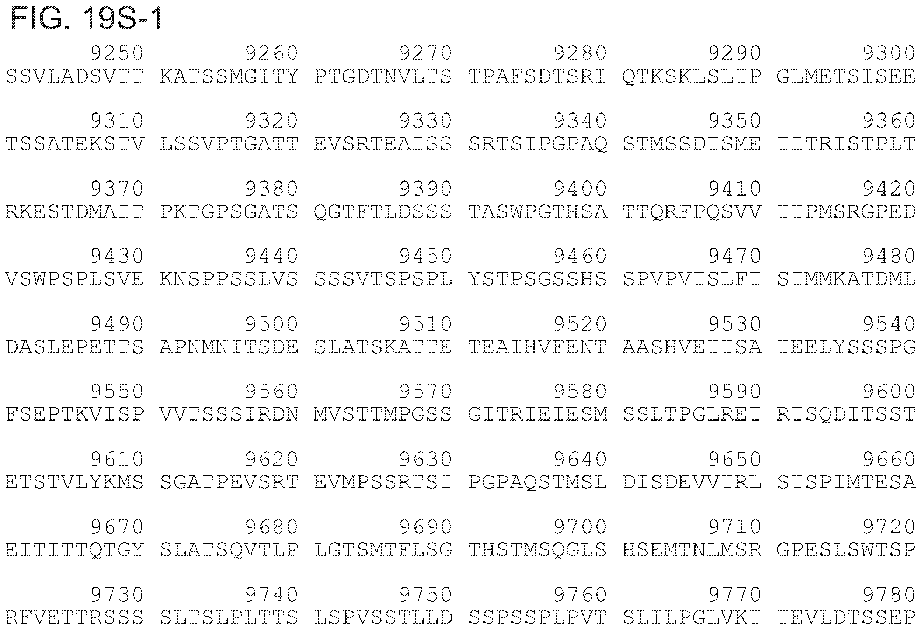

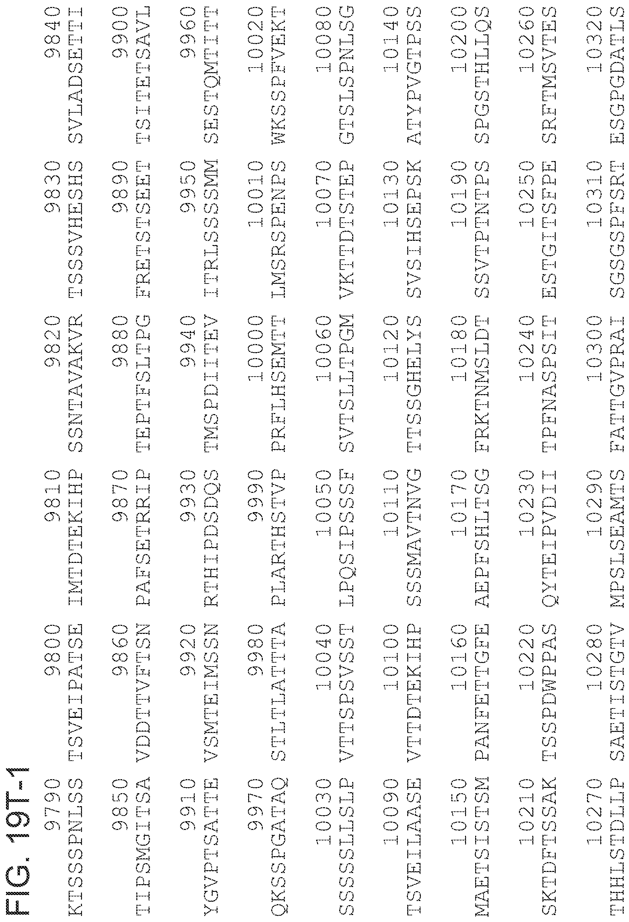

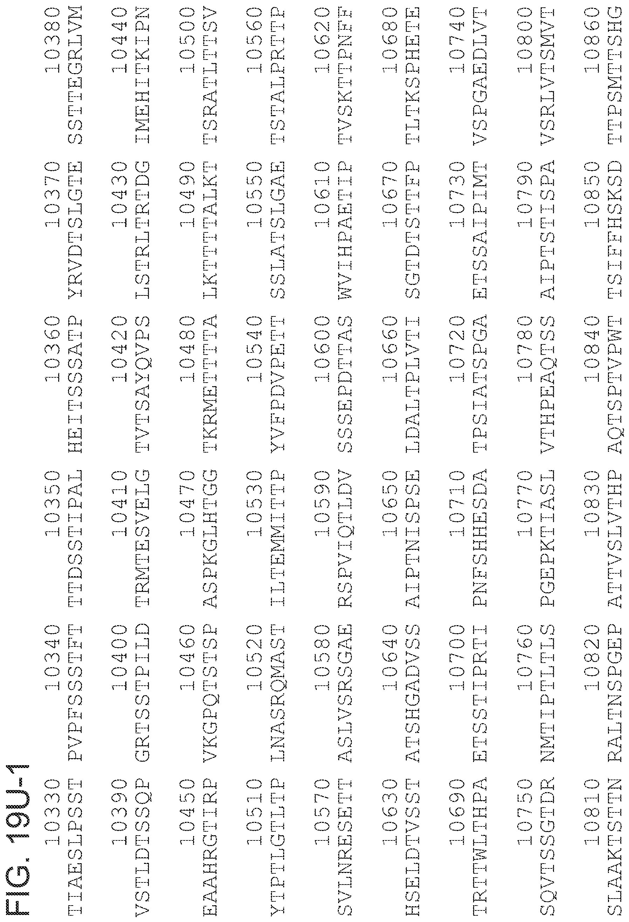

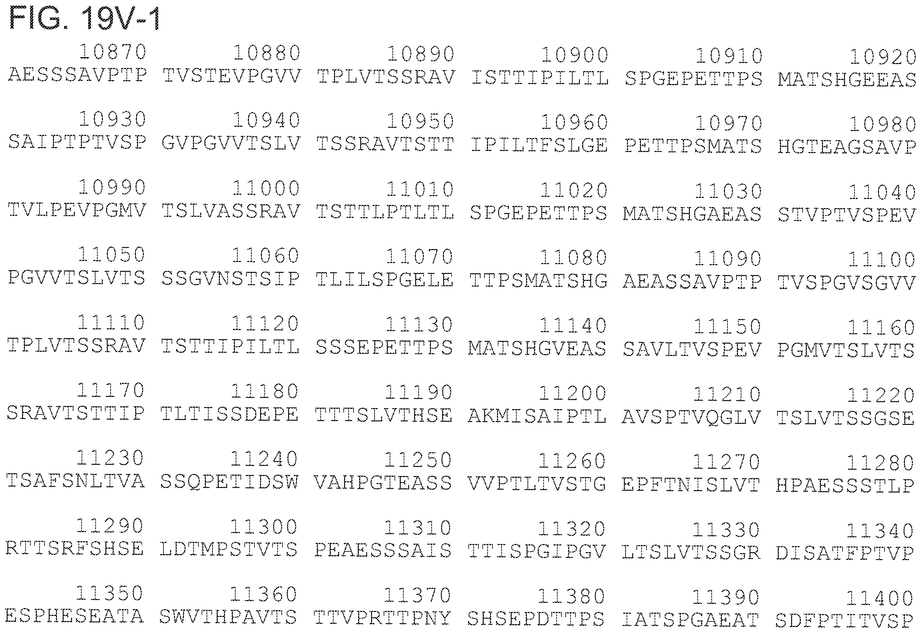

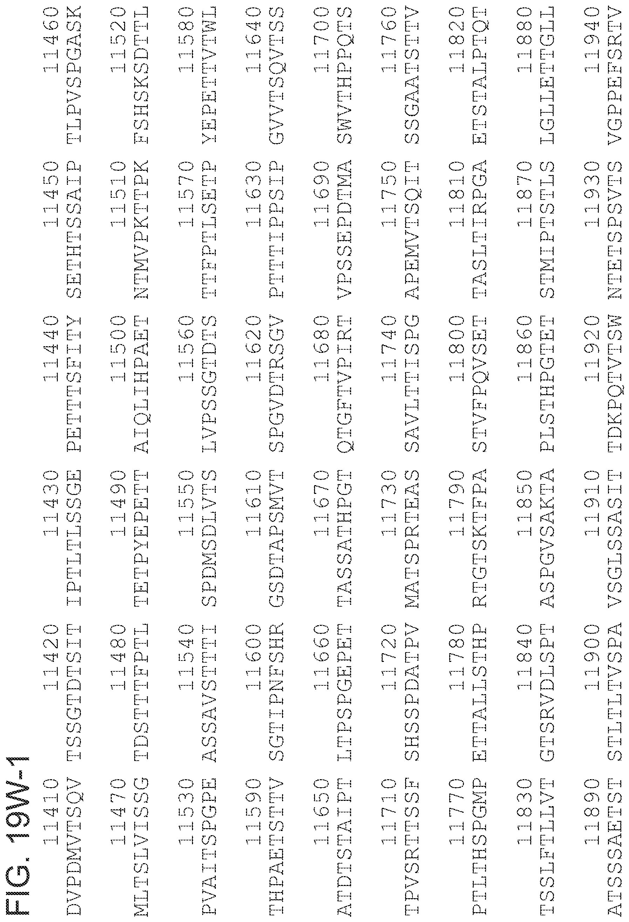

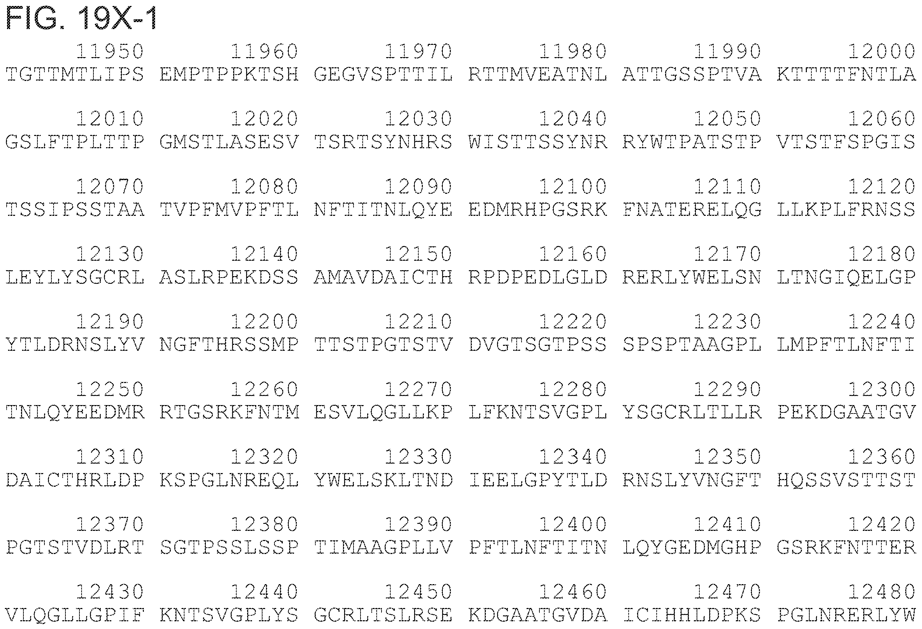

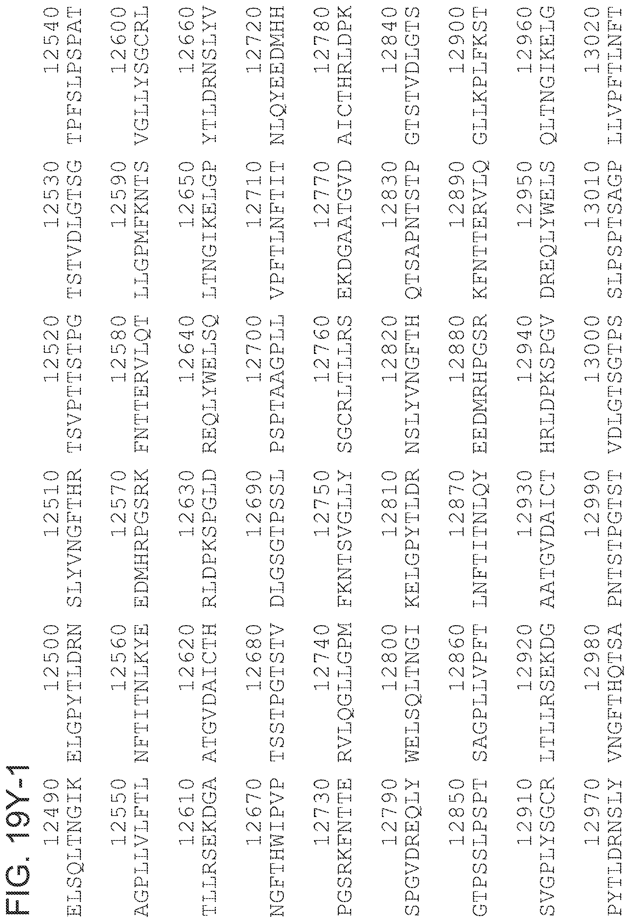

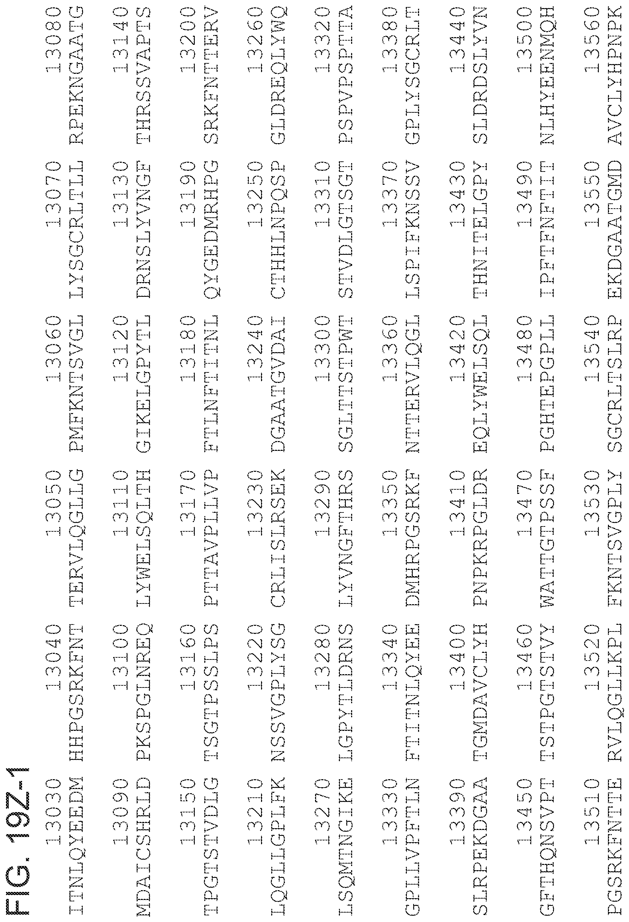

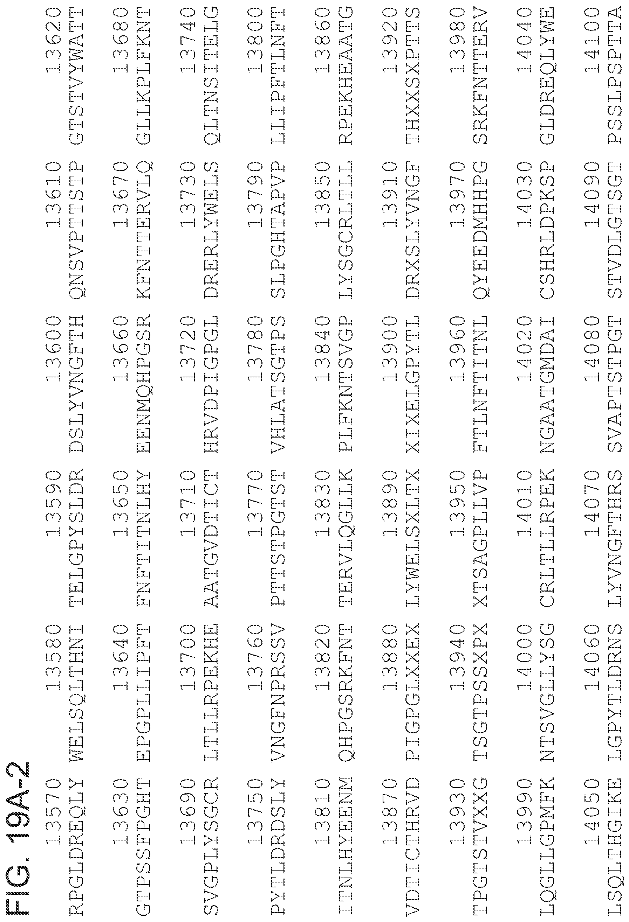

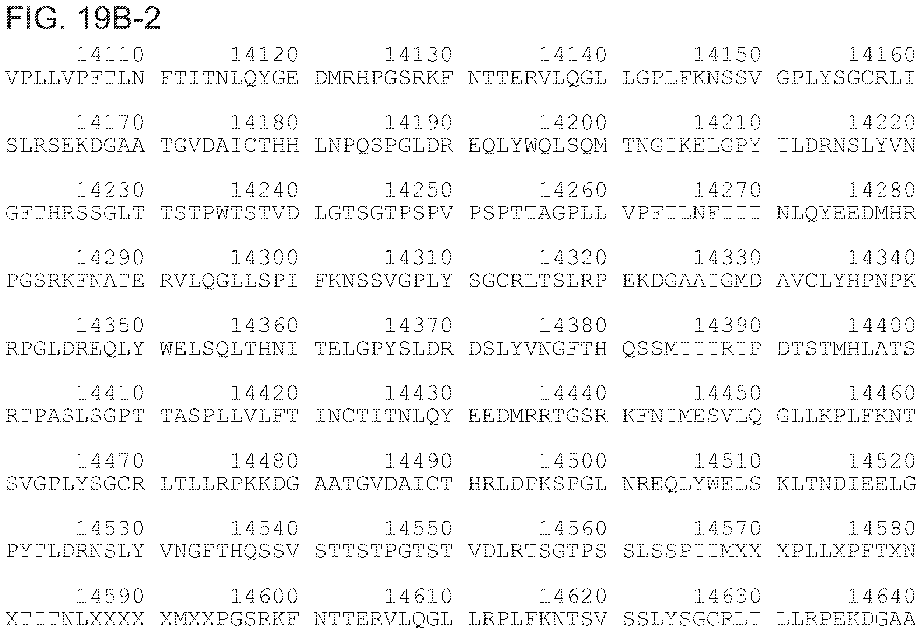

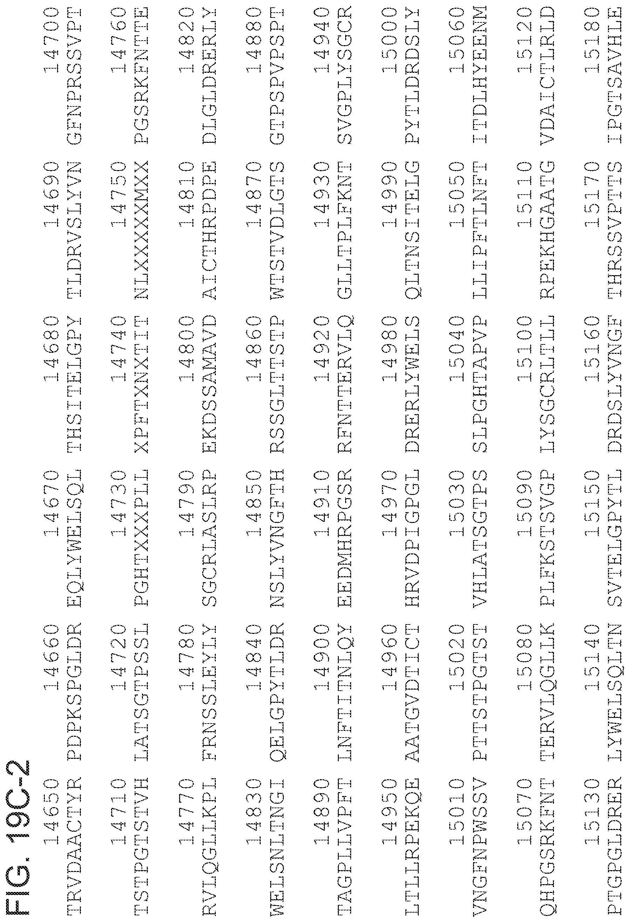

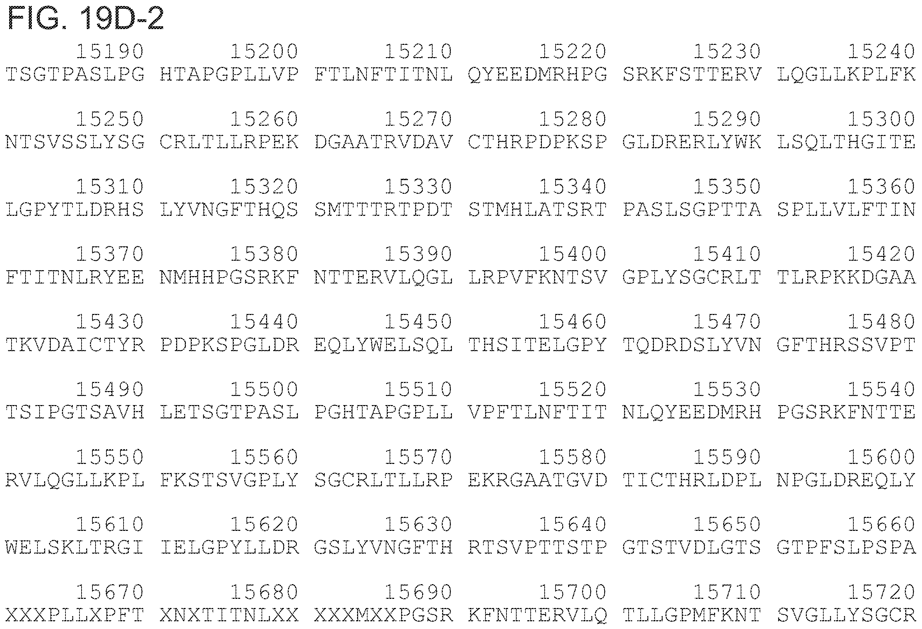

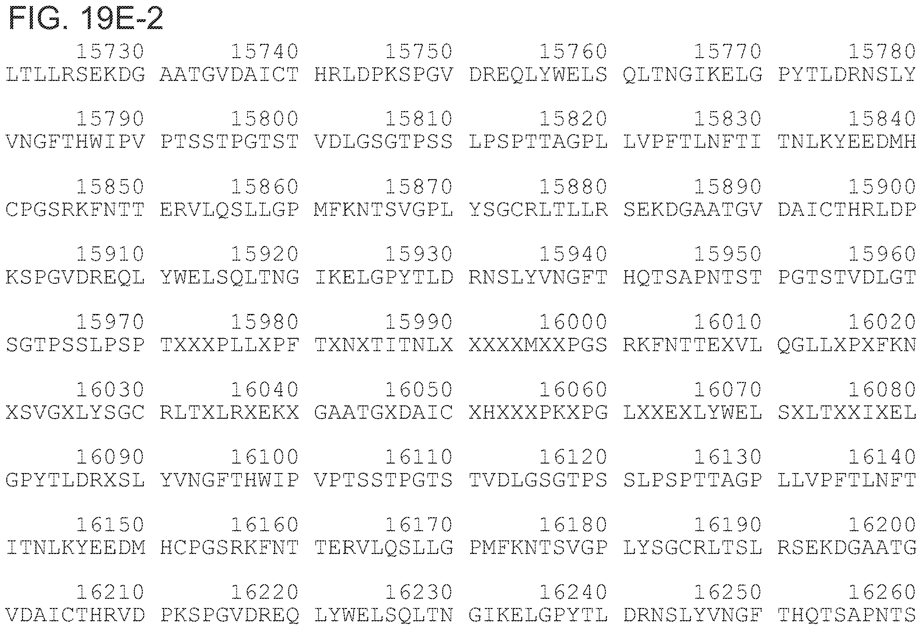

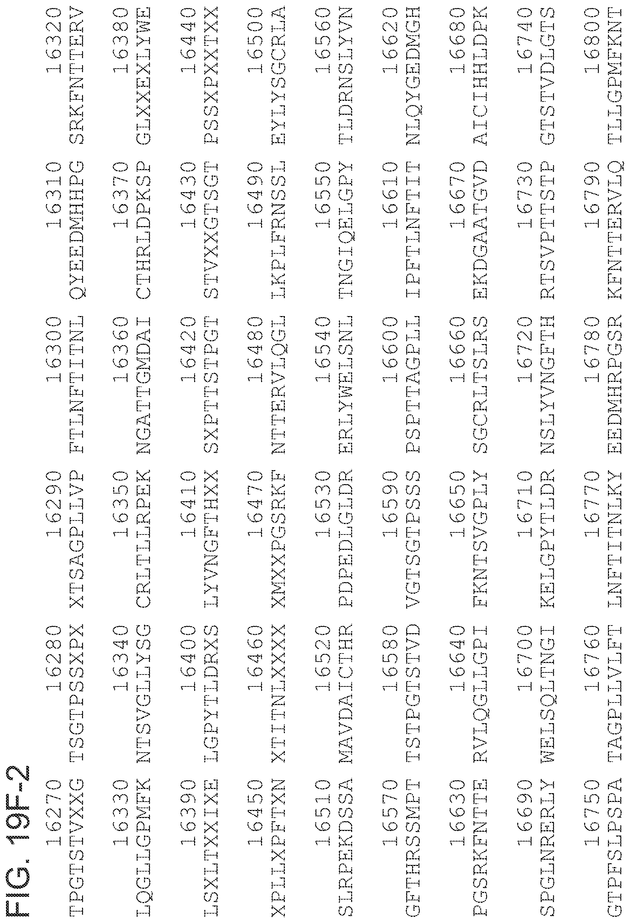

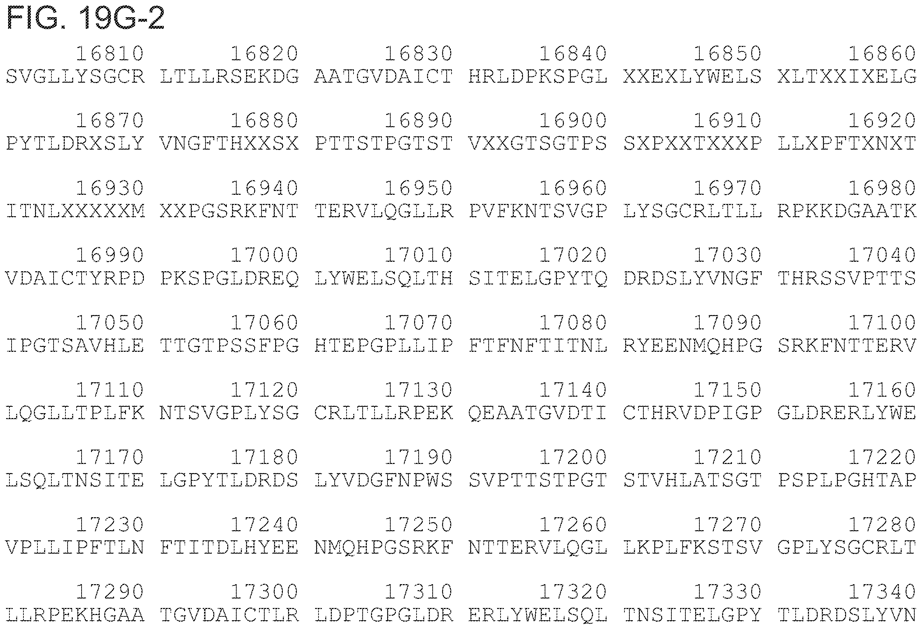

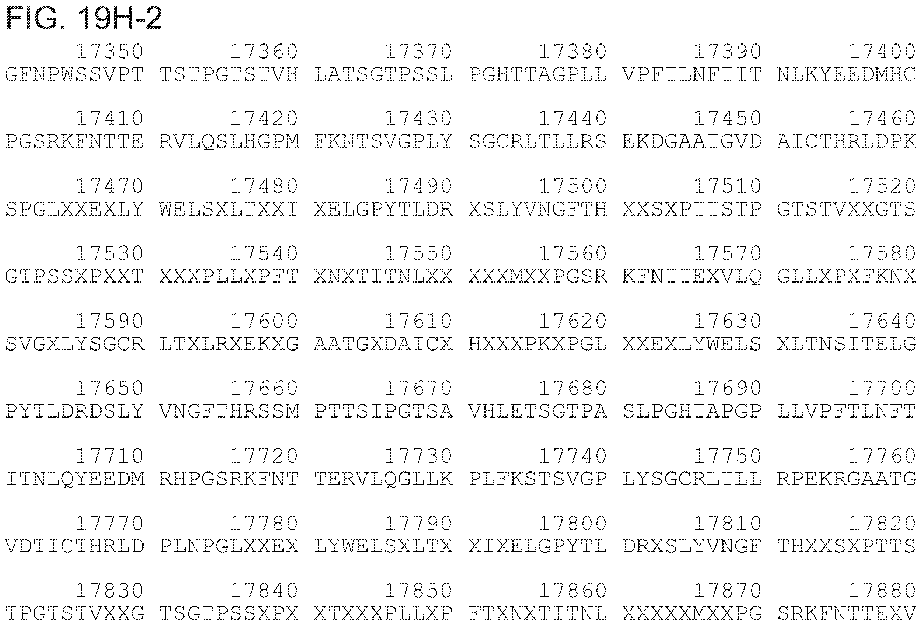

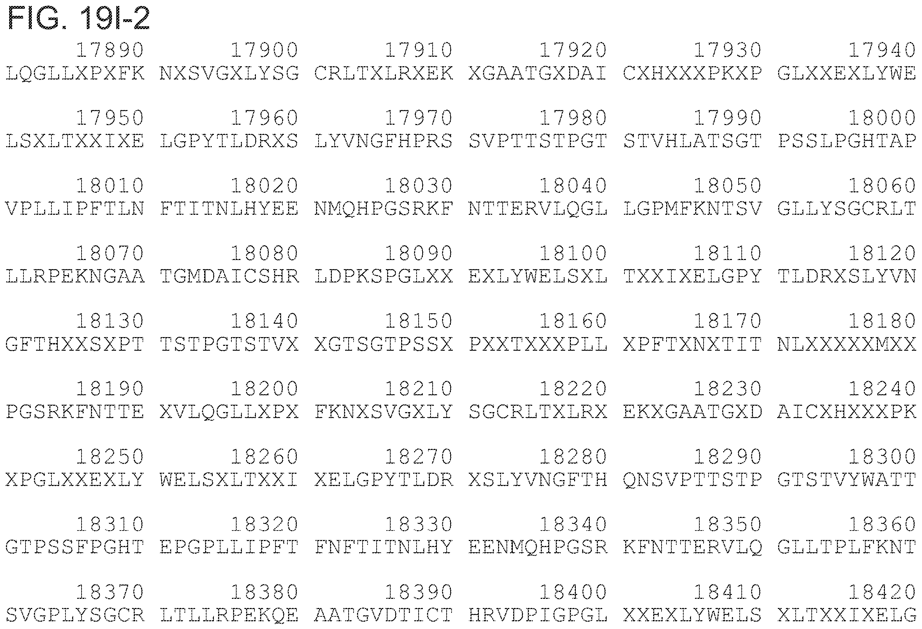

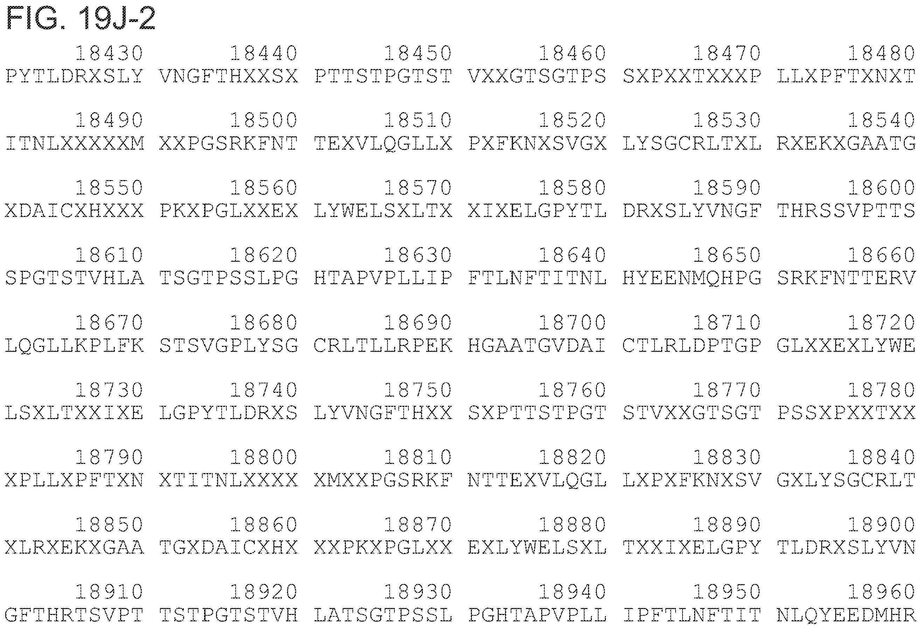

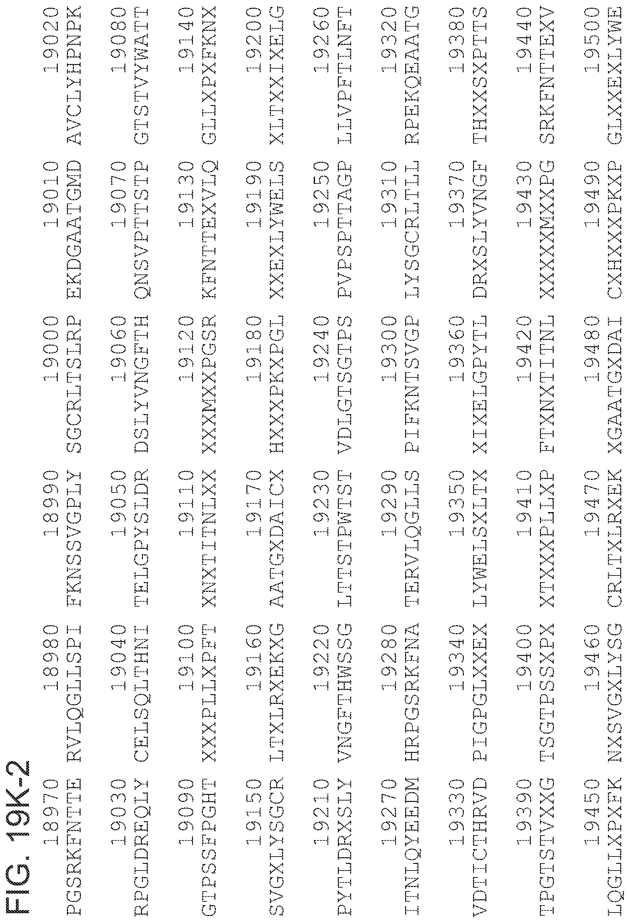

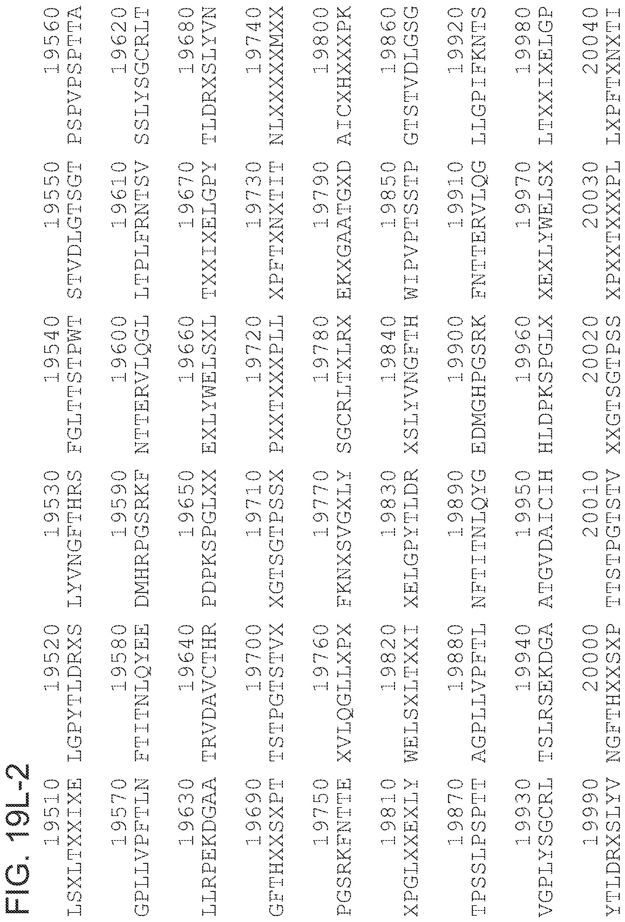

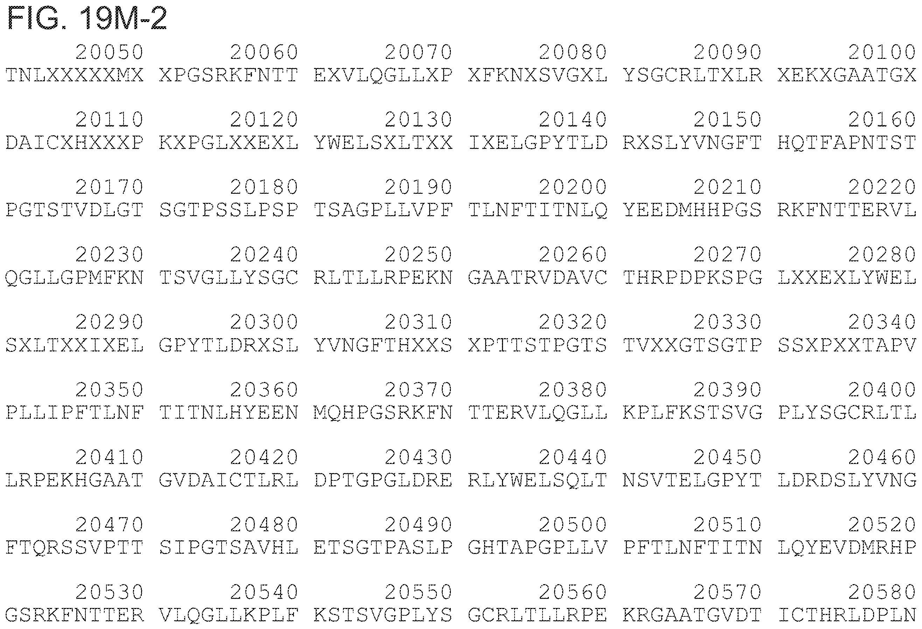

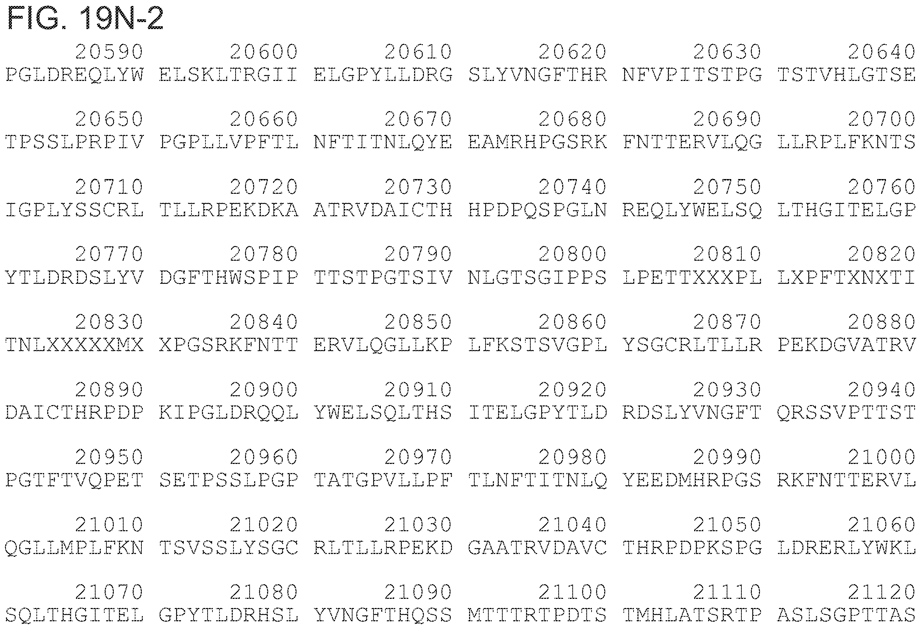

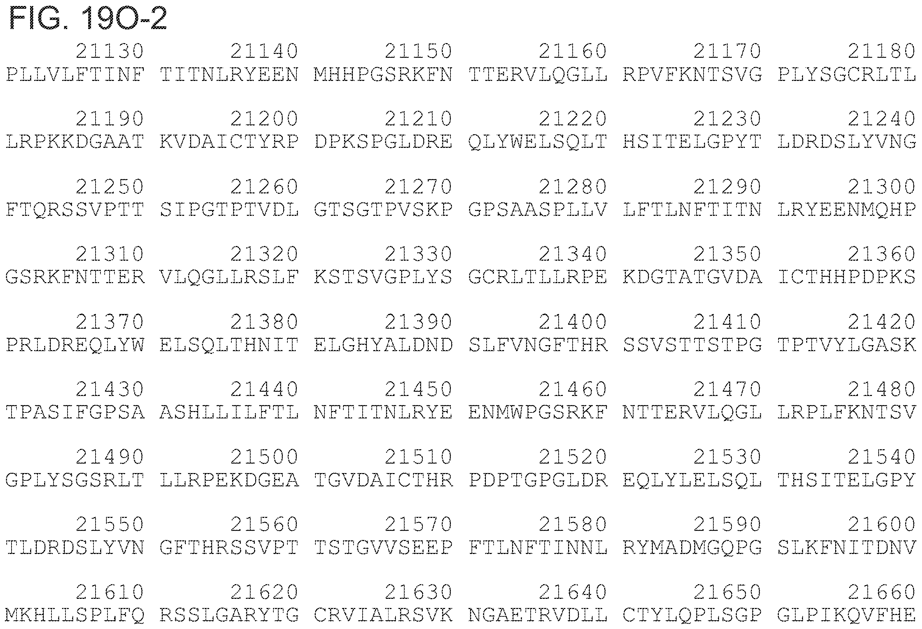

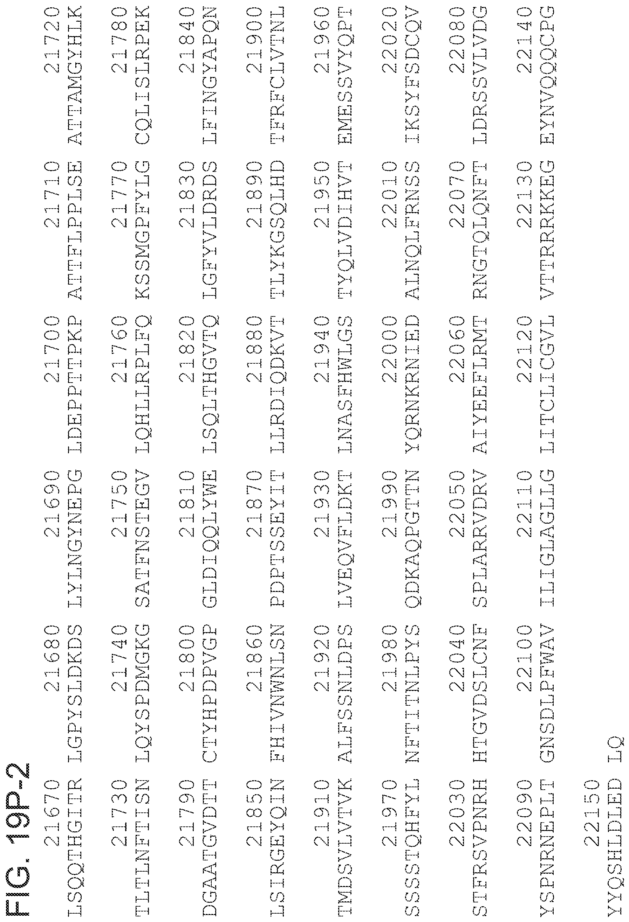

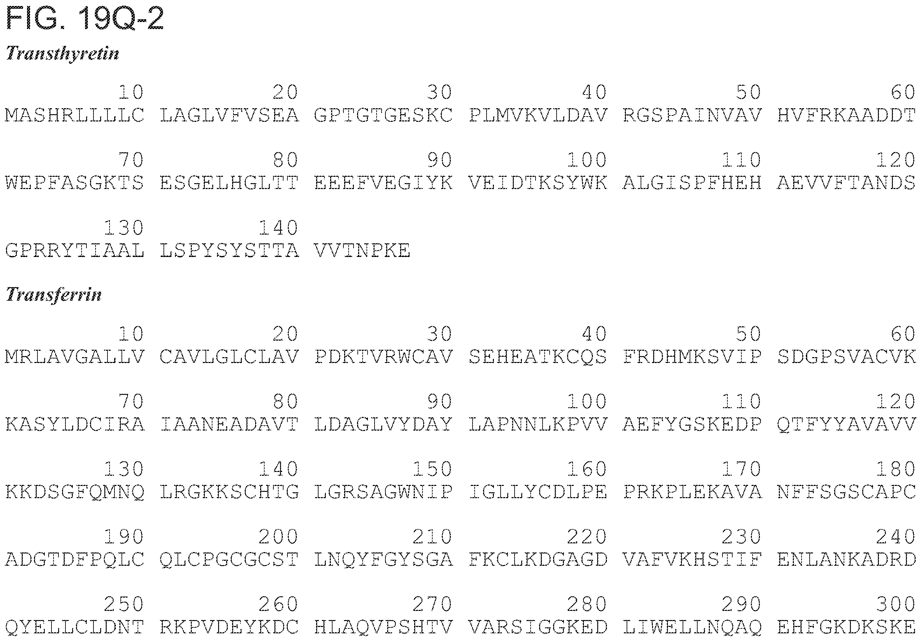

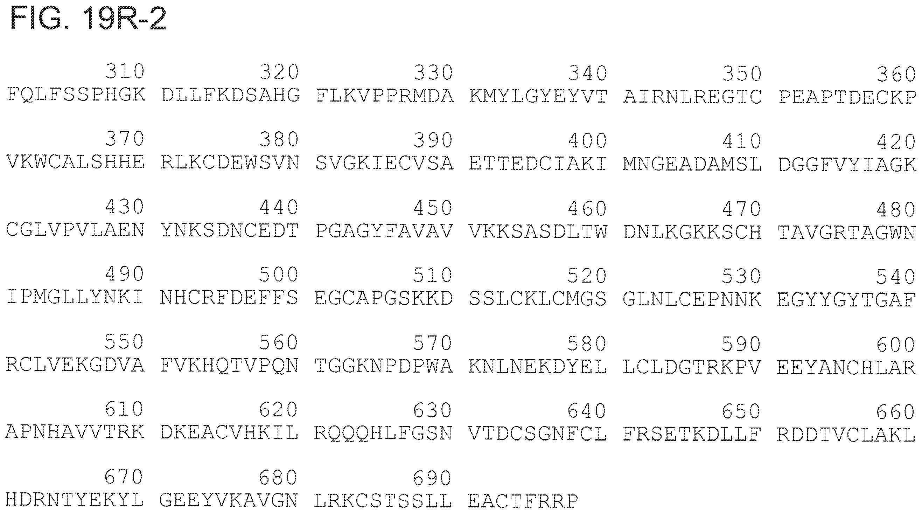

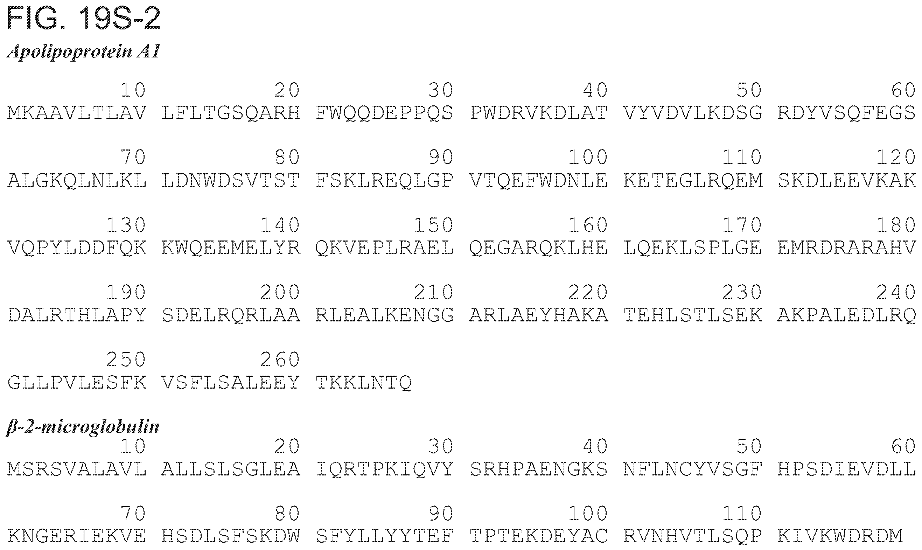

Select exemplary sequences delineated herein are shown at Figure 19.

By "tumor associated glycoprotein (TAG)-72" is meant the antigen measured using the CA72-4 assay. For example, Guadagni,et al., CANCER RESEARCH 52, 1222-1227, March 1, 1992, describes the measurement of TAG-72 using an immunoradiometric assay kit, CA 72-4, supplied by Centocor (Malvern, PA). Either "TAG-72" or "CA72-4" may be used to refer to the antigen identified by CA72-4 as indicated by the term "TAG-72/CA72-4."

By "agent" is meant any small molecule chemical compound, antibody, nucleic acid molecule, or polypeptide, or fragments thereof.

By "alteration" or "change" is meant an increase or decrease. An alteration may be by as little as 1%, 2%, 3%, 4%, 5%, 10%, 20%, 30%, or by 40%, 50%, 60%, or even by as much as 70%, 75%, 80%, 90%, or 100%.

By "biologic sample" is meant any tissue, cell, fluid, or other material derived from an organism.

By "capture reagent" is meant a reagent that specifically binds a nucleic acid molecule or polypeptide to select or isolate the nucleic acid molecule or polypeptide.

By "clinical aggressiveness" is meant the severity of the neoplasia. Aggressive neoplasias are more likely to metastasize than less aggressive neoplasias. While conservative methods of treatment are appropriate for less aggressive neoplasias, more aggressive neoplasias require more aggressive therapeutic regimens.

As used herein, the terms "determining", "assessing", "assaying", "measuring" and "detecting" refer to both quantitative and qualitative determinations, and as such, the term "determining" is used interchangeably herein with "assaying," "measuring," and the like. Where a quantitative determination is intended, the phrase "determining an amount" of an analyte and the like is used. Where a qualitative and/or quantitative determination is intended, the phrase "determining a level" of an analyte or "detecting" an analyte is used.

The term "subject" or "patient" refers to an animal which is the object of treatment, observation, or experiment. By way of example only, a subject includes, but is not limited to, a mammal, including, but not limited to, a human or a non-human mammal, such as a non-human primate, murine, bovine, equine, canine, ovine, or feline.

By "Marker profile" is meant a characterization of the expression or expression level of two or more polypeptides or polynucleotides.

By "neoplasia" is meant any disease that is caused by or results in inappropriately high levels of cell division, inappropriately low levels of apoptosis, or both. Examples of cancers include, without limitation, prostate cancer, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, nile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma). Lymphoproliferative disorders are also considered to be proliferative diseases.

The term "ovarian cancer" refers to both primary ovarian tumors as well as metastases of the primary ovarian tumors that may have settled anywhere in the body.

The term "ovarian cancer status" refers to the status of the disease in the patient. Examples of types of ovarian cancer statuses include, but are not limited to, the subject's risk of cancer, the presence or absence of disease, the stage of disease in a patient, and the effectiveness of treatment of disease. According to the invention, a subject identified as having a pelvic mass can be assessed to identify if their ovarian cancer status is benign or malignant.

Nucleic acid molecules useful in the methods disclosed herein include any nucleic acid molecule that encodes a polypeptide as disclosed herein or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. According to one example, hybridization will occur at 30° C in 750 mM NaCl, 75 mM trisodium citrate, and 1% SDS. Alternatively, hybridization will occur at 37° C in 500 mM NaCl, 50 mM trisodium citrate, 1% SDS, 35% formamide, and 100 µg/ml denatured salmon sperm DNA (ssDNA). Further alternativelyy, hybridization will occur at 42° C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25° C, more preferably of at least about 42° C, and even more preferably of at least about 68° C. According to one example, wash steps will occur at 25° C in 30 mM NaCl, 3 mM trisodium citrate, and 0.1% SDS. Alternatively, wash steps will occur at 42° C in 15 mM NaCl, 1.5 mM trisodium citrate, and 0.1% SDS. Further alternatively, wash steps will occur at 68° C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence.

By "reference" is meant a standard of comparison. For example, the marker level(s) present in a patient sample may be compared to the level of the marker in a corresponding healthy cell or tissue or in a diseased cell or tissue (*e.g*., a cell or tissue derived from a subject having ovarian cancer). In particular, the IGFBP2, IL6, FSH, HE4, CA 125; Transthyretin, Transferrin, TAG-72/CA 72-4 polypeptide level present in a patient sample may be compared to the level of said polypeptide present in a corresponding sample obtained at an earlier time point (i.e., prior to treatment), to a healthy cell or tissue or a neoplastic cell or tissue that lacks a propensity to metastasize. As used herein, the term "sample" includes a biologic sample such as any tissue, cell, fluid, or other material derived from an According to the invention, the subject has an adnexal mass.

By "specifically binds" is meant a compound (e.g., antibody) that recognizes and binds a molecule (e.g., polypeptide), but which does not substantially recognize and bind other molecules in a sample, for example, a biological sample.

The accuracy of a diagnostic test can be characterized using any method well known in the art, including, but not limited to, a Receiver Operating Characteristic curve ("ROC curve"). An ROC curve shows the relationship between sensitivity and specificity. Sensitivity is the percentage of true positives that are predicted by a test to be positive, while specificity is the percentage of true negatives that are predicted by a test to be negative. An ROC is a plot of the true positive rate against the false positive rate for the different possible cutpoints of a diagnostic test. Thus, an increase in sensitivity will be accompanied by a decrease in specificity. The closer the curve follows the left axis and then the top edge of the ROC space, the more accurate the test. Conversely, the closer the curve comes to the 45-degree diagonal of the ROC graph, the less accurate the test. The area under the ROC is a measure of test accuracy. The accuracy of the test depends on how well the test separates the group being tested into those with and without the disease in question. An area under the curve (referred to as "AUC") of 1 represents a perfect test. Biomarkers and diagnostic methods as disclosed herein may lead to an AUC greater than 0.50, greater than 0.60, greater than 0.70, greater than 0.80, or greater than 0.9.

Other useful measures of the utility of a test are positive predictive value ("PPV") and negative predictive value ("NPV"). PPV is the percentage of actual positives who test as positive. NPV is the percentage of actual negatives that test as negative.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

Any compounds, compositions, or methods provided herein can be combined with one or more of any of the other compositions and methods provided herein.

As used herein, the singular forms "a", "an", and "the" include plural forms unless the context clearly dictates otherwise. Thus, for example, reference to "a biomarker" includes reference to more than one biomarker.

Unless specifically stated or obvious from context, as used herein, the term "or" is understood to be inclusive.

The term "including" is used herein to mean, and is used interchangeably with, the phrase "including but not limited to."

As used herein, the terms "comprises," "comprising," "containing," "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited.

### BRIEF DESCRIPTION OF THE DRAWINGS

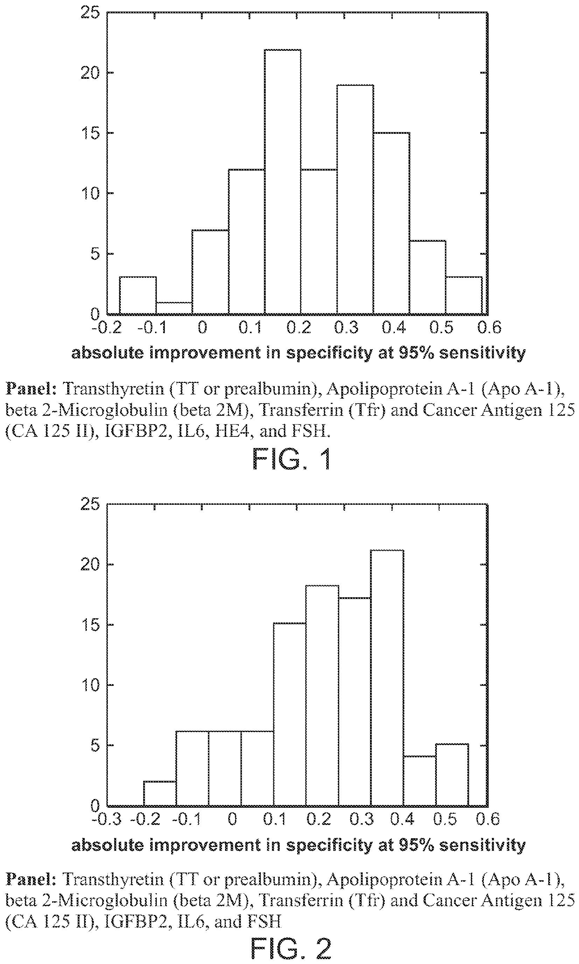

Figure 1 is a plot depicting distribution of absolute improvement in specificity at fixed 95% sensitivity in testing over 100 rounds of cross-validation of multivariate models using nine markers Transthyretin (TT or prealbumin), Apolipoprotein A-1 (Apo A-1), beta 2-Microglobulin (beta 2M), Transferrin (Tfr) and Cancer Antigen 125 (CA 125 II), insulin growth factor binding protein 2 (IGFBP2), interleukin-6 (IL6), Human Epididymis Protein 4 (HE4), and follicle stimulating hormone (FSH). Mean and median absolute improvement were 24.1% (95% CI: 21.0-27.2%) and 24.6%, respectively, over Transthyretin (TT or prealbumin), Apolipoprotein A-1 (Apo A-1), beta 2-Microglobulin (beta 2M), Transferrin (Tfr) and Cancer Antigen 125 (CA 125 II).
Figure 2 is a plot depicting distribution of absolute improvement in specificity for a model using 8 biomarkers: Transthyretin (TT or prealbumin), Apolipoprotein A-1 (Apo A-1), beta 2-Microglobulin (beta 2M), Transferrin (Tfr) and Cancer Antigen 125 (CA 125 II), IGFBP2, IL6, and FSH over the specificity provided by the following markers: Transthyretin (TT or prealbumin), Apolipoprotein A-1 (Apo A-1), beta 2-Microglobulin (beta 2M), Transferrin (Tfr) and Cancer Antigen 125 (CA 125 II) at fixed 95% sensitivity in testing over 100 rounds of cross-validation of multivariate models. Mean and median absolute improvement were 22.0% (95% CI: 18.8-25.3%) and 23.3%, respectively.

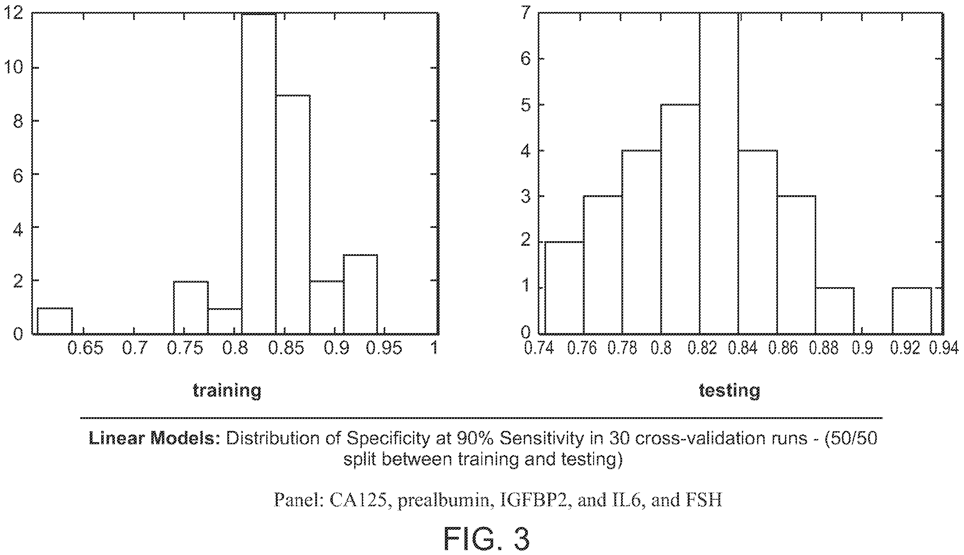

Figure 3 shows plots depicting Distribution of Specificity in linear models at 90% Sensitivity in 30 cross-validation runs - (50/50 split between training (left panel) and testing (right panel). Panel: CA125, prealbumin, IGFBP2, and IL6, and FSH.

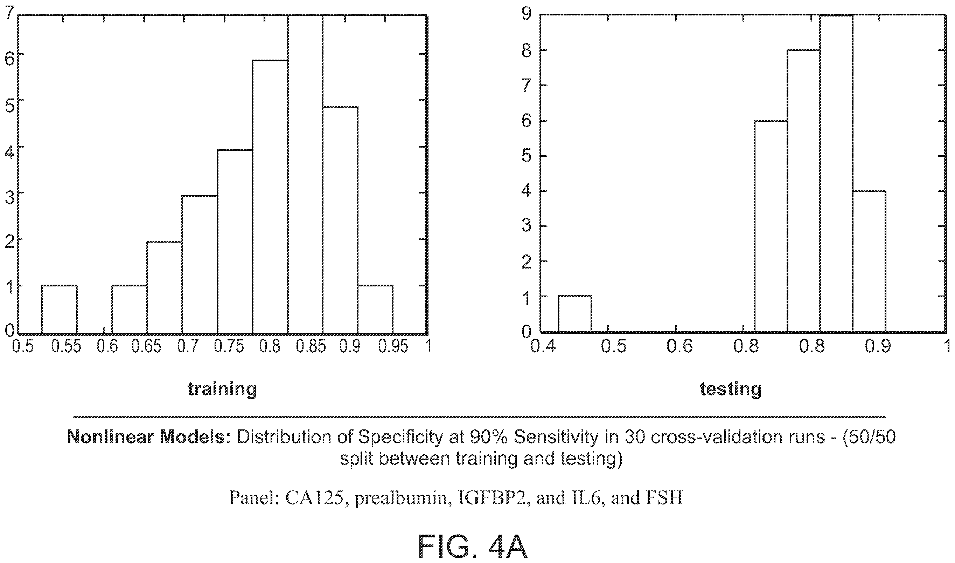

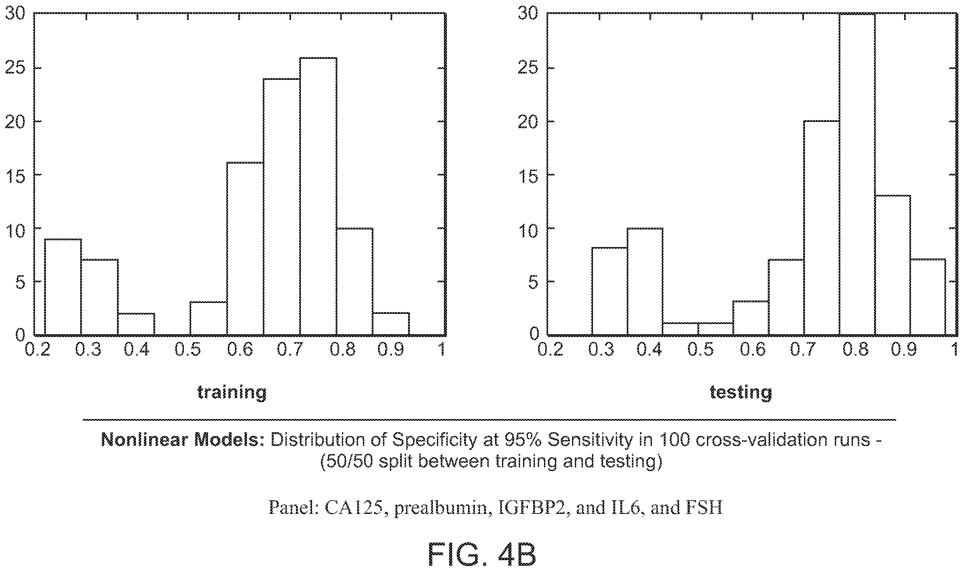

Figures 4A and 4B show plots depicting Distribution of Specificity in nonlinear models. Figure 4A shows plots depicting Distribution of Specificity at 90% Sensitivity in 30 cross-validation runs - (50/50 split between training (left panel) and testing (right panel). Figure 4B shows plots depicting Distribution of Specificity at 95% Sensitivity in 100 cross-validation runs - (50/50 split between training (left panel) and testing (right panel). Panel: CA125, prealbumin, IGFBP2, and IL6, and FSH

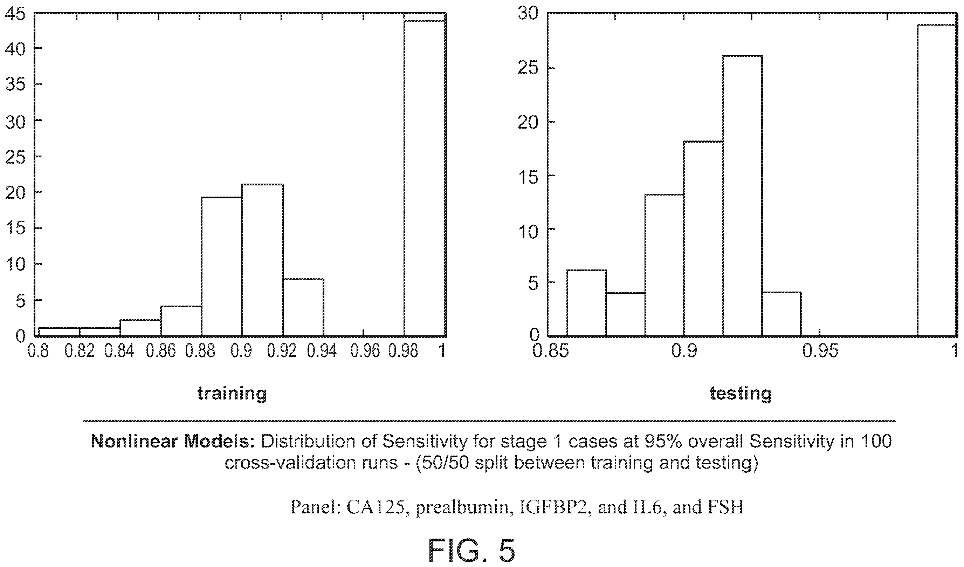

Figure 5 shows plots depicting Distribution of Sensitivity in nonlinear models for stage 1 cases at 95% overall Sensitivity in 100 cross-validation runs - (50/50 split between training (left panel) and testing (right panel). Panel: CA125, prealbumin, IGFBP2, and IL6, and FSH

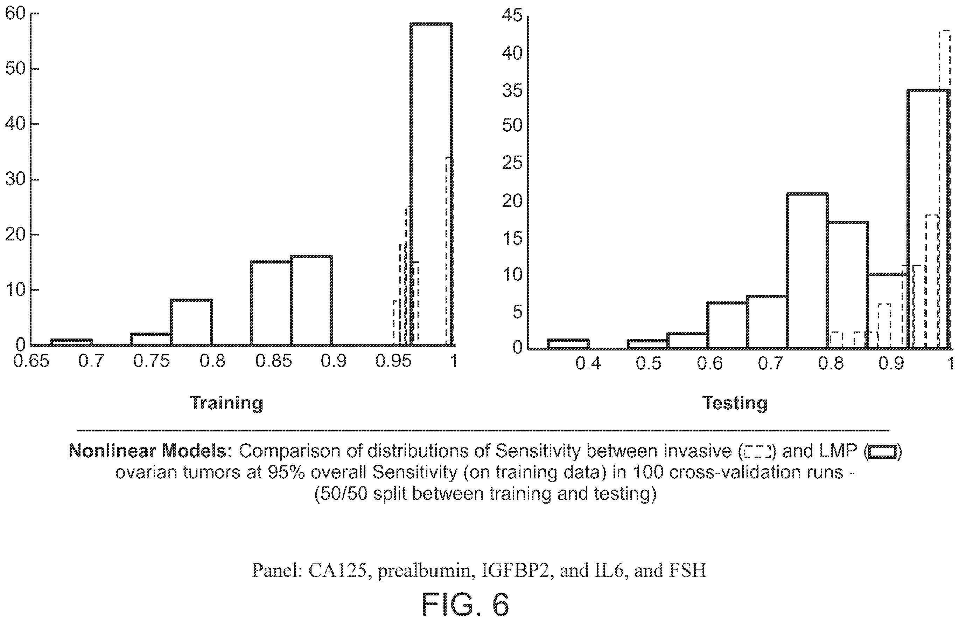

Figure 6 shows plots depicting comparison of distributions of Sensitivity (nonlinear models) between invasive (red) and LMP (blue) ovarian tumors at 95% overall Sensitivity (on training data) in 100 cross-validation runs - (50/50 split between training (left panel) and testing (right panel). Panel: CA125, prealbumin, IGFBP2, and IL6, and FSH

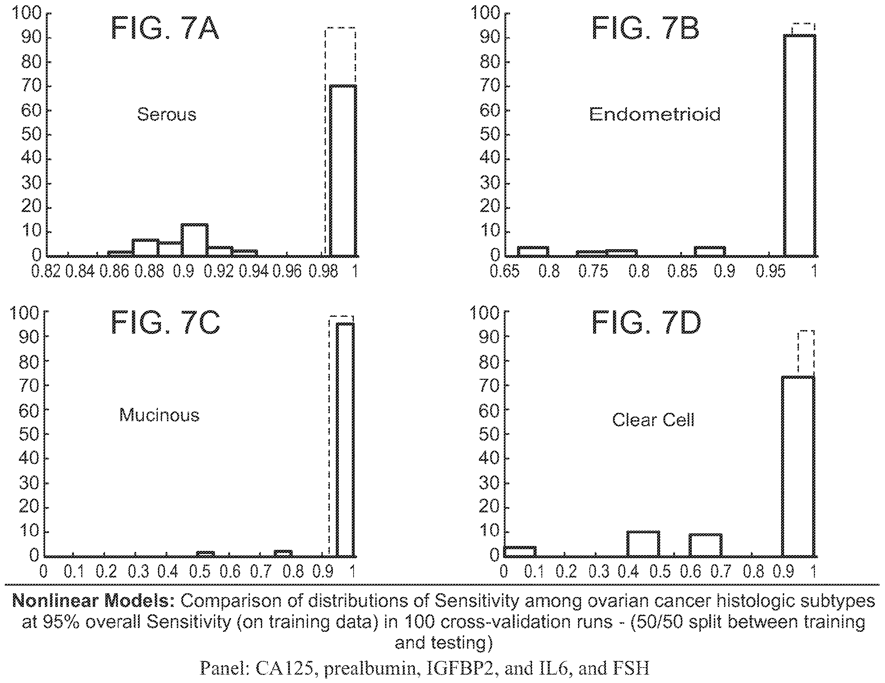

Figures 7A-7D show plots depicting comparison of distributions of Sensitivity in nonlinear models among ovarian cancer histologic subtypes. Figure 7A is a plot depicting Distribution of Specificity at 95% overall Sensitivity (on training data) in 100 cross-validation runs - (50/50 split between training and testing) for serous ovarian cancer. Figure 7B is a plot depicting Distribution of Specificity at 95% overall Sensitivity (on training data) in 100 cross-validation runs - (50/50 split between training and testing) for endometrioid ovarian cancer. Figure 7C is a plot depicting Distribution of Specificity at 95% overall Sensitivity (on training data) in 100 cross-validation runs - (50/50 split between training and testing) for mucinous ovarian cancer. Figure 7D is a plot depicting Distribution of Specificity at 95% overall Sensitivity (on training data) in 100 cross-validation runs - (50/50 split between training and testing) for clear cell ovarian cancer. Panel: CA125, prealbumin, IGFBP2, and IL6, and FSH.

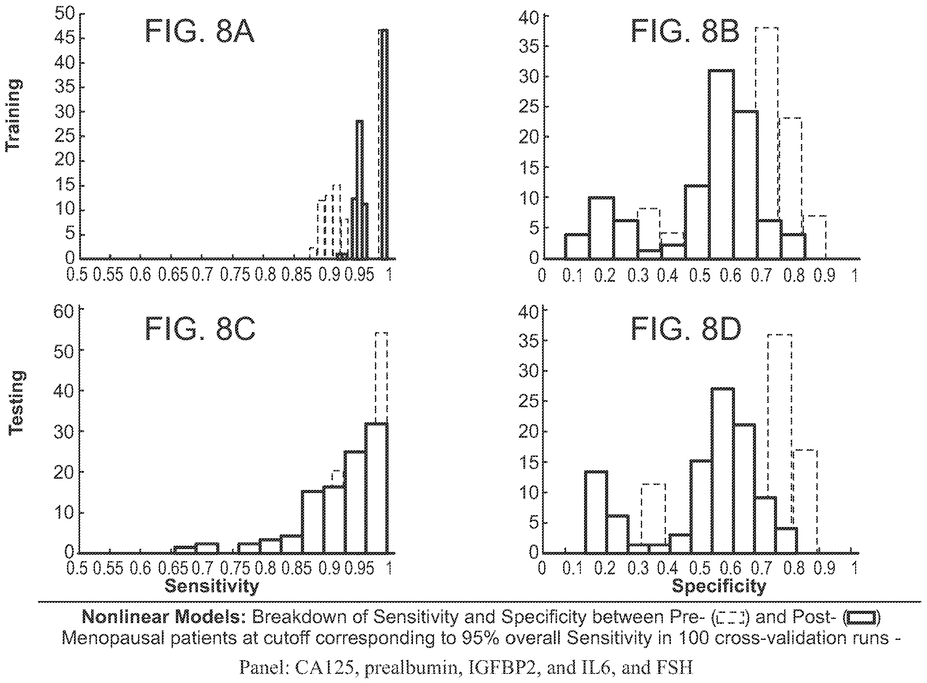

Figures 8A-8D show plots depicting Distributions of Sensitivity and Specificity in training and testing sets between Pre- (red) and Post- (blue) Menopausal patients. Figure 8A is a plot depicting Distributions of Sensitivity between Pre- (red) and Post- (blue) Menopausal patients at cutoff corresponding to 95% overall Sensitivity in 100 cross-validation runs - (50/50 split between training and testing) in the training set. Figure 8B is a plot depicting Distributions of Specificity between Pre- (red) and Post- (blue) Menopausal patients at cutoff corresponding to 95% overall Sensitivity in 100 cross-validation runs - (50/50 split between training and testing) in the training set. Figure 8C is a plot depicting Distributions of Sensitivity between Pre- (red) and Post- (blue) Menopausal patients at cutoff corresponding to 95% overall Sensitivity in 100 cross-validation runs - (50/50 split between training and testing) in the testing set. Figure 8D is a plot depicting Distributions of Specificity between Pre- (red) and Post- (blue) Menopausal patients at cutoff corresponding to 95% overall Sensitivity in 100 cross-validation runs - (50/50 split between training and testing) in the testing set. Panel: CA125, prealbumin, IGFBP2, and IL6, and FSH.

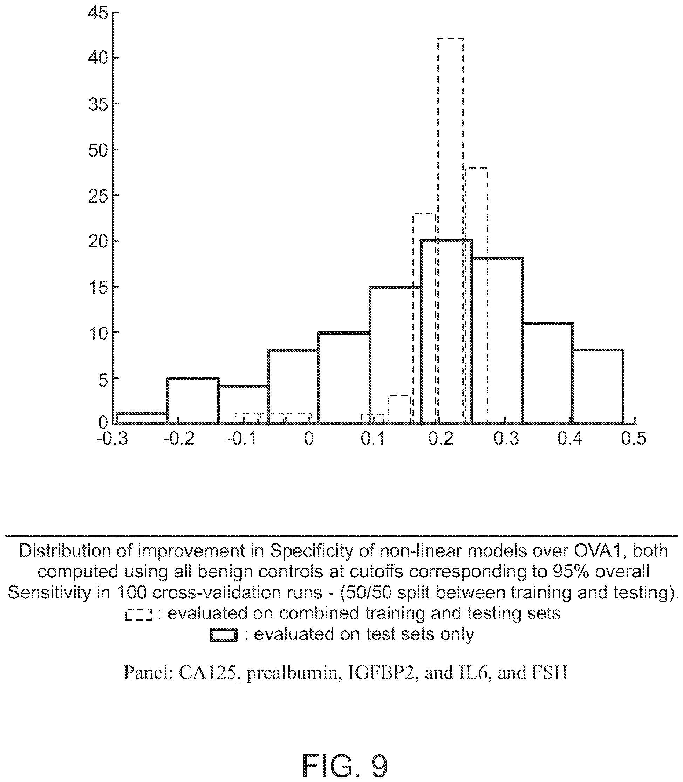

Figure 9 shows plots depicting Distribution of improvement in Specificity of nonlinear models over the following panel of markers: Transthyretin (TT or prealbumin), Apolipoprotein A-1 (Apo A-1), beta 2-Microglobulin (beta 2M), Transferrin (Tfr) and Cancer Antigen 125 (CA 125 II), both computed using all benign controls at cutoffs corresponding to 95% overall Sensitivity in 100 cross-validation runs - (50/50 split between training and testing). Red hollow bars: evaluated on combined training and testing sets, blue solid bars: evaluated on test sets only. Panel: CA125, prealbumin, IGFBP2, IL6, and FSH.

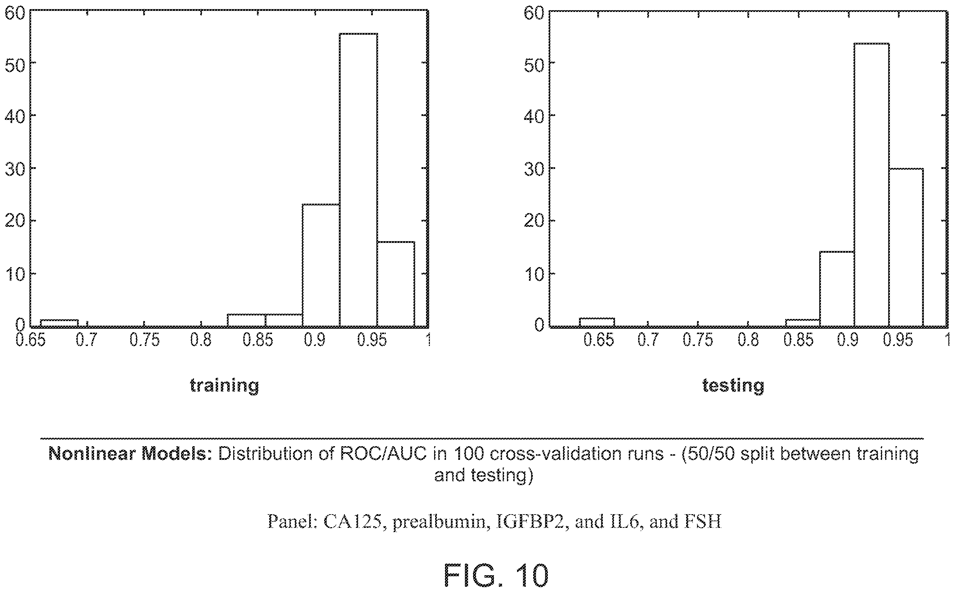

Figure 10 shows plots depicting Distribution of ROC/AUC for a nonlinear model in 100 cross-validation runs - (50/50 split between training (left panel) and testing (right panel). Panel: CA125, prealbumin, IGFBP2, and IL6, and FSH

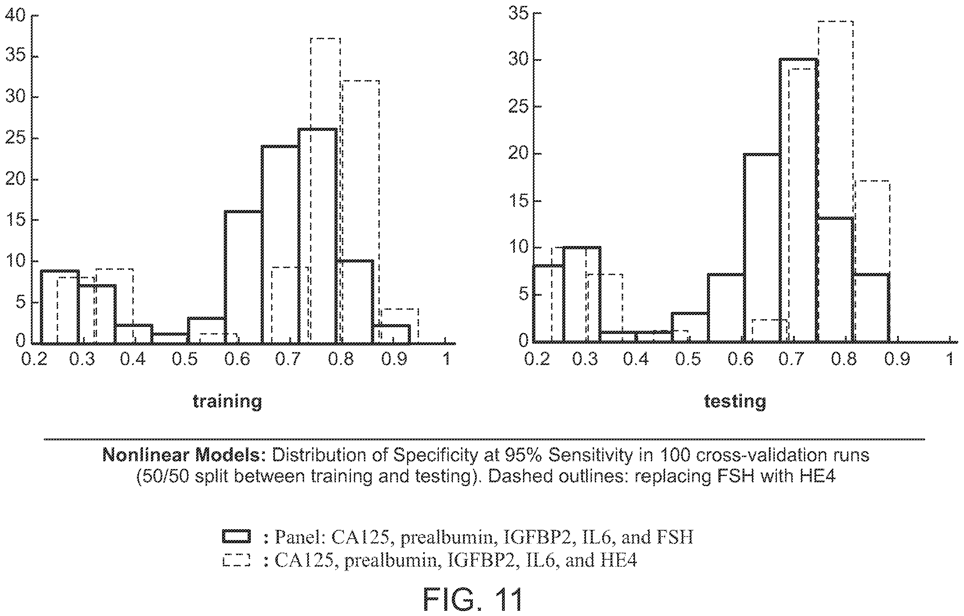

Figure 11 shows plots depicting Distribution of Specificity for a nonlinear model at 95% Sensitivity in 100 cross-validation runs (50/50 split between training (left panel) and testing (right panel). Red hollow bars: HE4 replacing FSH. Blue: Panel: CA125, prealbumin, IGFBP2, IL6, and FSH; Red Outline: CA125, prealbumin, IGFBP2, IL6, and HE4

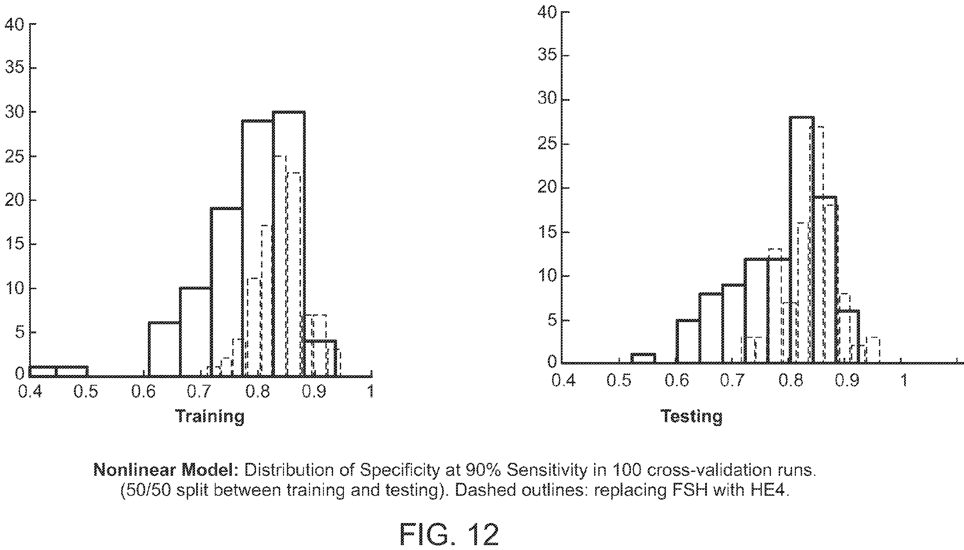

Figure 12 shows plots depicting Distribution of Specificity for a nonlinear model at 90% Sensitivity in 100 cross-validation runs (50/50 split between training (left panel) and testing (right panel). Red hollow bars: Red Outline: CA125, prealbumin, IGFBP2, IL6, and HE4. Blue: Panel: CA125, prealbumin, IGFBP2, IL6, and FSH

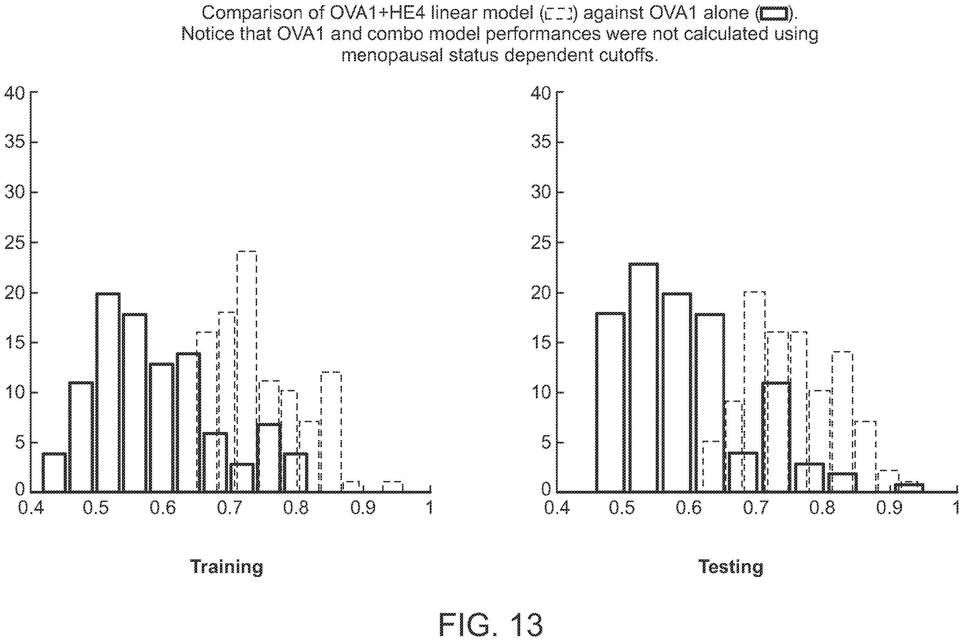

Figure 13 shows plots depicting Distribution of Specificity for panel of markers described in the prior art and in clinical use for characterizing ovarian cancer: Transthyretin (TT or prealbumin), Apolipoprotein A-1 (Apo A-1), beta 2-Microglobulin (beta 2M), Transferrin (Tfr) and Cancer Antigen 125 (CA 125 II) +HE4 linear model at 90% Sensitivity in 100 cross-validation runs (50/50 split between training (left panel) and testing (right panel). Red hollow bars: Transthyretin (TT or prealbumin), Apolipoprotein A-1 (Apo A-1), beta 2-Microglobulin (beta 2M), Transferrin (Tfr) and Cancer Antigen 125 (CA 125 II) +HE4 linear model; Solid blue: Transthyretin (TT or prealbumin), Apolipoprotein A-1 (Apo A-1), beta 2-Microglobulin (beta 2M), Transferrin (Tfr) and Cancer Antigen 125 (CA 125 II). Cutoffs are selected to maintain 90% sensitivity.

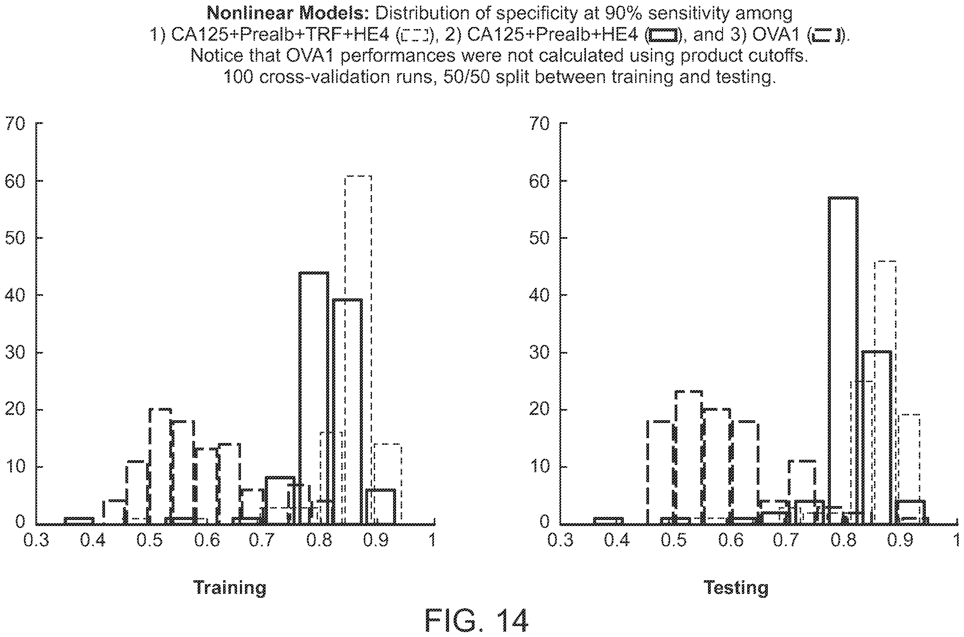

Figure 14 shows plots depicting Distributions of Specificities for CA125+Preabl+TRF+HE4 and CA125+Preabl+TRF+HE4 nonlinear models at 90% Sensitivity in 100 cross-validation runs (50/50 split between training (left panel) and testing (right panel). Red: CA125+Preabl+TRF+HE4; Blue: CA125+Preabl+TRF+HE4; Green: Transthyretin (TT or prealbumin), Apolipoprotein A-1 (Apo A-1), beta 2-Microglobulin (beta 2M), Transferrin (Tfr) and Cancer Antigen 125 (CA 125 II).

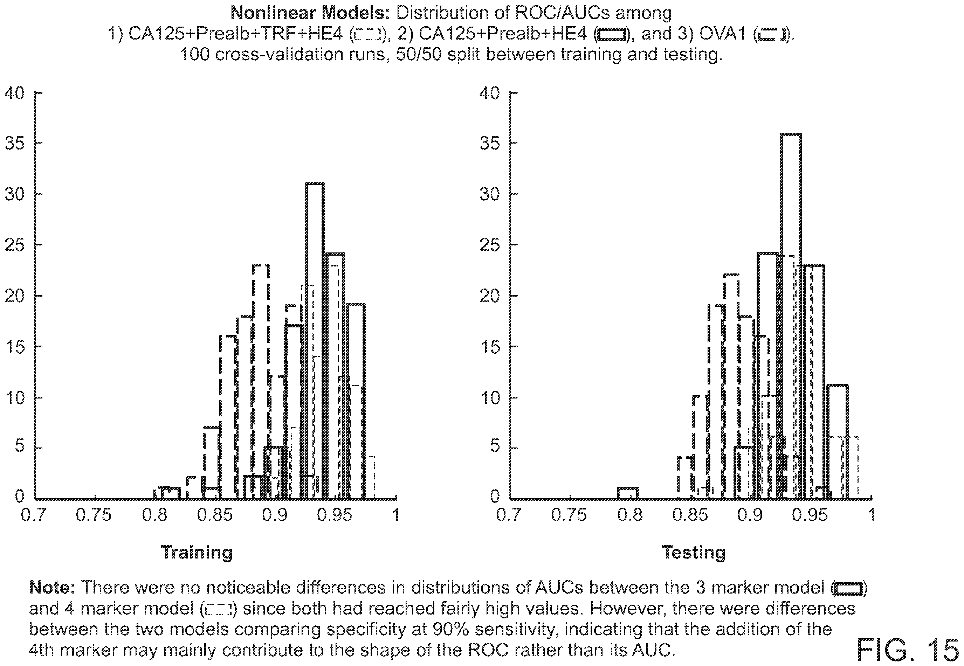

Figure 15 shows plots depicting Distributions of ROC/AUCs for CA125+Preabl+TRF+HE4 and CA125+Preabl+TRF+HE4 nonlinear models at 90% Sensitivity in 100 cross-validation runs (50/50 split between training (left panel) and testing (right panel). Red: CA125+Preabl+TRF+HE4; Blue: CA125+Preabl+TRF+HE4; Green: Transthyretin (TT or prealbumin), Apolipoprotein A-1 (Apo A-1), beta 2-Microglobulin (beta 2M), Transferrin (Tfr) and Cancer Antigen 125 (CA 125 II).

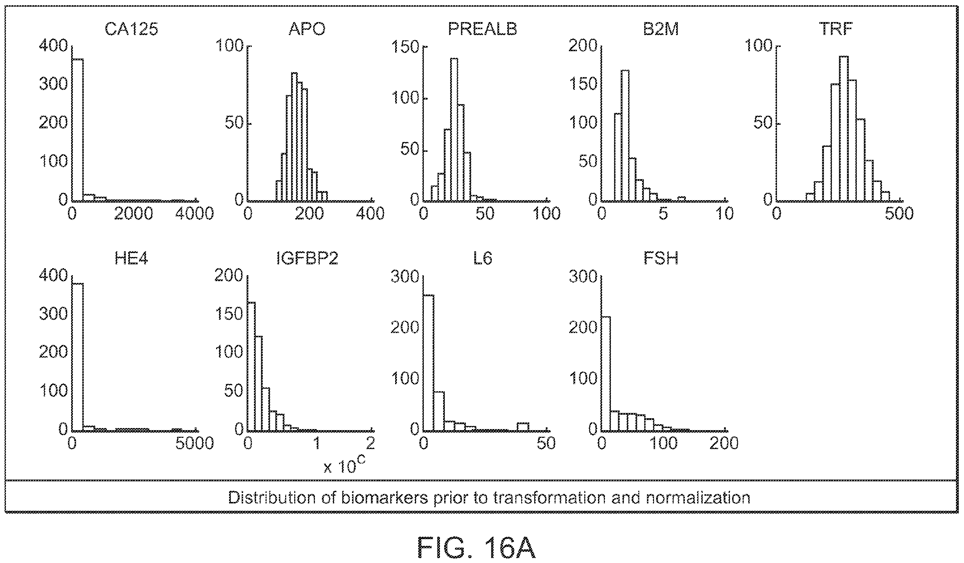

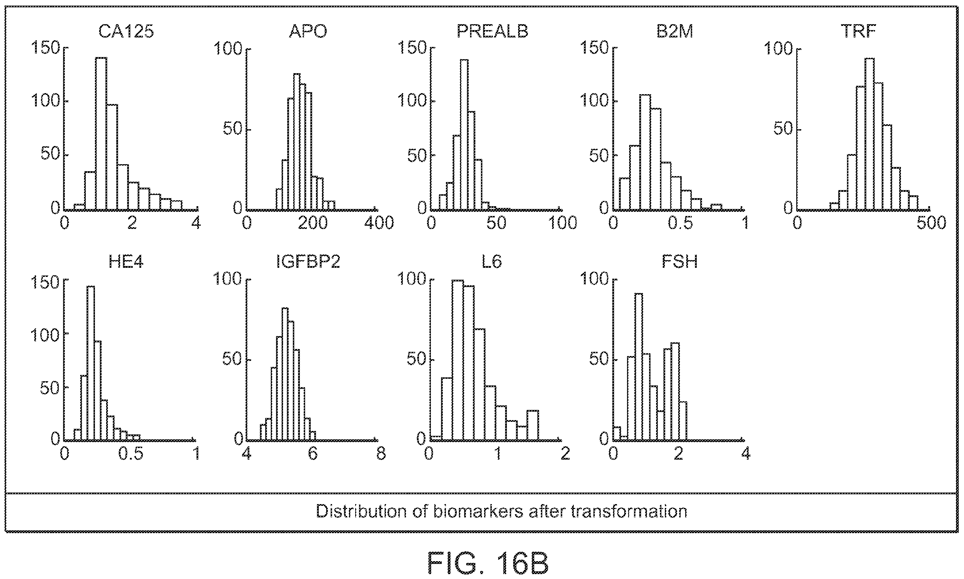

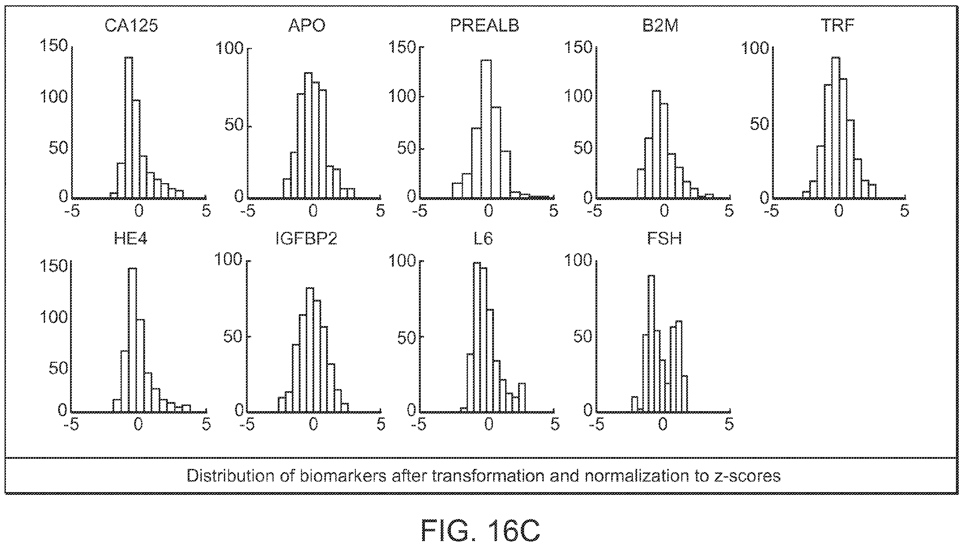

Figures 16A-16C shows plots depicting Distribution of biomarkers before and after preprocessing and normalization. Figure 16A is a plot depicting Distribution of biomarkers prior to transformation and normalization Figure 16B is a plot depicting Distribution of biomarkers after transformation. Figure 16C is a plot depicting Distribution of biomarkers after transformation and normalization to z-scores

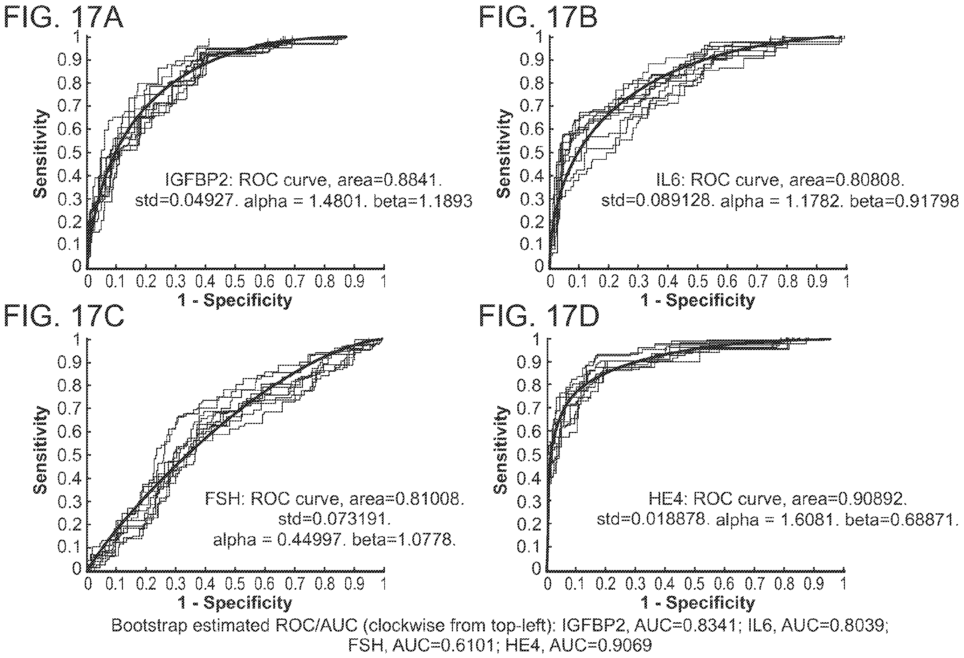

Figures 17A-17D shows plots depicting Bootstrap estimated ROC/AUC. Figure 17A is a plot depicting Bootstrap estimated ROC/AUC for IGFBP2, AUC=0.8341. Figure 17B is a plot depicting Bootstrap estimated ROC/AUC for IL6, AUC=0.8039. Figure 17C is a plot depicting Bootstrap estimated ROC/AUC for FSH, AUC=0.6101. Figure 17D is a plot depicting Bootstrap estimated ROC/AUC for HE4, AUC=0.9069.

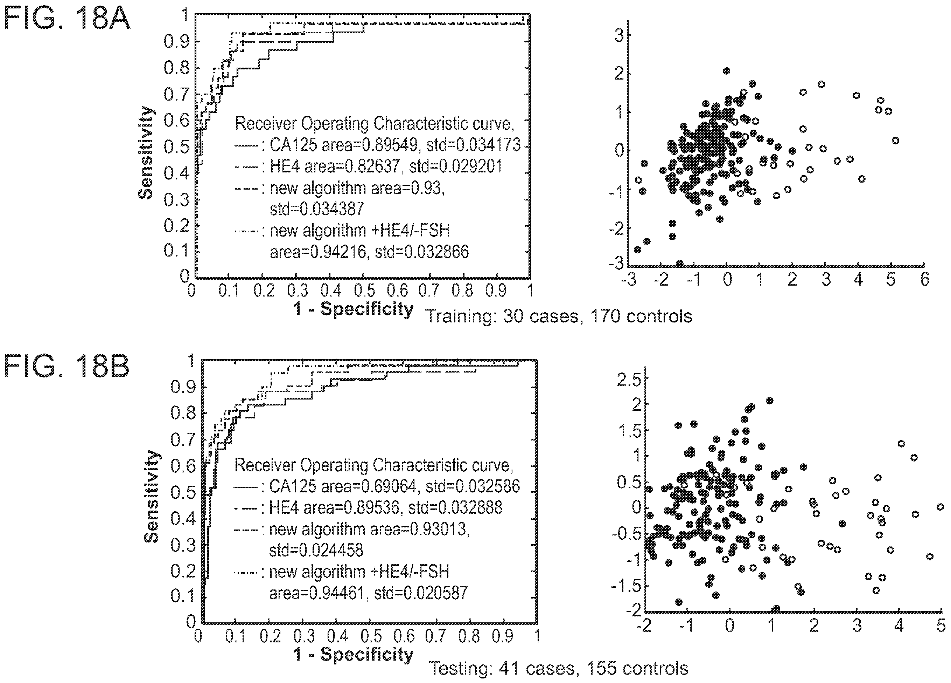

Figures 18A and 18B depict receiver operating characteristic curves for a single training/test run using a linear model. Figure 18A depicts receiver operating characteristic curves for a training run using a linear model. Figure 18B depicts receiver operating characteristic curves for a test run using a linear model.
Figure 19 provides exemplary sequences of human Insulin-like growth factor binding protein (IGFBP2); Interleukin 6 (IL6); Follicle-stimulating hormone (FSH); Human Epididymis Protein 4 (HE4); Cancer Antigen 125 (CA 125); Transthyretin (prealbumin); Transferrin; apolipoprotein A-1 (ApoA1), and β2-microglobulin (B2MG) polypeptides.

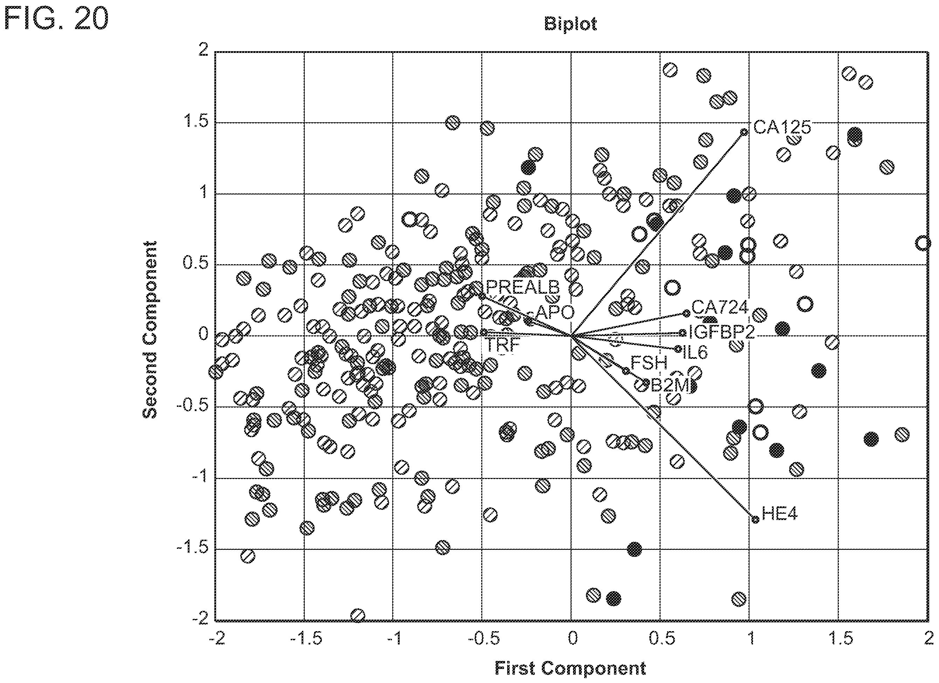

Figure 20 is a biplot of supervised component analysis results using all ten (10) biomarkers (CA125, APOA1, PREALB, B2M, TRF, HE4, IGFBP2, IL6, FSH, TAG 72/CA724). Hollow red circles: training samples for cancer; hollow green circles: training samples for benign; Solid red circles: testing samples for cancer; solid green circles: testing samples for benign. Markers that are to the right of the center vertical axis (First component) are upregulated in ovarian cancer. Markers to left of the center axis are down regulated. The length of the line is an indication of the contribution of each marker to the characterization of ovarian cancer. Because markers CA724, IGFBP2, and IL6 have equal contributions and are all upregulated, these markers are typically interchangeable in any of the panels where they are listed. As shown, Prealbumin, CA125 and HE4 provide one preferred panel for characterizing ovarian cancer. Any one of IL6, IGFBP2 or CA 724 may be added to this panel.

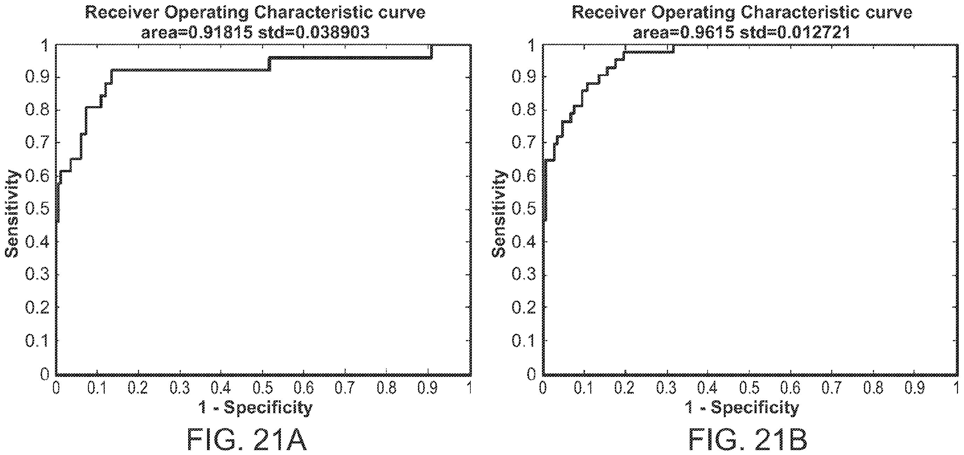

Figure 21 shows receiver-operating characteristic (ROC) curves of a linear classifier (represented by the x-axis in figure 1) on the training data (left) and testing data (right) set forth in Figure 20.

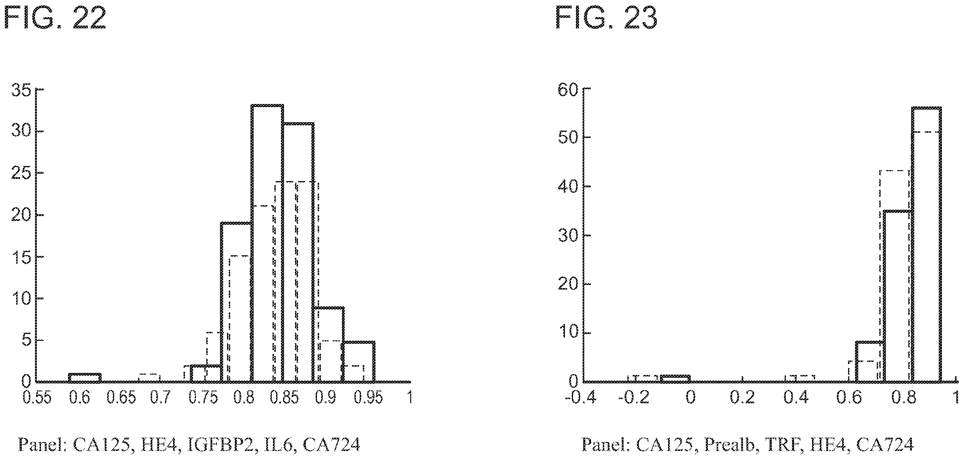

Figure 22 is a graph showing results using CA125, HE4, IGFBP2, IL6, CA724 (biomarkers with the best ranking in bootstrap feature selection). These results were obtained using a Linear Model. Shown is the distribution of Specificity at 90% Sensitivity in 100 cross-validation runs. (50/50 split between training and testing). Blue: training, Red: cross-validation.
Figure 23 is a graph showing results using CA125, Prealb, TRF, HE4, CA724. These results were obtained using a Linear Model. Shown is the distribution of Specificity at 90% Sensitivity in 100 cross-validation runs. (50/50 split between training and testing). Blue: training, Red: cross-validation.

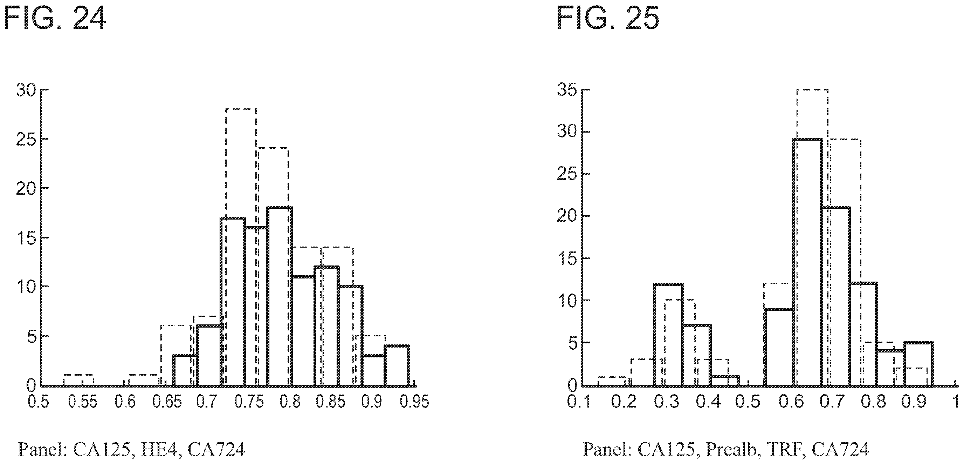

Figure 24 is a graph showing results using markers CA125, HE4, CA724. These results were obtained using a Linear Model. Shown is the distribution of Specificity at 90% Sensitivity in 100 cross-validation runs. (50/50 split between training and testing). Blue: training, Red: cross-validation.
Figure 25 is a graph showing results using markers CA125, Prealb, TRF, CA724 (without HE4). These results were obtained using a Linear Model. Shown is the distribution of Specificity at 90% Sensitivity in 100 cross-validation runs. (50/50 split between training and testing). Blue: training, Red: cross-validation.

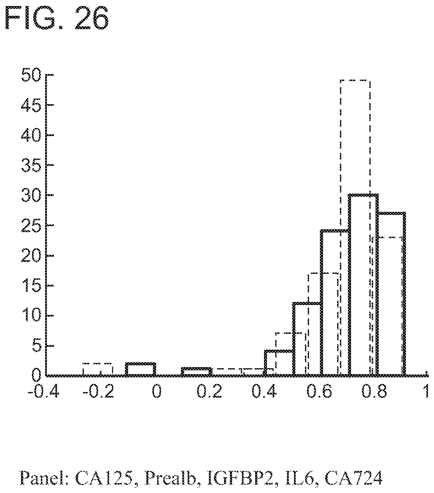

Figure 26 is a graph showing results using markers CA125, Prealb, IGFBP2, IL6, CA724 (without HE4). These results were obtained using a Linear Model. Shown is the distribution of Specificity at 90% Sensitivity in 100 cross-validation runs. (50/50 split between training and testing). Blue: training, Red: cross-validation.

### DETAILED DESCRIPTION OF THE INVENTION

Disclosed herein are panels of biomarkers and the use of such panels for characterizing ovarian cancer.

The invention is based, at least in part, on the discovery that panels of the invention advantageously enhance specificity (e.g., to about mean/median 70%, 75%, 80%, 85%, 90%) at a sensitivity of 90% or 95% and reduce false positives identified by conventional panels of biomarkers.

In particular, the invention relates to the in vitro use of a panel for pre-operatively assessing the risk of a subject having an adnexal mass as having ovarian cancer, wherein the panel consists of the following markers: CA125, Prealbumin, Transferrin, and HE4.

The panel can be used for characterizing ovarian cancer (e.g., assessing risk of malignancy, diagnosis, prognosis). In particular, the use of such panels provides methods for pre-surgically characterizing a pelvic mass in a subject and identifying subjects that might benefit from evaluation/treatment by a gynecological oncologist.

### Ovarian Cancer

Ovarian tumors are being detected with increasing frequency in women of all ages, yet there is no standardized or reliable method to determine which are malignant prior to surgery. In 1994, the National Institutes of Health (NIH) released a consensus statement indicating that women with ovarian masses having been identified preoperatively as having a significant risk of ovarian cancer should be given the option of having their surgery performed by a gynecologic oncologist. At present, the National Comprehensive Cancer Network (NCCN), the Society of Gynecologic Oncologists (SGO), SOGC clinical practice guidelines, Standing Subcommittee on Cancer of the Medical Advisory Committee, and several other published statements, all recommend that women with ovarian cancer be under the care of a gynecologic oncologist (GO).

Recent publications on breast, bladder, gastrointestinal, and ovarian cancers have reported improved outcome when cancer management involves a surgical specialist. In addition, a recent meta-analysis of 18 ovarian cancer studies found that the early involvement of a gynecologic oncologist, rather than a general surgeon or general gynecologist, improved patient outcomes. The authors concluded: 1) subjects with early stage disease are more likely to have comprehensive surgical staging, facilitating appropriate adjuvant chemotherapy, 2) subjects with advanced disease are more likely to receive optimal cytoreductive surgery, and 3) subjects with advanced disease have an improved median and overall 5-year survival. Despite the availability of this important information, only a fraction of women with malignant ovarian tumors (an estimated 33%) are referred to a gynecologic oncologist for the primary surgery. Based on reported patterns of care for ovarian cancer management, the majority of women in the United States may not be receiving optimal care for this disease.

The decision for operative removal of an ovarian tumor, and whether a generalist or specialist should perform the surgery, is based on interpretations of physical examination, imaging studies, laboratory tests, and clinical judgment. Pelvic examination alone is inadequate to reliably detect or differentiate ovarian tumors, particularly in early stages when ovarian cancer treatment is most successful. Examination has also been eliminated from the Prostate, Lung, Colorectal and Ovarian cancer screening trial algorithm. Pelvic ultrasound is clinically useful and the least expensive imaging modality, but has limitations in consistently identifying malignant tumors. In general, nearly all unilocular cysts are benign, whereas complex cystic tumors with solid components or internal papillary projections are more likely to be malignant. CA 125 has been used alone or in conjunction with other tests in an effort to establish risk of malignancy. Unfortunately, CA 125 has low sensitivity (50%) in early stage ovarian cancers, and low specificity resultant from numerous false positives in both pre- and postmenopausal women.

The American College of Obstetrics and Gynecology (ACOG) and the SGO have published referral guidelines for patients with a pelvic mass. These guidelines include: patient age, serum CA 125 level, physical examination, imaging results, and family history. This referral strategy has been evaluated both retrospectively and prospectively. In a single institution review, Dearking and colleagues concluded that the guidelines were useful in predicting advanced stage ovarian cancer, but "performed poorly in identifying early-stage disease, especially in premenopausal women, primarily due to lack of early markers and signs of ovarian cancer".

### BIOMARKERS

The term "biomarker" as used herein is to be understood as an organic biomolecule that is differentially present in a sample taken from a subject of one phenotypic status (e.g., having a disease) as compared with another phenotypic status (e.g., not having the disease). A biomarker is differentially present between different phenotypic statuses if the mean or median expression level of the biomarker in the different groups is calculated to be statistically significant. Common tests for statistical significance include, among others, t-test, ANOVA, Kruskal-Wallis, Wilcoxon, Mann-Whitney and odds ratio. Biomarkers, alone or in combination, provide measures of relative risk that a subject belongs to one phenotypic status or another. Therefore, they are useful as markers for characterizing a disease.

### BIOMARKERS FOR OVARIAN CANCER

Disclosed herein is a panel of polypeptide biomarkers that are differentially present in subjects having ovarian cancer, in particular, a benign vs. malignant pelvic mass. The biomarkers are differentially present depending on ovarian cancer status, including, subjects having ovarian cancer vs. subjects that do not have ovarian cancer.

The biomarker panel disclosed herein comprises one or more of the biomarkers presented in the following Table 1.

**Table 1**

| **Biomarker** | **Differential Regulation in ovarian cancer** |
|---|---|
| ApoA1 | Decreased |
| Beta2 Microglobulin (B2M) | Increased |
| Insulin-like growth factor binding protein (IGFBP2) | Increased |
| Interleukin 6 (IL6) | Increased |
| Follicle-stimulating hormone (FSH) | Increased |
| Human Epididymis Protein 4 (HE4) | Increased |
| Cancer Antigen 125 (CA 125) | Increased |
| Transthyretin (prealbumin) | Decreased |
| Transferrin | Decreased |
| TAG 72/CA 72-4 | Increased |

As would be understood, references herein to a biomarker of Table 1, a panel of biomarkers, or other similar phrase indicates one or more of the biomarkers set forth in Table 1 or otherwise described herein.

A biomarker may be detected in a biological sample of the subject (e.g., tissue, fluid), including, but not limited to, blood, blood serum, plasma, saliva, urine, ascites, cyst fluid, a homogenized tissue sample (e.g., a tissue sample obtained by biopsy), a cell isolated from a patient sample, and the like.

Disclosed herein are panels comprising isolated biomarkers. The biomarkers can be isolated from biological fluids, such as urine or serum. They can be isolated by any method known in the art. For example, this isolation can be accomplished using the mass and/or binding characteristics of the markers. For example, a sample comprising the biomolecules can be subject to chromatographic fractionation and subject to further separation by, *e.g.,* acrylamide gel electrophoresis. Knowledge of the identity of the biomarker also allows their isolation by immunoaffinity chromatography. By "isolated biomarker" is meant at least 60%, by weight, free from proteins and naturally-occurring organic molecules with which the marker is naturally associated. Preferably, the preparation is at least 75%, more preferably 80, 85, 90 or 95% pure or at least 99%, by weight, a purified marker.

### Insulin-like growth factor binding protein (IGFBP2)

One exemplary biomarker present in the panel is IGFBP2. IGFBP2 is a 328 amino acid protein (NCBI Accession number NP_000588). The amino acid sequence of an exemplary IGFBP2 polypeptide is set forth in Figure 19. Antibodies to IGFBP2 can be made using any method well known in the art, or can be purchased from, for example, Santa Cruz Biotechnology, Inc. (e.g., Catalog Number sc-130070) (www.scbt.com, Santa Cruz, CA). IGFBP2 is upregulated in subjects with ovarian cancer as compared to subjects that do not have ovarian cancer.

### Interleukin 6 (IL6)

One exemplary biomarker present in the panel is IL6. IL6 is a 212 amino acid protein (NCBI Accession number NP_000591). The amino acid sequence of an exemplary IL6 polypeptide is set forth in Figure 19. Antibodies to IL6 can be made using any method well known in the art, or can be purchased from, for example, Santa Cruz Biotechnology, Inc. (e.g., Catalog Number sc-1265) (www.scbt.com, Santa Cruz, CA). IL6 is upregulated in subjects with ovarian cancer as compared to subjects that do not have ovarian cancer.

### Follicle-stimulating hormone (FSH)

One exemplary biomarker present in the panel used in the context of the invention is FSH. FSH is a 128 amino acid protein (NCBI Accession number NP_000501). The amino acid sequence of an exemplary FSH polypeptide is set forth in Figure 19. Antibodies to FSH can be made using any method well known in the art, or can be purchased from, for example, Santa Cruz Biotechnology, Inc. (e.g., Catalog Number sc-57149) (www.scbt.com, Santa Cruz, CA). FSH is upregulated in subjects with ovarian cancer as compared to subjects that do not have ovarian cancer.

### Human Epididymis Protein 4 (HE4)

One exemplary biomarker present in the panel used in the context of the invention is HE4. HE4 is a 124 amino acid protein (NCBI Accession number NP_006094). The amino acid sequence of an exemplary HE4 polypeptide is set forth in Figure 19. Antibodies to HE4 can be made using any method well known in the art, or can be purchased from, for example, Santa Cruz Biotechnology, Inc. (Catalog Number sc-27570) (www.scbt.com, Santa Cruz, CA). HE4 is upregulated in subjects with ovarian cancer as compared to subjects that do not have ovarian cancer.

### Cancer Antigen 125 (CA 125)

One exemplary biomarker present in the panel used in the context of the invention is CA 125. CA 125 is a 22152 amino acid protein (Swiss-Prot Accession number Q8WXI7). The amino acid sequence of an exemplary CA 125 polypeptide is set forth in Figure 19. Antibodies to CA 125 can be made using any method well known in the art, or can be purchased from, for example, Santa Cruz Biotechnology, Inc. (Catalog Number sc-52095) (www.scbt.com, Santa Cruz, CA). CA 125 is upregulated in subjects with ovarian cancer as compared to subjects that do not have ovarian cancer.

### Transthyretin (Prealbumin)

Another exemplary biomarker present in the panel used in the context of the invention is a form of pre-albumin, also referred to herein as transthyretin. Transthyretin is a 147 amino acid protein (Swiss Prot Accession number P02766). The amino acid sequence of an exemplary transthyretin polypeptide is set forth in Figure 19. Antibodies to transthyretin can be made using any method well known in the art, or can be purchased from, for example, Santa Cruz Biotechnology, Inc. (Catalog Number sc-13098) (www.scbt.com, Santa Cruz, CA). Transthyretin is downregulated in subjects with ovarian cancer as compared to subjects that do not have ovarian cancer.

### Transferrin

Transferrin is another exemplary biomarker used in the context of the panel of biomarkers of the invention. Transferrin is a 698 amino acid protein (UniProtKB/TrEMBL Accession number Q06AH7). The amino acid sequence of an exemplary transferring polypeptide is set forth in Figure 19. Antibodies to transferrin can be made using any method well known in the art, or can be purchased from, for example, Santa Cruz Biotechnology, Inc. (Catalog Number sc-52256) (www.scbt.com, Santa Cruz, CA). Transferrin is downregulated in subjects with ovarian cancer as compared to subjects that do not have ovarian cancer.

### Apolipoprotein A1

Apolipoprotein A1, also referred to herein as "Apo A1" is another exemplary biomarker. Apo A1 is a 267 amino acid protein (Swiss Prot Accession number P02647). The amino acid sequence of an exemplary Apo A1 is set forth in Figure 19. Antibodies to Apolipoprotein A1 can be made using any method well known in the art, or can be purchased from, for example, Santa Cruz Biotechnology, Inc. (Catalog Number sc-130503) (www.scbt.com, Santa Cruz, CA). Apo A1 is downregulated in subjects with ovarian cancer as compared to subjects that do not have ovarian cancer.

### β-2 microglobulin

A further exemplary biomarker is β2-microglobulin. β2-microglobulin is described as a biomarker for ovarian cancer in US provisional patent publication 60/693,679, filed June 24, 2005 (Fung et al.). The mature form of β2-microglobulin is a 99 amino acid protein derived from an 119 amino acid precursor (GI:179318; SwissProt Accession No. P61769). The amino acid sequence of an exemplary β-2-microglobulin polypeptide is set forth in Figure 19. The mature form of β-2-microglobulin consist of residues 21-119 of the β-2-microglobulin set forth in Figure 19. β2-microglobulin is recognized by antibodies. Such antibodies can be made using any method well known in the art, and can also be commercially purchased from, e.g., Abcam (catalog AB759) (www.abcam.com, Cambridge, MA). β2-microglobulin is upregulated in subjects with ovarian cancer as compared to subjects that do not have ovarian cancer.

### BIOMARKERS AND DIFFERENT FORMS OF A PROTEIN

Proteins frequently exist in a sample in a plurality of different forms. These forms can result from pre- and/or post-translational modification. Pre-translational modified forms include allelic variants, splice variants and RNA editing forms. Post-translationally modified forms include forms resulting from proteolytic cleavage (e.g., cleavage of a signal sequence or fragments of a parent protein), glycosylation, phosphorylation, lipidation, oxidation, methylation, cysteinylation, sulphonation and acetylation. When detecting or measuring a protein in a sample, any or all of the forms may be measured to determine the level of biomarker or a form of interest is measured. The ability to differentiate between different forms of a protein depends upon the nature of the difference and the method used to detect or measure the protein. For example, an immunoassay using a monoclonal antibody will detect all forms of a protein containing the epitope and will not distinguish between them. However, a sandwich immunoassay that uses two antibodies directed against different epitopes on a protein will detect all forms of the protein that contain both epitopes and will not detect those forms that contain only one of the epitopes. Distinguishing different forms of an analyte or specifically detecting a particular form of an analyte is referred to as "resolving" the analyte.

Mass spectrometry is a particularly powerful methodology to resolve different forms of a protein because the different forms typically have different masses that can be resolved by mass spectrometry. Accordingly, if one form of a protein is a superior biomarker for a disease than another form of the biomarker, mass spectrometry may be able to specifically detect and measure the useful form where traditional immunoassay fails to distinguish the forms and fails to specifically detect to useful biomarker.

One useful methodology combines mass spectrometry with immunoassay. For example, a biospecific capture reagent (e.g., an antibody, aptamer, Affibody, and the like that recognizes the biomarker and other forms of it) is used to capture the biomarker of interest. The biospecific capture reagent can be bound to a solid phase, such as a bead, a plate, a membrane or an array. After unbound materials are washed away, the captured analytes are detected and/or measured by mass spectrometry. This method will also result in the capture of protein interactors that are bound to the proteins or that are otherwise recognized by antibodies and that, themselves, can be biomarkers. Various forms of mass spectrometry are useful for detecting the protein forms, including laser desorption approaches, such as traditional MALDI or SELDI, electrospray ionization, and the like.

Thus, when reference is made herein to detecting a particular protein or to measuring the amount of a particular protein, it means detecting and measuring the protein with or without resolving various forms of protein. For example, the step of "detecting β-2 microglobulin" includes measuring β-2 microglobulin by means that do not differentiate between various forms of the protein (e.g., certain immunoassays) as well as by means that differentiate some forms from other forms or that measure a specific form of the protein.

### DETECTION OF BIOMARKERS FOR OVARIAN CANCER

The biomarkers used in the context of this invention can be detected by any suitable method. The methods described herein can be used individually or in combination for a more accurate detection of the biomarkers (e.g., biochip in combination with mass spectrometry, immunoassay in combination with mass spectrometry, and the like).

Detection paradigms that can be employed in the context of the invention include, but are not limited to, optical methods, electrochemical methods (voltametry and amperometry techniques), atomic force microscopy, and radio frequency methods, *e.g*., multipolar resonance spectroscopy. Illustrative of optical methods, in addition to microscopy, both confocal and non-confocal, are detection of fluorescence, luminescence, chemiluminescence, absorbance, reflectance, transmittance, and birefringence or refractive index (*e.g*., surface plasmon resonance, ellipsometry, a resonant mirror method, a grating coupler waveguide method or interferometry).

These and additional methods are described *infra.*

### Detection by Immunoassay

In particular embodiments, the biomarkers used in the context of the invention are measured by immunoassay. Immunoassay typically utilizes an antibody (or other agent that specifically binds the marker) to detect the presence or level of a biomarker in a sample. Antibodies can be produced by methods well known in the art, *e.g*., by immunizing animals with the biomarkers. Biomarkers can be isolated from samples based on their binding characteristics. Alternatively, if the amino acid sequence of a polypeptide biomarker is known, the polypeptide can be synthesized and used to generate antibodies by methods well known in the art.

This invention contemplates traditional immunoassays including, for example, Western blot, sandwich immunoassays including ELISA and other enzyme immunoassays, fluorescence-based immunoassays, chemiluminescence,. Nephelometry is an assay done in liquid phase, in which antibodies are in solution. Binding of the antigen to the antibody results in changes in absorbance, which is measured. Other forms of immunoassay include magnetic immunoassay, radioimmunoassay, and real-time immunoquantitative PCR (iqPCR).

Immunoassays can be carried out on solid substrates (e.g., chips, beads, microfluidic platforms, membranes) or on any other forms that supports binding of the antibody to the marker and subsequent detection. A single marker may be detected at a time or a multiplex format may be used. Multiplex immunoanalysis may involve planar microarrays (protein chips) and bead-based microarrays (suspension arrays).

In a SELDI-based immunoassay, a biospecific capture reagent for the biomarker is attached to the surface of an MS probe, such as a pre-activated ProteinChip array. The biomarker is then specifically captured on the biochip through this reagent, and the captured biomarker is detected by mass spectrometry.

### Detection by Biochip

A sample can be analyzed by means of a biochip (also known as a microarray). The polypeptides and nucleic acid molecules disclosed herein are useful as hybridizable array elements in a biochip. Biochips generally comprise solid substrates and have a generally planar surface, to which a capture reagent (also called an adsorbent or affinity reagent) is attached. Frequently, the surface of a biochip comprises a plurality of addressable locations, each of which has the capture reagent bound there.

The array elements are organized in an ordered fashion such that each element is present at a specified location on the substrate. Useful substrate materials include membranes, composed of paper, nylon or other materials, filters, chips, glass slides, and other solid supports. The ordered arrangement of the array elements allows hybridization patterns and intensities to be interpreted as expression levels of particular genes or proteins. Methods for making nucleic acid microarrays are known to the skilled artisan and are described, for example, in U.S. Pat. No. 5,837,832, Lockhart, et al. (Nat. Biotech. 14:1675-1680, 1996), and Schena, et al. (Proc. Natl. Acad. Sci. 93:10614-10619, 1996). Methods for making polypeptide microarrays are described, for example, by Ge (Nucleic Acids Res. 28: e3. i-e3. vii, 2000), MacBeath et al., (Science 289:1760-1763, 2000), Zhu et al.(Nature Genet. 26:283-289), and in U.S. Pat. No. 6,436,665.

### Detection by Protein Biochip

A sample can be analyzed by means of a protein biochip (also known as a protein microarray). Such biochips are useful in high-throughput low-cost screens to identify alterations in the expression or post-translation modification of a polypeptide disclosed herein, or a fragment thereof. A respective protein biochip may bind a biomarker present in a subject sample and detects an alteration in the level of the biomarker. Typically, a protein biochip features a protein, or fragment thereof, bound to a solid support. Suitable solid supports include membranes (e.g., membranes composed of nitrocellulose, paper, or other material), polymer-based films (e.g., polystyrene), beads, or glass slides. For some applications, proteins (e.g., antibodies that bind a marker disclosed herein) are spotted on a substrate using any convenient method known to the skilled artisan (e.g., by hand or by inkjet printer).

The protein biochip can be hybridized with a detectable probe. Such probes can be polypeptide, nucleic acid molecules, antibodies, or small molecules. For some applications, polypeptide and nucleic acid molecule probes are derived from a biological sample taken from a patient, such as a bodily fluid (such as blood, blood serum, plasma, saliva, urine, ascites, cyst fluid, and the like); a homogenized tissue sample (e.g., a tissue sample obtained by biopsy); or a cell isolated from a patient sample. Probes can also include antibodies, candidate peptides, nucleic acids, or small molecule compounds derived from a peptide, nucleic acid, or chemical library. Hybridization conditions (e.g., temperature, pH, protein concentration, and ionic strength) are optimized to promote specific interactions. Such conditions are known to the skilled artisan and are described, for example, in Harlow, E. and Lane, D., Using Antibodies : A Laboratory Manual. 1998, New York: Cold Spring Harbor Laboratories. After removal of non-specific probes, specifically bound probes are detected, for example, by fluorescence, enzyme activity (e.g., an enzyme-linked calorimetric assay), direct immunoassay, radiometric assay, or any other suitable detectable method known to the skilled artisan.

Many protein biochips are described in the art. These include, for example, protein biochips produced by Ciphergen Biosystems, Inc. (Fremont, CA), Zyomyx (Hayward, CA), Packard BioScience Company (Meriden, CT), Phylos (Lexington, MA), Invitrogen (Carlsbad, CA), Biacore (Uppsala, Sweden) and Procognia (Berkshire, UK). Examples of such protein biochips are described in the following patents or published patent applications: U.S. Patent Nos. 6,225,047; 6,537,749; 6,329,209; and 5,242,828; PCT International Publication Nos. WO 00/56934; WO 03/048768; and WO 99/51773.

### Detection by Nucleic Acid Biochip

A sample can be analyzed by means of a nucleic acid biochip (also known as a nucleic acid microarray). To produce a nucleic acid biochip, oligonucleotides may be synthesized or bound to the surface of a substrate using a chemical coupling procedure and an inkjet application apparatus, as described in PCT application WO95/251116 (Baldeschweiler et al.). Alternatively, a gridded array may be used to arrange and link cDNA fragments or oligonucleotides to the surface of a substrate using a vacuum system, thermal, UV, mechanical or chemical bonding procedure.

A nucleic acid molecule (e.g. RNA or DNA) derived from a biological sample may be used to produce a hybridization probe as described herein. The biological samples are generally derived from a patient, e.g., as a bodily fluid (such as blood, blood serum, plasma, saliva, urine, ascites, cyst fluid, and the like); a homogenized tissue sample (e.g., a tissue sample obtained by biopsy); or a cell isolated from a patient sample. For some applications, cultured cells or other tissue preparations may be used. The mRNA is isolated according to standard methods, and cDNA is produced and used as a template to make complementary RNA suitable for hybridization Such methods are well known in the art. The RNA is amplified in the presence of fluorescent nucleotides, and the labeled probes are then incubated with the microarray to allow the probe sequence to hybridize to complementary oligonucleotides bound to the biochip.

Incubation conditions are adjusted such that hybridization occurs with precise complementary matches or with various degrees of less complementarity depending on the degree of stringency employed. For example, stringent salt concentration will ordinarily be less than about 750 mM NaCl and 75 mM trisodium citrate, less than about 500 mM NaCl and 50 mM trisodium citrate, or less than about 250 mM NaCl and 25 mM trisodium citrate. Low stringency hybridization can be obtained in the absence of organic solvent, e.g., formamide, while high stringency hybridization can be obtained in the presence of at least about 35% formamide, and most preferably at least about 50% formamide. Stringent temperature conditions will ordinarily include temperatures of at least about 30°C, of at least about 37°C., or of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. Hybridization may occur at 30°C in 750 mM NaCl, 75 mM trisodium citrate, and 1% SDS. Alternatively, hybridization will occur at 37°C in 500 mM NaCl, 50 mM trisodium citrate, 1% SDS, 35% formamide, and 100 µg/ml denatured salmon sperm DNA (ssDNA). Further alternatively, hybridization will occur at 42°C in 250 mM NaCl, 25 mM trisodium citrate, 1% SDS, 50% formamide, and 200 µg/ml ssDNA. Useful variations on these conditions will be readily apparent to those skilled in the art.

The removal of nonhybridized probes may be accomplished, for example, by washing. The washing steps that follow hybridization can also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, of at least about 42°C, or of at least about 68°C. Wash steps may occur at 25°C in 30 mM NaCl, 3 mM trisodium citrate, and 0.1% SDS. Alternatively, wash steps will occur at 42 C in 15 mM NaCl, 1.5 mM trisodium citrate, and 0.1% SDS. Further alternatively, wash steps will occur at 68 C in 15 mM NaCl, 1.5 mM trisodium citrate, and 0.1% SDS. Additional variations on these conditions will be readily apparent to those skilled in the art.

Detection system for measuring the absence, presence, and amount of hybridization for all of the distinct nucleic acid sequences are well known in the art. For example, simultaneous detection is described in Heller et al., Proc. Natl. Acad. Sci. 94:2150-2155, 1997. In one example, a scanner is used to determine the levels and patterns of fluorescence.

### Detection by Mass Spectrometry

The biomarkers used in the context of this invention can be detected by mass spectrometry (MS). Mass spectrometry is a well-known tool for analyzing chemical compounds that employs a mass spectrometer to detect gas phase ions. Mass spectrometers are well known in the art and include, but are not limited to, time-of-flight, magnetic sector, quadrupole filter, ion trap, ion cyclotron resonance, electrostatic sector analyzer and hybrids of these. The method may be performed in an automated (Villanueva, et al., Nature Protocols (2006) 1(2):880-891) or semi-automated format. This can be accomplished, for example with the mass spectrometer operably linked to a liquid chromatography device (LC-MS/MS or LC-MS) or gas chromatography device (GC-MS or GC-MS/MS). Methods for performing mass spectrometry are well known and have been disclosed, for example, in US Patent Application Publication Nos: 20050023454; 20050035286; US Patent No. 5,800,979 and the references disclosed therein.

### Laser Desorption/Ionization

The mass spectrometer can be a laser desorption/ionization mass spectrometer. In laser desorption/ionization mass spectrometry, the analytes are placed on the surface of a mass spectrometry probe, a device adapted to engage a probe interface of the mass spectrometer and to present an analyte to ionizing energy for ionization and introduction into a mass spectrometer. A laser desorption mass spectrometer employs laser energy, typically from an ultraviolet laser, but also from an infrared laser, to desorb analytes from a surface, to volatilize and ionize them and make them available to the ion optics of the mass spectrometer. The analysis of proteins by LDI can take the form of MALDI or of SELDI. The analysis of proteins by LDI can take the form of MALDI or of SELDI.

Laser desorption/ionization in a single time of flight instrument typically is performed in linear extraction mode. Tandem mass spectrometers can employ orthogonal extraction modes.

### Matrix-assisted Laser Desorption/Ionization (MALDI) and Electrospray Ionization (ESI)

The mass spectrometric technique for use in the invention can be matrix-assisted laser desorption/ionization (MALDI) or electrospray ionization (ESI). For example, the procedure is MALDI with time of flight (TOF) analysis, known as MALDI-TOF MS. This involves forming a matrix on a membrane with an agent that absorbs the incident light strongly at the particular wavelength employed. The sample is excited by UV or IR laser light into the vapor phase in the MALDI mass spectrometer. Ions are generated by the vaporization and form an ion plume. The ions are accelerated in an electric field and separated according to their time of travel along a given distance, giving a mass/charge (m/z) reading which is very accurate and sensitive. MALDI spectrometers are well known in the art and are commercially available from, for example, PerSeptive Biosystems, Inc. (Framingham, Mass., USA).

Magnetic-based serum processing can be combined with traditional MALDI-TOF. Through this approach, improved peptide capture is achieved prior to matrix mixture and deposition of the sample on MALDI target plates. Accordingly, methods of peptide capture can be enhanced through the use of derivatized magnetic bead based sample processing.

MALDI-TOF MS allows scanning of the fragments of many proteins at once. Thus, many proteins can be run simultaneously on a polyacrylamide gel to produce an array of spots on a collecting membrane, and the array may be analyzed. Subsequently, automated output of the results is provided by using an server (e.g., ExPASy) to generate the data in a form suitable for computers.

Other techniques for improving the mass accuracy and sensitivity of the MALDI-TOF MS can be used to analyze the fragments of protein obtained on a collection membrane. These include, but are not limited to, the use of delayed ion extraction, energy reflectors, ion-trap modules, and the like. In addition, post source decay and MS-MS analysis are useful to provide further structural analysis. With ESI, the sample is in the liquid phase and the analysis can be by ion-trap, TOF, single quadrupole, multi-quadrupole mass spectrometers, and the like. The use of such devices (other than a single quadrupole) allows MS-MS or MSⁿ analysis to be performed. Tandem mass spectrometry allows multiple reactions to be monitored at the same time.

Capillary infusion may be employed to introduce the marker to a desired mass spectrometer implementation, for instance, because it can efficiently introduce small quantities of a sample into a mass spectrometer without destroying the vacuum. Capillary columns are routinely used to interface the ionization source of a mass spectrometer with other separation techniques including, but not limited to, gas chromatography (GC) and liquid chromatography (LC). GC and LC can serve to separate a solution into its different components prior to mass analysis. Such techniques are readily combined with mass spectrometry. One variation of the technique is the coupling of high performance liquid chromatography (HPLC) to a mass spectrometer for integrated sample separation/and mass spectrometer analysis.

Quadrupole mass analyzers may also be employed as needed to practice the invention. Fourier-transform ion cyclotron resonance (FTMS) can also be used. It offers high resolution and the ability of tandem mass spectrometry experiments. FTMS is based on the principle of a charged particle orbiting in the presence of a magnetic field. Coupled to ESI and MALDI, FTMS offers high accuracy with errors as low as 0.001%.

### Surface-enhanced laser desorption/ionization (SELDI)

The mass spectrometric technique for use in the invention can be "Surface Enhanced Laser Desorption and Ionization" or "SELDI," as described, for example, in U.S. Patents No. 5,719,060 and No. 6,225,047, both to Hutchens and Yip. This refers to a method of desorption/ionization gas phase ion spectrometry (e.g., mass spectrometry) in which an analyte (here, one or more of the biomarkers) is captured on the surface of a SELDI mass spectrometry probe.

SELDI has also been called "affinity capture mass spectrometry." It also is called "Surface-Enhanced Affinity Capture" or "SEAC". This version involves the use of probes that have a material on the probe surface that captures analytes through a non-covalent affinity interaction (adsorption) between the material and the analyte. The material is variously called an "adsorbent," a "capture reagent," an "affinity reagent" or a "binding moiety." Such probes can be referred to as "affinity capture probes" and as having an "adsorbent surface." The capture reagent can be any material capable of binding an analyte. The capture reagent is attached to the probe surface by physisorption or chemisorption. The probes may have the capture reagent already attached to the surface. Alternatively, the probes can be pre-activated and include a reactive moiety that is capable of binding the capture reagent, e.g., through a reaction forming a covalent or coordinate covalent bond. Epoxide and acyl-imidizole are useful reactive moieties to covalently bind polypeptide capture reagents such as antibodies or cellular receptors. Nitrilotriacetic acid and iminodiacetic acid are useful reactive moieties that function as chelating agents to bind metal ions that interact non-covalently with histidine containing peptides. Adsorbents are generally classified as chromatographic adsorbents and biospecific adsorbents.

"Chromatographic adsorbent" refers to an adsorbent material typically used in chromatography. Chromatographic adsorbents include, for example, ion exchange materials, metal chelators (*e.g*., nitrilotriacetic acid or iminodiacetic acid), immobilized metal chelates, hydrophobic interaction adsorbents, hydrophilic interaction adsorbents, dyes, simple biomolecules (*e.g*., nucleotides, amino acids, simple sugars and fatty acids) and mixed mode adsorbents (*e.g*., hydrophobic attraction/electrostatic repulsion adsorbents).

"Biospecific adsorbent" refers to an adsorbent comprising a biomolecule, *e.g*., a nucleic acid molecule (*e.g*., an aptamer), a polypeptide, a polysaccharide, a lipid, a steroid or a conjugate of these *(e.g.,* a glycoprotein, a lipoprotein, a glycolipid, a nucleic acid (*e.g.,* DNA)-protein conjugate). In certain instances, the biospecific adsorbent can be a macromolecular structure such as a multiprotein complex, a biological membrane or a virus. Examples of biospecific adsorbents are antibodies, receptor proteins and nucleic acids. Biospecific adsorbents typically have higher specificity for a target analyte than chromatographic adsorbents. Further examples of adsorbents for use in SELDI can be found in U.S. Patent No. 6,225,047. A "bioselective adsorbent" refers to an adsorbent that binds to an analyte with an affinity of at least 10⁻⁸ M.

Protein biochips produced by Ciphergen comprise surfaces having chromatographic or biospecific adsorbents attached thereto at addressable locations. Ciphergen's ProteinChip^{®} arrays include NP20 (hydrophilic); H4 and H50 (hydrophobic); SAX-2, Q-10 and (anion exchange); WCX-2 and CM-10 (cation exchange); IMAC-3, IMAC-30 and IMAC-50 (metal chelate);and PS-10, PS-20 (reactive surface with acyl-imidizole, epoxide) and PG-20 (protein G coupled through acyl-imidizole). Hydrophobic ProteinChip arrays have isopropyl or nonylphenoxy-poly(ethylene glycol)methacrylate functionalities. Anion exchange ProteinChip arrays have quaternary ammonium functionalities. Cation exchange ProteinChip arrays have carboxylate functionalities. Immobilized metal chelate ProteinChip arrays have nitrilotriacetic acid functionalities (IMAC 3 and IMAC 30) or O-methacryloyl-N,N-bis-carboxymethyl tyrosine functionalities (IMAC 50) that adsorb transition metal ions, such as copper, nickel, zinc, and gallium, by chelation. Preactivated ProteinChip arrays have acyl-imidizole or epoxide functional groups that can react with groups on proteins for covalent binding.

Such biochips are further described in: U.S. Patent No. 6,579,719 (Hutchens and Yip, "Retentate Chromatography," June 17, 2003); U.S. Patent 6,897,072 (Rich et al., "Probes for a Gas Phase Ion Spectrometer," May 24, 2005); U.S. Patent No. 6,555,813 (Beecher et al., "Sample Holder with Hydrophobic Coating for Gas Phase Mass Spectrometer," April 29, 2003); U.S. Patent Publication No. U.S. 2003 -0032043 A1 (Pohl and Papanu, "Latex Based Adsorbent Chip," July 16, 2002); and PCT International Publication No. WO 03/040700 (Um et al., "Hydrophobic Surface Chip," May 15, 2003); U.S. Patent Application Publication No. US 2003/-0218130 A1 (Boschetti et al., "Biochips With Surfaces Coated With Polysaccharide-Based Hydrogels," April 14, 2003) and U.S. Patent 7,045,366 (Huang et al., "Photocrosslinked Hydrogel Blend Surface Coatings" May 16, 2006).

In general, a probe with an adsorbent surface is contacted with the sample for a period of time sufficient to allow the biomarker or biomarkers that may be present in the sample to bind to the adsorbent. After an incubation period, the substrate is washed to remove unbound material. Any suitable washing solutions can be used; preferably, aqueous solutions are employed. The extent to which molecules remain bound can be manipulated by adjusting the stringency of the wash. The elution characteristics of a wash solution can depend, for example, on pH, ionic strength, hydrophobicity, degree of chaotropism, detergent strength, and temperature. Unless the probe has both SEAC and SEND properties (as described herein), an energy absorbing molecule then is applied to the substrate with the bound biomarkers.

One can capture the biomarkers with a solid-phase bound immuno-adsorbent that has antibodies that bind the biomarkers. After washing the adsorbent to remove unbound material, the biomarkers are eluted from the solid phase and detected by applying to a SELDI biochip that binds the biomarkers and analyzing by SELDI.

The biomarkers bound to the substrates are detected in a gas phase ion spectrometer such as a time-of-flight mass spectrometer. The biomarkers are ionized by an ionization source such as a laser, the generated ions are collected by an ion optic assembly, and then a mass analyzer disperses and analyzes the passing ions. The detector then translates information of the detected ions into mass-to-charge ratios. Detection of a biomarker typically will involve detection of signal intensity. Thus, both the quantity and mass of the biomarker can be determined.

### METHODS

Panels comprising biomarkers as described herein can be used to characterize a pelvic mass in a subject to determine whether the subject should be seen by a general surgeon or should be evaluated and/or treated by a gynecologic oncologist. A panel as disclosed herein can also be used to diagnose or stage an ovarian cancer by determining the molecular profile of the cancer. Panels as disclosed herein can be used to select a course of treatment for a subject. The phrase "ovarian cancer status" includes any distinguishable manifestation of the disease, including non-disease. For example, ovarian cancer status includes, without limitation, the presence or absence of disease (e.g., ovarian cancer v. non-ovarian cancer), the risk of developing disease, i. e. the determination of whether a pelvic mass is malignant of benign. Based on this status, further procedures may be indicated, including additional diagnostic tests or therapeutic procedures or regimens.

The biomarkers used in the context of the invention can be used in diagnostic tests to identify early stage ovarian cancer in a subject.

The correlation of test results with ovarian cancer involves applying a classification algorithm of some kind to the results to generate the status. The classification algorithm may be as simple as determining whether or not the amounts of the markers listed in Table 1 are above or below a particular cut-off number. When multiple biomarkers are used, the classification algorithm may be a linear regression formula. Alternatively, the classification algorithm may be the product of any of a number of learning algorithms described herein.

In the case of complex classification algorithms, it may be necessary to perform the algorithm on the data, thereby determining the classification, using a computer, e.g., a programmable digital computer. In either case, one can then record the status on tangible medium, for example, in computer-readable format such as a memory drive or disk or simply printed on paper. The result also could be reported on a computer screen.

### Biomarkers

Individual biomarkers are useful diagnostic biomarkers. In addition, as described in the examples, it has been found that a specific combination of biomarkers provides greater predictive value of a particular status than any single biomarker alone, or any other combination of previously identified biomarkers. Specifically, the detection of a plurality of biomarkers in a sample can increase the sensitivity, accuracy and specificity of the test.

Each biomarkers described herein can be differentially present in ovarian cancer, and, therefore, each is individually useful in aiding in the determination of ovarian cancer status. A respective in vitro method for reducing the rate of false positive pre-operative ovarian cancer assessment in a subject having an adnexal mass or for pre-operatively assessing the risk of a subject having an adnexal mass as having ovarian cancer involves, first, measuring the level of a panel of markers consisting of CA125, Prealbumin, Transferrin, and HE4 in a biological sample derived from the subject using any method well known in the art, including but not limited to the methods described herein, e.g. capture on a SELDI biochip followed by detection by mass spectrometry and, second, comparing the level of said markers to a reference level. Also disclosed is comparing said level measurement with a diagnostic amount or cut-off that distinguishes a positive ovarian cancer status from a negative ovarian cancer status. The diagnostic amount represents a measured amount of a biomarker above which or below which a subject is classified as having a particular ovarian cancer status. For example, if the biomarker is up-regulated compared to normal during ovarian cancer, then a measured amount above the diagnostic cutoff provides a diagnosis of ovarian cancer. Alternatively, if the biomarker is down-regulated during ovarian cancer, then a measured amount below the diagnostic cutoff provides a diagnosis of ovarian cancer. As is well understood in the art, by adjusting the particular diagnostic cut-off used in an assay, one can increase sensitivity or specificity of the diagnostic assay depending on the preference of the diagnostician. The particular diagnostic cut-off can be determined, for example, by measuring the amount of the biomarker in a statistically significant number of samples from subjects with the different ovarian cancer statuses, as was done here, and drawing the cut-off to suit the diagnostician's desired levels of specificity and sensitivity.

The biomarkers disclosed herein (used alone or in combination) show a statistical difference in different ovarian cancer statuses of at least p ≤ 0.05, p ≤ 10⁻², p ≤ 10⁻³, p ≤ 10⁻⁴, or p ≤ 10⁻⁵. Diagnostic tests that use these biomarkers alone or in combination show a sensitivity and specificity of at least 75%, at least 80%, at least 85%, at least 90%, at least 95%, at least 98%, or about 100%.

### Determining Course (Progression/Remission) of Disease

Also disclosed but not forming part of the present invention are methods for determining the course of disease in a subject. Disease course refers to changes in disease status over time, including disease progression (worsening) and disease regression (improvement). Over time, the amounts or relative amounts (e.g., the pattern) of the biomarkers change. Accordingly, this method involves measuring the panel of biomarkers in a subject at least two different time points, e.g., a first time and a second time, and comparing the change in amounts, if any. The course of disease (e.g., during treatment) is determined based on these comparisons.

### Reporting the Status

Also disclosed is the communication of assay results or diagnoses or both to technicians, physicians or patients, for example. According to one example, computers will be used to communicate assay results or diagnoses or both to interested parties, e.g., physicians and their patients. The assays can be performed or the assay results can be analyzed in a country or jurisdiction which differs from the country or jurisdiction to which the results or diagnoses are communicated.

A diagnosis based on the differential presence or absence in a test subject of the biomarkers of Table 1 can be communicated to the subject as soon as possible after the diagnosis is obtained. The diagnosis may be communicated to the subject by the subject's treating physician. Alternatively, the diagnosis may be sent to a test subject by email or communicated to the subject by phone. A computer may be used to communicate the diagnosis by email or phone. The message may contain results of a diagnostic test may be generated and delivered automatically to the subject using a combination of computer hardware and software which will be familiar to artisans skilled in telecommunications. One example of a healthcare-oriented communications system is described in U.S. Patent Number 6,283,761. All or some of the method steps, including the assaying of samples, diagnosing of diseases, and communicating of assay results or diagnoses, may be carried out in diverse (*e.g*., foreign) jurisdictions.

### Subject Management

Also disclosed is managing subject treatment based on the status. Such management includes referral, for example, to a gynecologic oncologist, or other actions of the physician or clinician subsequent to determining ovarian cancer status. For example, if a physician makes a diagnosis of ovarian cancer, then a certain regime of treatment, such as prescription or administration of therapeutic agent might follow. Alternatively, a diagnosis of non-ovarian cancer or non-ovarian cancer might be followed with further testing to determine a specific disease that might the patient might be suffering from. Also, if the diagnostic test gives an inconclusive result on ovarian cancer status, further tests may be called for.

The diagnosis may be determining if a pelvic mass is benign or malignant. If the diagnosis is malignant, a gynecologic oncologist may be chosen to perform the surgery. In contrast, if the diagnosis is benign, a general surgeon or a gynecologist may be chosen to perform the surgery.

Also disclosed is the communication of assay results or diagnoses or both to technicians, physicians or patients, for example. In some examples, computers will be used to communicate assay results or diagnoses or both to interested parties, e.g., physicians and their patients. Further, the assays can be be performed or the assay results can be analyzed in a country or jurisdiction which differs from the country or jurisdiction to which the results or diagnoses are communicated.

### HARDWARE AND SOFTWARE

In any of the methods described herein, the step of correlating the measurement of the biomarker(s) with ovarian cancer can be performed on general-purpose or speciallyprogrammed hardware or software.

The analysis can be performed by a software classification algorithm. The analysis of analytes by any detection method well known in the art, including, but not limited to the methods described herein, will generate results that are subject to data processing. Data processing can be performed by the software classification algorithm. Such software classification algorithms are well known in the art and one of ordinary skill can readily select and use the appropriate software to analyze the results obtained from a specific detection method.

The analysis can be performed by a computer-readable medium. The computer-readable medium can be non-transitory and/or tangible. For example, the computer readable medium can be volatile memory (*e.g*., random access memory and the like) or non-volatile memory (*e.g*., read-only memory, hard disks, floppy discs, magnetic tape, optical discs, paper table, punch cards, and the like).

For example, analysis of analytes by time-of-flight mass spectrometry generates a time-of-flight spectrum. The time-of-flight spectrum ultimately analyzed typically does not represent the signal from a single pulse of ionizing energy against a sample, but rather the sum of signals from a number of pulses. This reduces noise and increases dynamic range. This time-of-flight data is then subject to data processing. Exemplary software includes, but is not limited to, Ciphergen's ProteinChip^{®} software, in which data processing typically includes TOF-to-M/Z transformation to generate a mass spectrum, baseline subtraction to eliminate instrument offsets and high frequency noise filtering to reduce high frequency noise

Data generated by desorption and detection of biomarkers can be analyzed with the use of a programmable digital computer. The computer program analyzes the data to indicate the number of biomarkers detected, and optionally the strength of the signal and the determined molecular mass for each biomarker detected. Data analysis can include steps of determining signal strength of a biomarker and removing data deviating from a predetermined statistical distribution. For example, the observed peaks can be normalized, by calculating the height of each peak relative to some reference. The reference can be background noise generated by the instrument and chemicals such as the energy absorbing molecule which is set at zero in the scale.

The computer can transform the resulting data into various formats for display. The standard spectrum can be displayed, but in one useful format only the peak height and mass information are retained from the spectrum view, yielding a cleaner image and enabling biomarkers with nearly identical molecular weights to be more easily seen. In another useful format, two or more spectra are compared, conveniently highlighting unique biomarkers and biomarkers that are up- or down-regulated between samples. Using any of these formats, one can readily determine whether a particular biomarker is present in a sample.

Analysis generally involves the identification of peaks in the spectrum that represent signal from an analyte. Peak selection can be done visually, but software is available, for example, as part of Ciphergen's ProteinChip^{®} software package, that can automate the detection of peaks. This software functions by identifying signals having a signal-to-noise ratio above a selected threshold and labeling the mass of the peak at the centroid of the peak signal. Many spectra can be compared to identify identical peaks present in some selected percentage of the mass spectra. One version of this software clusters all peaks appearing in the various spectra within a defined mass range, and assigns a mass (N/Z) to all the peaks that are near the mid-point of the mass (M/Z) cluster.

Software used to analyze the data can include code that applies an algorithm to the analysis of the results (e.g., signal to determine whether the signal represents a peak in a signal that corresponds to a biomarker disclosed herein). The software also can subject the data regarding observed biomarker peaks to classification tree or ANN analysis, to determine whether a biomarker peak or combination of biomarker peaks is present that indicates the status of the particular clinical parameter under examination. Analysis of the data may be "keyed" to a variety of parameters that are obtained, either directly or indirectly, from the mass spectrometric analysis of the sample. These parameters include, but are not limited to, the presence or absence of one or more peaks, the shape of a peak or group of peaks, the height of one or more peaks, the log of the height of one or more peaks, and other arithmetic manipulations of peak height data.

### CLASSIFICATION ALGORITHMS FOR QUALIFYING OVARIAN CANCER STATUS

Data derived from the assays (e.g., ELISA assays) that are generated using samples such as "known samples" can be used to "train" a classification model. A "known sample" is a sample that has been pre-classified. The data that are derived from the spectra and are used to form the classification model can be referred to as a "training data set." Once trained, the classification model can recognize patterns in data derived from spectra generated using unknown samples. The classification model can then be used to classify the unknown samples into classes. This can be useful, for example, in predicting whether or not a particular biological sample is associated with a certain biological condition (*e.g*., diseased versus non-diseased).

The training data set that is used to form the classification model may comprise raw data or pre-processed data. For example, raw data can be obtained directly from time-of-flight spectra or mass spectra, and then may be optionally "pre-processed" as described above.

Classification models can be formed using any suitable statistical classification (or "learning") method that attempts to segregate bodies of data into classes based on objective parameters present in the data. Classification methods may be either supervised or unsupervised. Examples of supervised and unsupervised classification processes are described in Jain, "Statistical Pattern Recognition: A Review", IEEE Transactions on Pattern Analysis and Machine Intelligence, Vol. 22, No. 1, January 2000.

In supervised classification, training data containing examples of known categories are presented to a learning mechanism, which learns one or more sets of relationships that define each of the known classes. New data may then be applied to the learning mechanism, which then classifies the new data using the learned relationships. Examples of supervised classification processes include linear regression processes (*e.g*., multiple linear regression (MLR), partial least squares (PLS) regression and principal components regression (PCR)), binary decision trees (*e.g*., recursive partitioning processes such as CART - classification and regression trees), artificial neural networks such as back propagation networks, discriminant analyses (*e.g*., Bayesian classifier or Fischer analysis), logistic classifiers, and support vector classifiers (support vector machines).

A supervised classification method can be a recursive partitioning process. Recursive partitioning processes use recursive partitioning trees to classify spectra derived from unknown samples. Further details about recursive partitioning processes are provided in U.S. Patent Application No. 2002 0138208 A1 to Paulse et al.*,* "Method for analyzing mass spectra."

The classification models that are created can be formed using unsupervised learning methods. Unsupervised classification attempts to learn classifications based on similarities in the training data set, without pre-classifying the spectra from which the training data set was derived. Unsupervised learning methods include cluster analyses. A cluster analysis attempts to divide the data into "clusters" or groups that ideally should have members that are very similar to each other, and very dissimilar to members of other clusters. Similarity is then measured using some distance metric, which measures the distance between data items, and clusters together data items that are closer to each other. Clustering techniques include the MacQueen's K-means algorithm and the Kohonen's Self-Organizing Map algorithm.

Learning algorithms asserted for use in classifying biological information are described, for example, in PCT International Publication No. WO 01/31580 (Barnhill *et al.,* "Methods and devices for identifying patterns in biological systems and methods of use thereof'), U.S. Patent Application No. 2002 0193950 A1 (Gavin *et al.,* "Method or analyzing mass spectra"), U.S. Patent Application No. 2003 0004402 A1 (Hitt *et al.,* "Process for discriminating between biological states based on hidden patterns from biological data"), and U.S. Patent Application No. 2003 0055615 A1 (Zhang and Zhang, "Systems and methods for processing biological expression data").

The classification models can be formed on and used on any suitable digital computer. Suitable digital computers include micro, mini, or large computers using any standard or specialized operating system, such as a Unix, Windows^{™} or Linux^{™} based operating system. The digital computer that is used may be physically separate from the mass spectrometer that is used to create the spectra of interest, or it may be coupled to the mass spectrometer.

The training data set and the classification models can be embodied by computer code that is executed or used by a digital computer. The computer code can be stored on any suitable computer readable media including optical or magnetic disks, sticks, tapes, etc., and can be written in any suitable computer programming language including C, C++, visual basic, etc.

The learning algorithms described above are useful both for developing classification algorithms for the biomarkers already discovered, or for finding new biomarkers for ovarian cancer. The classification algorithms, in turn, form the base for diagnostic tests by providing diagnostic values (*e.g*., cut-off points) for biomarkers used singly or in combination.

### KITS FOR DETECTION OF BIOMARKERS FOR OVARIAN CANCER

Also disclosed (not part of the invention) are kits for aiding in the diagnosis of ovarian cancer (e.g., identifying ovarian cancer status, detecting ovarian cancer, identifying early stage ovarian cancer, selecting a treatment method for a subject at risk of having ovarian cancer, and the like), which kits are used to detect biomarkers disclosed herein. The kit may comprise agents that specifically recognize the biomarkers identified in Table 1. The agents can be antibodies. The kit may contain 1, 2, 3, 4, 5, or more different antibodies that each specifically recognize one of the biomarkers set forth in Table 1.

The kit may comprise a solid support, such as a chip, a microtiter plate or a bead or resin having capture reagents attached thereon, wherein the capture reagents bind the biomarkers disclosed herein. Thus, for example, the kits disclosed herein can comprise mass spectrometry probes for SELDI, such as ProteinChip^{®} arrays. In the case of biospecfic capture reagents, the kit can comprise a solid support with a reactive surface, and a container comprising the biospecific capture reagents.

The kit can also comprise a washing solution or instructions for making a washing solution, in which the combination of the capture reagent and the washing solution allows capture of the biomarker or biomarkers on the solid support for subsequent detection by, e.g., mass spectrometry. The kit may include more than type of adsorbent, each present on a different solid support.

Such a kit can comprise instructions for use in any of the methods described herein. For example, the instructions may provide suitable operational parameters in the form of a label or separate insert. For example, the instructions may inform a consumer about how to collect the sample, how to wash the probe or the particular biomarkers to be detected.

The kit may further comprise one or more containers with controls (e.g., biomarker samples) to be used as standard(s) for calibration.

### EXAMPLES

(Examples relating to the use of marker panels other than the one consisting of markers CA125, Prealbumin, Transferrin and HE4 are for comparative reasons only.)

### Example 1: Panels of biomarkers that included IGFBP2, Interleukin 6 (IL-6), and FSH and/or HE4 improved the preoperative assessment of ovarian tumors compared to a diagnostic panel comprising Transthyretin (TT or prealbumin), Apolipoprotein A-1 (Apo A-1), beta 2-Microglobulin (beta 2M), Transferrin (Tfr) and Cancer Antigen 125 (CA 125 II)

OVA1 provides an important method for characterizing ovarian cancer. OVA1 involves measuring biomarkers Apolipoprotein A1 (ApoA1), Beta2 microglobulin (B2MG), CA-125, Transthyretin/prealbumin, and Transferrin for pre-surgical assessment of adnexal masses for risk of ovarian malignancy. To further improve the specificity of these markers, potential biomarkers and panels of biomarkers were evaluated using a set of clinical samples enriched for OVA1 false positive benign patients to identify panels that would improve the specificity of the test and properly exclude these false positive subjects. This testing identified a panel comprising or consisting of CAl25-II, transthyretin/prealbumin, IGFBP2, IL6, and FSH. At a fixed sensitivity of 90%, the mean and median specificity of models using the new panel in testing were 78.2% (95% Cl: 76.7 - 79.8%), and 80.6%, respectively. The mean and median absolute improvements over that of Transthyretin (TT or prealbumin), Apolipoprotein A-1 (Apo A-1), beta 2-Microglobulin (beta 2M), Transferrin (Tfr) and Cancer Antigen 125 (CA 125 II) were 18.6% (95% Cl: 16.4% - 20.9%) and 20.3%, respectively.

A list of 81 potential biomarkers was initially looked at for value in increasing specificity. From this list, 30 potential biomarkers were selected. A screening panel of serum samples from 19 patients with ovarian cancer and 22 patients with benign pelvic masses was designed and used to perform initial assessment of candidate biomarkers. The benign patients were specifically selected so that the majority of them would have false positive scores when evaluated with biomarkers: Transthyretin (TT or prealbumin), Apolipoprotein A-1 (Apo A-1), beta 2-Microglobulin (beta 2M), Transferrin (Tfr) and Cancer Antigen 125 (CA 125 II) markers.

Based on availability and quality of ELISA assays, a total of 13 candidate biomarkers were evaluated using the designed screening sample panel. When possible, ELISA kits from different vendors were evaluated to select those with better analytical performance. From results of this screening sample set, insulin-like growth factor-binding protein 2 (IGFBP2) and interleukin 6 (IL6) were selected to be evaluated on the study sample set, denoted as "OVA500." In addition, Human Epididymis Protein 4 (HE4) and Follicle-stimulating hormone (FSH) were also evaluated on the OVA500 study sample set. The inclusion of FSH potentially eliminates the need to have separate menopausal status-dependent cutoffs (In Vitro Diagnostic Multivariate Index Assay (IVDMIA) values can be adjusted for menopausal status internally at the algorithm level based on serum FSH level). In all, a set of 9 biomarkers have been evaluated on the OVA500 study samples, including those for OVA1 calculation: Transthyretin (TT or prealbumin), Apolipoprotein A-1 (Apo A-1), β2-Microglobulin (beta 2M), Transferrin (Tfr) and Cancer Antigen 125 (CA 125 II), IGFBP2, IL6, HE4, and FSH.

Insulin-like growth factor binding protein 2 (IGFBP2), interleukin 6 (IL6), and follicle stimulating hormone (FSH) were among the biomarkers selected to be further evaluated on a prospectively collected clinical sample set, along with the original five biomarkers of OVA1. Consecutive patients with a documented pelvic mass planned for surgical intervention were prospectively enrolled at 27 sites. Exclusion criteria included a diagnosis of malignancy in the previous 5 years or initial enrollment by a gynecologic oncologist. At the time of analysis, 384 subjects had all biomarker values. Among them 69 were ovarian cancer cases (13 low malignancy potential (LMP), 27 stages 1/2, 19 serous, 11 endometrioid, 5 mucinous, and 4 clear cell). Biomarkers were tested by ELISA and reported as continuous values. Using a subset of the samples, the biomarkers were first selected for inclusion in a final panel based on contributions in multivariate models estimated by bootstrap. The selected biomarkers were further assessed for ability to improve specificity of risk stratification at a fixed sensitivity over that of biomarkers Transthyretin (TT or prealbumin), Apolipoprotein A-1 (Apo A-1), beta 2-Microglobulin (beta 2M), Transferrin (Tfr) and Cancer Antigen 125 (CA 125 II) using the full data set. This was done by cross-validation of multivariate models with 50/50 split between training and testing.

In models using all 9 biomarkers (i.e., Transthyretin (TT or prealbumin), Apolipoprotein A-1 (Apo A-1), β2-Microglobulin (beta 2M), Transferrin (Tfr) and Cancer Antigen 125 (CA 125 II), IGFBP2, IL6, HE4, and FSH), distribution of absolute improvement by in specificity over that of biomarkers Transthyretin (TT or prealbumin), Apolipoprotein A-1 (Apo A-1), beta 2-Microglobulin (beta 2M), Transferrin (Tfr) and Cancer Antigen 125 (CA 125 II) at fixed 95% sensitivity in testing over 100 rounds of cross-validation of multivariate models was observed. Mean and median absolute improvement were 24.1% (95% Confidence Interval (CI): 21.0-27.2%) and 24.6%, respectively (Figure 1).

In models using 8 of the 9 biomarkers (Transthyretin (TT or prealbumin), Apolipoprotein A-1 (Apo A-1), β2-Microglobulin (beta 2M), Transferrin (Tfr) and Cancer Antigen 125 (CA 125 II), IGFBP2, IL6, and FSH, without HE4), distribution of absolute improvement in specificity over that of Transthyretin (TT or prealbumin), Apolipoprotein A-1 (Apo A-1), beta 2-Microglobulin (beta 2M), Transferrin (Tfr) and Cancer Antigen 125 (CA 125 II) at fixed 95% sensitivity in testing over 100 rounds of cross-validation of multivariate models was also observed. Mean and median absolute improvement were 22.0% (95% Confidence Interval (CI): 18.8-25.3%) and 23.3%, respectively (Figure 2).

Bootstrap analysis of linear models of each of the markers was performed, and ranked accordingly (Table 2).

**Table 2. Rankings of Ovarian Cancer Biomarkers**

| Biomarker | Mean Rank | STD of Rank | Median Rank |
|---|---|---|---|
| CA125 | 1.0 | 1.1 | 1 |
| ApoA1 | 5.6 | 1.1 | 6 |
| PreAlb | 4.6 | 0.9 | 4 |
| B2M | 5.7 | 1.1 | 6 |
| TRF | 6.0 | 0.5 | 3 |
| IGFBP2 | 2.8 | 0.5 | 3 |
| IL6 | 2.3 | 0.2 | 2 |

The top ranked (mean rank and median rank) four biomarkers from bootstrap analysis of linear models were selected for a panel of biomarkers for further study. Additionally, FSH was "artificially" added to the panel mainly for its high correlation with menopausal status which can be used in place of menopausal status-dependent cutoffs (as in other ovarian cancer diagnostics). Thus, the panel of biomarkers selected for further study included: CA125, PREALB, IGFBP2, IL6, and FSH.

The performance of multivariate index assays was assessed. Distribution of Specificity at 90% Sensitivity in 30 cross-validation runs (50/50 split between training and testing) showed that for both linear and nonlinear models, the majority of the models had specificity > 70% at the fixed sensitivity of 90% (Figures 3 and 4A).

Distribution of Specificity at 95% Sensitivity in 100 cross-validation runs (50/50 split between training and testing) for nonlinear models only, indicated that the majority of the models have specificity > 60% (Figure 4B).

At cutoffs corresponding to overall sensitivity of 95%, the majority of models have sensitivities ranging from ~85-95% for stage 1 cases (Figure 5). The distribution of specificity was the same as in Figure 4.

Distribution of sensitivity was also evaluated between invasive and low malignancy potential (LMP) ovarian tumor, and among the four major histologic subtypes. At a cutoff corresponding to 95% overall sensitivity (on training data), as expected, the models offered higher sensitivity for invasive cases than those with LMP ovarian tumors (Figure 6).

The breakdown comparison of sensitivities (training: red, testing blue) among the four major histologic subtypes of ovarian cancer was statistically not very meaningful due to the small number of cases in each of the subtypes (Figures 7A-7D).

Distributions of sensitivity and specificity were evaluated for pre-/post-menopausal groups and training/test using cutoffs for 95% overall Sensitivity in 100 cross-validation runs. From the distributions, specificities were somewhat higher among the pre-menopausal than that among the post-menopausal benign patients (Figures 8A-8D).

The distribution of improvement of models with the new panel comprising or consisting of CA125, prealbumin, IGFBP2, IL6, and FSH (Figure 9) over a panel comprising Transthyretin (TT or prealbumin), Apolipoprotein A-1 (Apo A-1), beta 2-Microglobulin (beta 2M), Transferrin (Tfr) and Cancer Antigen 125 (CA 125 II) showed that the selected panels of biomarkers had absolute improvement > 15% when evaluated on combined training and test sets (Figure 9). Moreover, the null hypothesis that the absolute improvement in specificity is ≤ 15% was rejected with statistical significance (p-value < 2.5e-017). When evaluated on the test sets only, the distribution of the absolute improvement had a wider spread, partially due to smaller sample sizes of the test sets and possible "shrinkage in performance" when models are generalized on independent sets. However, the majority still had an absolute improvement > 12%. Again, the null hypothesis that the absolute improvement in specificity is ≤ 12% is rejected with statistical significance (p-value = 0.0010).

Distribution of area-under-curve (AUC) from receiver-operating-characteristic (ROC) analysis from 100 cross-validation runs - nonlinear models only, confirmed that the majority of models have very high AUC values (Figure 10). However, for study purposes, the improvement in specificity at extremely high sensitivity may not be reflected in the overall AUCs. In actual model development, "partial AUC" may be chosen to access model performance and drive model training.

An improvement in specificity of the 5-biomarker panel: CA125, prealbumin, IGFBP2, IL6, and FSH was observed after replacing FSH with HE4. Thus HE4, with its relatively high specificity among benign ovarian tumor patients, further improved the performance of multivariate models (Figures 11 and 12). The performance of the 5-biomarker panels comprising CA125, prealbumin, IGFBP2, IL6, and FSH are summarized at Table 3.

**Table 3**

| | New Panel | | New Panel, FSH replaced by HE4 | |
|---|---|---|---|---|
| | Training | Testing | Training | Testing |
| Mean | 78.3% | 78.2% | 84.1% | 83.8% |
| 99%C1 | (76.2, 79.8) | (76.5, 80.8) | (83.3, 84.9) | (82.8, 84.8) |
| Median | 80.3% | 80.6% | 84.6% | 84.9% |
| 95%C1 | (78.5, 81.1) | (77.4, 81.4) | (83.4, 85.3) | (83.7, 85.6) |

The performance estimates are based on 50/50 cross-validation, and actual performance on a totally independent test set may have a certain degree of "shrinkage." Additionally, the mean is not as informative as the median, as the distributions are not very symmetric.

### Example 2: 3- and 4-marker panels dramatically improve the specificity of ovarian cancer assessment

Comparison of the following panel: Transthyretin (TT or prealbumin), Apolipoprotein A-1 (Apo A-1), beta 2-Microglobulin (beta 2M), Transferrin (Tfr) and Cancer Antigen 125 (CA 125 II) +HE4 (combination) using a linear model against HE4 alone and Transthyretin (TT or prealbumin), Apolipoprotein A-1 (Apo A-1), beta 2-Microglobulin (beta 2M), Transferrin (Tfr) and Cancer Antigen 125 (CA 125 II) also showed improved specificity using the combination model (at 90% Sensitivity in 100 cross-validation runs; 50/50 split between training and testing) (Figure 13). In particular, the combination model performance was not calculated using menopausal status dependent cutoffs.

As shown in Figure 13, right hand panel, the median for specificity in the set of markers including HE4 is between 75-80% at 90% sensitivity. This represents a dramatic improvement over all panels currently in clinical use to characterize ovarian cancer. An exemplary panel is shown in solid bars in the right hand panel, where specificity is between 55 and 60%.

Bootstrap estimated rank and standard rank of OVA1 component markers and HE4 using linear models showed that the 4 highest ranked biomarkers were CA125, Transthyretin/ Prealbumin, Transferrin, and HE4; and the 3 highest ranked biomarkers were CA125, Transthyretin/Prealbumin, and HE4 (Table 4).

**Table 4**

| Biomarker | Mean Rank | STD of Rank | Median Rank |
|---|---|---|---|
| CA125 | 1.8 | 0.43 | 2 |
| ApoA1 | 5.8 | 0.46 | 6 |
| PreAlb | 3.4 | 0.63 | 3 |
| B2M | 5.0 | 0.53 | 5 |
| TRF | 3.7 | 0.64 | 4 |
| HE4 | 1.2 | 0.43 | 1 |

Panels having the 4 and 3 highest ranked biomarkers were evaluated for distributions of specificity at 90% sensitivity (100 cross-validation runs, 50/50 split between training and testing), and compared to OVA1 (OVA1 performances were not calculated using product cutoffs).

Comparison of CA125+PreAlb+TRF+HE4 and CA125+PreAlb+HE4 non-linear models against Transthyretin (TT or prealbumin), Apolipoprotein A-1 (Apo A-1), beta 2-Microglobulin (beta 2M), Transferrin (Tfr) and Cancer Antigen 125 (CA 125 II) also showed improved specificity over Transthyretin (TT or prealbumin), Apolipoprotein A-1 (Apo A-1), beta 2-Microglobulin (beta 2M), Transferrin (Tfr) and Cancer Antigen 125 (CA 125 II) (at 90% Sensitivity in 100 cross-validation runs; 50/50 split between training and testing) (Figure 14). The performance of a panel comprising Transthyretin (TT or prealbumin), Apolipoprotein A-1 (Apo A-1), beta 2-Microglobulin (beta 2M), Transferrin (Tfr) and Cancer Antigen 125 (CA 125 II) was computed on the same training/test sets used for cross-validation of other models, and product cut-offs were not used.

As shown in Figure 14, right hand panel, the specificity of the four marker panel including HE4 showed more than 90% specificity. This represents a dramatic improvement over all panels currently in clinical use to characterize ovarian cancer. An exemplary panel is shown in solid bars in the right hand panel, where specificity is between 55 and 60%.

Both the 3-marker (CA125, prealbumin, HE4) panel and the 4-marker (CA125, prealbumin, transferring, HE4) panel show unexpectedly superior specificity relative to the prior art without sacrificing sensitivity. Currently, 1 out of every 2 women evaluated with state of the art diagnostic tests are needlessly referred to a gynecological oncologist. When adnexal masses are assessed using the 3 or 4 marker panels disclosed herein, the number of false positives is greatly reduced. The 3 marker panel provides a 19% false positive rate, which is less than half the false positive rate provided by ovarian cancer panels present in the prior art. The 4 marker panel provides a 13-14% false positive rate-cutting the false positive rate provided by prior art diagnostics by two thirds. The 3- and 4-marker panels disclosed herein ensure that all women receive the correct diagnosis and a referral to the appropriate physician.

The performance of CA125+PreAlb+TRF+HE4, CA125+PreAlb+HE4, and Transthyretin (TT or prealbumin), Apolipoprotein A-1 (Apo A-1), beta 2-Microglobulin (beta 2M), Transferrin (Tfr) and Cancer Antigen 125 (CA 125 II) (bootstrap estimated Means, Medians and 95%CIs of specificity at 90% sensitivity) are summarized at Table 6.

**Table 5**

| | OVA1* (did not use product cutoffs) | | CA 125+Prealb+TRF+HE4 | | CA125+Prealb+HE4 | |
|---|---|---|---|---|---|---|
| | Training | Testing | Training | Testing | Training | Testing |
| Mean | 58.8% | 59.6% | 85.3% | 85.4% | 80.6% | 80.9% |
| 95%C1 | (57.3, 61.3) | (57.7, 61.7) | (84.0, 86.6) | (83.9, 86.3) | (78.7, 81.9) | (79.0, 81.8) |
| Median | 56.5% | 58.0% | 86.3% | 86.3% | 80.8% | 81.3% |
| 95%C1 | (55.1, 60.7) | (55.2, 61.3) | (85.3, 87.3) | (85.9, 87.2) | (80.4, 82.5) | (80.3, 81.8) |

Comparison of CA125+PreAlb+TRF+HE4 and CA125+PreAlb+HE4 non-linear models against Transthyretin (TT or prealbumin), Apolipoprotein A-1 (Apo A-1), beta 2-Microglobulin (beta 2M), Transferrin (Tfr) and Cancer Antigen 125 (CA 125 II) also showed an improvement in ROC/AUCs over Transthyretin (TT or prealbumin), Apolipoprotein A-1 (Apo A-1), beta 2-Microglobulin (beta 2M), Transferrin (Tfr) and Cancer Antigen 125 (CA 125 II) (100 cross-validation runs; 50/50 split between training and testing) (Figure 15). Transthyretin (TT or prealbumin), Apolipoprotein A-1 (Apo A-1), beta 2-Microglobulin (beta 2M), Transferrin (Tfr) and Cancer Antigen 125 (CA 125 II) performance was computed on the same training/test sets used for cross-validation of other models, and product cut-offs were not used. The performance of CA125+PreAlb+TRF+HE4, CA125+PreAlb+HE4, and Transthyretin (TT or prealbumin), Apolipoprotein A-1 (Apo A-1), beta 2-Microglobulin (beta 2M), Transferrin (Tfr) and Cancer Antigen 125 (CA 125 II) (bootstrap estimated Means, Medians and 95%CIs of ROC/AUCs) are summarized at Table 7.

**Table 6**

| | OVA1* (did not use product cutoffs) | | CA125+Prealb+TRF+HE4 | | CA125+Prealb+HE4 | |
|---|---|---|---|---|---|---|
| | Training | Testing | Training | Testing | Training | Testing |
| Mean | 88.4% | 89.0% | 94.1% | 94.1% | 93.5% | 93.4% |
| 95%C1 | (88.0, 88.9) | (88.5, 89.5) | (93.4, 94.5) | (93.6, 94.4) | (92.8, 93.9) | (93.0, 93.8) |
| Median | 88.5% | 88.9% | 94.3% | 94.1% | 93.9% | 93.5% |
| 95%C1 | (88.0, 88.8) | (88.7, 89.7) | (93.6, 94.8) | (93.6, 94.4) | (93.3, 94.1) | (93.1, 93.9) |

There was no significant difference in distributions of AUCs between the 3-biomarker model and 4-biomarker model, as both had reached fairly high values. However, the difference between the two models comparing specificity at 90% sensitivity (Figure 14) indicated that the addition of the 4^{th} marker mainly contributed to the shape of the ROC rather than its AUC.

In summary, the identified panels demonstrated the potential to significantly improve specificity over that of the first-generation of ovarian cancer diagnostics that rely on a panel that includes Transthyretin (TT or prealbumin), Apolipoprotein A-1 (Apo A-1), beta 2-Microglobulin (beta 2M), Transferrin (Tfr) and Cancer Antigen 125 (CA 125 II). The performance estimation by leave-k-out cross-validation indicated that at cutoffs corresponding to a sensitivity of 95% for all malignant cases (~90% for stage I cases), the panel of biomarkers with nonlinear classification models demonstrated a significantly improved specificity over a panel comprising Transthyretin (TT or prealbumin), Apolipoprotein A-1 (Apo A-1), beta 2-Microglobulin (beta 2M), Transferrin (Tfr) and Cancer Antigen 125 (CA 125 II). The overall improvement in ROC/AUC was mostly from better specificity at ROC curve range >90% sensitivity. The observed distribution and variation of cross-validation performance indicated that a IVDMIA model will have clinically meaningful improvement (~15% or more) in specificity over OVA1. In particular, the null hypothesis that "the absolute improvement in specificity is ≤ 15%" was rejected with statistical significance (p-value < 2.5e-017). Thus, the identified panels of biomarkers have improved specificity over the first-generation ovarian cancer diagnostics, while maintaining a high sensitivity in pre-surgical assessment of adnexal masses for risk of malignancy.

### Example 3: Panels comprising CA125, APOA1, PREALB, B2M, TRF, HE4, IGFBP2, IL6, FSH, TAG-72/CA724 are useful in characterizing ovarian cancer.

Serum samples from patients (n=381) were obtained. These patients included 69 ovarian cancer cases and 312 patients with benign ovarian tumor as controls. The patient diagnoses were confirmed by surgery. Serum samples from these patients were divided into roughly equal proportions and used as a training set and a test set for plotting purposes. Figure 20A shows a biplot of supervised component analysis results using all ten (10) biomarkers: CA125, APOA1, PREALB, B2M, TRF, HE4, IGFBP2, IL6, FSH, TAG-72/CA724. A biplot is a generalization of the simple two-variable scatterplot. A biplot allows information on both samples and variables of a data matrix to be displayed graphically. Samples are displayed as points while variables are displayed either as vectors, linear axes or nonlinear trajectories.

The plot indicates that the ovarian cancer cases and benign controls were fairly well separated along the x-axis which is a linear combination of the 10 biomarkers derived using a supervised learning algorithm. The contributions of the 10 biomarkers are indicated as vectors from the origin of the plot. In this plot, the length of the projection of a vector to the x-axis indicates its contribution to the two group separation. The relative angular position provides information on whether the biomarkers are similar or complementary for the group separation.

Figure 21 provides an ROC analysis of the results using biomarkers CA125, APOA1, PREALB, B2M, TRF, HE4, IGFBP2, IL6, FSH, TAG 72/CA72-4.

A bootstrap analysis featuring ranking and selection was performed next. A total of 30 bootstrap runs were performed. In each run, a bootstrap sample which has the same sample size as the original sample were randomly selected with replacement from the original sample set. Using the same training parameters, a linearly classifier was derived, the individual features (biomarkers) were ranked according to their contribution to the linear classifier as indicated by their respective weights in the linear combination formula. In Table 8, the estimated mean and median rank of the features along with the standard deviations were listed in sorted order. A smaller rank indicates a more informative biomarker.

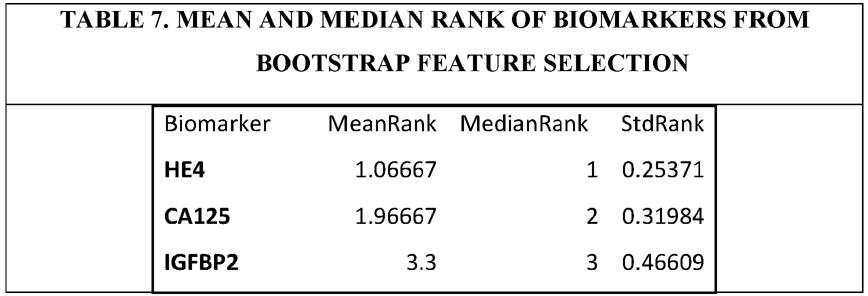

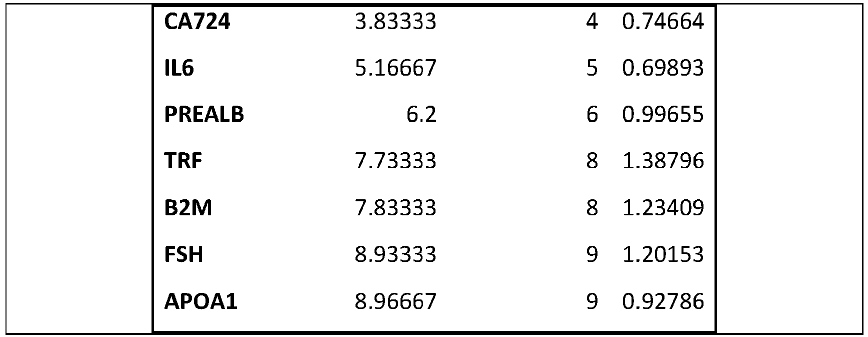

Cross-validation assessment of specificity at fixed 90% sensitivity was carried out. The other basic settings remained the same. The total available sample set (n=381) included 69 ovarian cancer cases and 312 patients with benign ovarian tumor as controls, all conformed by surgery. With these samples, cross-validation was used to assess the various models' potential performance in future samples. In each training session, the samples were randomly divided into a training set and a set-aside testing set at roughly 50/50 division. A model was derived on the training set and then tested (cross-validated) on the set-aside set. For both training and test sets, specificities were set at a fixed sensitivity of 90% were estimated and recorded. The same training sessions were repeated 100 times, each time with a new random division of the sample set. At the end of these analyses, the histograms (distributions) of the 100 estimated specificities (at fixed 90% sensitivity) from training and from cross-validation, respectively. In general, the histograms from cross-validation would have a lightly wide spread, due to the relatively small sample size and possible "shrinkage" in generalization. However, once the training parameters were optimized, the differences in results from training and cross-validation were typically not significant.

Table 8 shows feature ranks from Bootstrap Selection.

**Table 8: Feature Ranks from Bootstrap Selection**

| meanRank, seRank, medianRank | | | | | | | |
|---|---|---|---|---|---|---|---|
| 'CA125' | 2.2333 | 1.6955 | 2,000 | | | | |
| 'APO' | 9.2333 | 1.8134 | 10.0000 | Biomarker | Mean Rank | Median Rank | Std Rank |
| 'PREALB' | 6.2333 | 1.6333 | 7.0000 | CA125 | 1.96667 | 2 | 0.31984 |
| 'B2M' | 7.4333 | 1.2507 | 8.0000 | APOA1 | 8.96667 | 9 | 0.92786 |
| 'TRF' | 6.2000 | 1.7301 | 6.5000 | PREALB | 6.2 | 6 | 0.99655 |
| 'HE4' | 1.4667 | 1.1666 | 1.0000 | B2M | 7.83333 | 8 | 1.23409 |
| 'IGFBP2' | 4.5000 | 1.7171 | 4.0000 | TRF | 7.73333 | 8 | 1.38796 |
| 'IL6' | 4.8667 | 1.2521 | 5.0000 | HE4 | 1.06667 | 1 | 0.25371 |
| 'FSH' | 8.9333 | 0.8277 | 9.0000 | IGFBP2 | 3.3 | 3 | 0.46609 |
| 'CA724' | 3.9000 | 1.3734 | 3.5000 | IL6 | 5.16667 | 5 | 0.69893 |
| | | | | FSH | 8.93333 | 9 | 1.20153 |
| | | | | CA724 | 3.83333 | 4 | 0.74664 |

Biomarkers with the best ranking in bootstrap feature selection included CA125, HE4, IGFBP2, IL6, CA724 (Figure 22). Results using a panel comprising CA125, Prealb, TRF, HE4, and TAG-72/CA 72-4 are shown in Figure 23. Results obtained using a linear model with a panel comprising CA125, HE4, CA724 are shown in Figure 24. A linear model was also used to obtain the results shown in Figure 25, which analyzes markers CA125, Prealb, TRF, and CA724. Figure 26 shows results obtained using markers CA125, Prealb, IGFBP2, IL6, and CA724.

The results described herein were obtained using the following materials and methods.

### Study Population for Example 1 and Example 2

A multicenter prospective collection from non-gynecologic oncologist practices was obtained. Consecutive patients who met inclusion criteria were prospectively enrolled at 27 sites throughout the United States, with Institutional Review Board approval from each site. All clinicians initially enrolling patients were from non-gynecologic oncology specialty practices, although patients may ultimately have had consultation with or undergone surgery by a gynecologic oncologist. Inclusion criteria were: females age ≥18 years, signed informed consent and agreeable to phlebotomy, documented pelvic mass planned for surgical intervention within 3 months of imaging. A pelvic mass was confirmed by imaging (computed tomography, ultrasonography, or magnetic resonance imaging) prior to enrollment. Exclusion criteria included a diagnosis of malignancy in the previous 5 years (except of non-melanoma skin cancers) or enrollment by a gynecologic oncologist. Menopause was defined as the absence of menses for ≥12 months, or age ≥50. Demographic and clinic-pathologic information were collected on case report forms. A total of 494 subjects were evaluable (Table 9). Currently, 491 samples have values for all 9 biomarkers. Among them, 107 had IGFBP2 and IL6 analyzed using different batches of kits. With observed batch variations, data for these samples were not used in the current analysis.

**Table 10. Demographics of evaluable subjects of OVA500 study**

| | | | **All Evaluable Subjects (N=494)** | **Premenopausal Women (N=277)** | **Postmenopausal Women (N=217)** |
|---|---|---|---|---|---|
| **Age, years** | | | | | |
| | N | | 494 | 277 | 217 |
| | Mean (SD) | | 48.6 (14.15) | 39.6 (8.95) | 60.2(10.74) |
| | Median | | 48 | 41 | 60 |
| | Range(min to max) | | 18 to 87 | 18 to 60 | 33 to 87 |

| **Ethnicity/race, n (%)** | | | | | |
|---|---|---|---|---|---|
| | White | | 348 (70.4) | 174 (62.8) | 174 (80.2) |
| | African-American | | 81 (16.4) | 54 (19.5) | 27 (12.4) |
| | Hispanic or Latino | | 46 (9.3) | 36 (13.0) | 10 (4.6) |
| | Asian | | 13 (2.6) | 8 (2.9) | 5 (2.3) |
| | Native Hawaiian/Pacific Islander | | 1 (0.2) | 1 (0.4) | 0 (0.0) |
| | Other | | 5 (1.0) | 4 (1.4) | 1 (0.5) |

| **No. of pregnancies, n (%)** | | | | | |
|---|---|---|---|---|---|
| | None | | 80 (16.2) | 56 (20.2) | 24 (11.1) |
| | 1 | | 87 (17.6) | 53 (19.1) | 34 (15.7) |
| | 2 | | 131 (26.5) | 70 (25.3) | 61 (28.1) |
| | 3 | | 94 (19.0) | 50 (18.1) | 44 (20.3) |
| | 4 or more | | 102 (20.6) | 48 (17.3) | 54 (24.9) |

| **Physician's Assessment, n (%)** | | | | | |
|---|---|---|---|---|---|
| | Malignant | | 98 (19.8) | 39 (14.1) | 59 (27.2) |
| | Benign | | 396 (80.2) | 238 (85.9) | 158 (72.8) |

| **Pathology diagnosis, n (%)** | | | | | |
|---|---|---|---|---|---|
| | Benign | | 402 (81.4) | 246 (88.8) | 156 (71.9) |
| | Non-ovarian primary malignancy with no involvement of the ovaries | | 4 (0.8) | 1 (0.4) | 3 (1.4) |
| | | | | | |
| | Non-ovarian primary malignancy with involvement of the ovaries | | 6 (1.2) | 2 (0.7) | 4 (1.8) |
| | | | | | |
| | Ovarian low malignant potential (Borderline) | | 17 (3.4) | 5 (1.8) | 12 (5.5) |
| | | | | | |
| | Primary malignant ovarian tumor | | 65 (13.2) | 23 (8.3) | 42 (19.4) |
| | Epithelial ovarian cancer | | 60 (12.1) | 18 (6.5) | 42 (19.4) |
| | | Serous | 24 (4.9) | 8 (2.9) | 16 (7.4) |
| | | Mucinous | 9 (1.8) | 1 (0.4 | 8 (3.7) |
| | | Endometroid | 13 (2.6) | 5 (1.8) | 8 (3.7) |
| | | Clear cell | 5 (1.0) | 1 (0.4) | 4 (1.8) |
| | | Carcinosarcoma | 1 (0.2) | 1 (0.4) | 0 (0.0) |
| | | Mixed | 1 (0.2) | 0 (0.0) | 1 (0.5) |
| | | Other | 7 (1.4) | 2 (0.7) | 5 (2.3) |
| | Non-Epithelial; Other | | 5 (1.0) | 5 (1.8) | 0 (0.0) |

| | | **Tumor Stage, n (% of all primary malign ovarian tumor)** | | | |
|---|---|---|---|---|---|
| | | Stage 1 | 28 (43.1) | 9 (39.1) | 19 (45.2) |
| | | Stage 2 | 7(10.8) | 2(8.7) | 5(11.9) |
| | | Stage 3 | 25 (38.5) | 10 (43.5) | 15 (35.7) |
| | | Stage 4 | 5(7.7) | 2(8.7) | 3 (7.1) |

### Screening for candidate biomarkers

More than 30 potential biomarkers from an initial list of 81 were considered based on their reported performance on clinical samples and biological relevance to ovarian cancer. A "designed" screening panel of serum samples from 19 patients with ovarian cancer and 22 patients with benign pelvic masses were used to perform initial assessment of candidate biomarkers. The benign patients were specifically selected such that the majority of them would have false positive scores using an ovarian cancer panel comprising Transthyretin (TT or prealbumin), Apolipoprotein A-1 (Apo A-1), beta 2-Microglobulin (beta 2M), Transferrin (Tfr) and Cancer Antigen 125 (CA 125 II). The primary objective of the study was to assess additional biomarkers' ability to further improve specificity over that of Transthyretin (TT or prealbumin), Apolipoprotein A-1 (Apo A-1), beta 2-Microglobulin (beta 2M), Transferrin (Tfr) and Cancer Antigen 125 (CA 125 II).

Based on the availability and quality of ELISA assays, a total of 13 candidate biomarkers were evaluated using the designed screening sample panel. When possible, ELISA kits from different vendors were evaluated to select those with better analytical performance.
From results of this screening sample set, insulin-like growth factor-binding protein 2 (IGFBP2) and interleukin 6 (IL6) were selected to be evaluated on the OVA500 study sample set.

In addition, Human Epididymis Protein 4 (HE4) and Follicle-stimulating hormone (FSH) were also evaluated on the OVA500 study sample set. The inclusion of FSH potentially eliminates the need to have separate menopausal status-dependent cutoffs (IVDMIA values can be adjusted for menopausal status internally at the algorithm level based on serum FSH level).
Currently, a set of 9 biomarkers (which includes those for OVA1 calculation) have been evaluated on the OVA500 study samples: Transthyretin (TT or prealbumin), Apolipoprotein A-1 (Apo A-1), β2-Microglobulin (beta 2M), Transferrin (Tfr) and Cancer Antigen 125 (CA 125 II), IGFBP2, IL6, HE4, and FSH. The kit/system information of these biomarkers is provided at Table 9.

**Table 9**

| **Biomarker** | **Manufacturer/Kit** | **System** |
|---|---|---|
| **PreAlbumin** | Siemens Healthcare Diagnostics, #OUIF09 | Siemens BNII |
| **CA125 II** | Roche Diagnostics, #11776223 | Elecsys 2010 |
| **B2M** | The Binding Site, #LK043,T | Siemens BNII |
| **ApoA1** | Siemens Healthcare Diagnostics, #OUED15 | Siemens BNII |
| **TRF** | Siemens Healthcare Diagnostics, #OSAX15 | Siemens BNII |
| **IGF8P2*** | Ray Biotech, IGFBP-2 ELISA Kit #ELH-IGFBP2-001 | |
| **IL6*** | R&D Systems, Human IL-6 Quantikine HS SixPak #SS600B | |
| **FSH** | Roche Diagnostics, #11775863 | Elecsys 2010 |
| **HE4** | Abbott Diagnostics, #2P54-27 | Architect 12000 |

### Data preprocessing and normalization

In addition to the 9 biomarkers, OVA1 scores for the 491 samples were computed using the raw test values of the OVA1 five-biomarker panel. Individual biomarkers were first evaluated for their distribution patterns to decide whether numerical transformations were required to achieve reasonable symmetric distribution patterns. (Figure 16A). For further analysis, the individual biomarkers were transformed as provided in Table 10.

**Table 10. Transformation of biomarker to correct for skewness.**

| Biomarker | Transformation |
|---|---|
| CA125 | log10(CA125 + 90.001); |
| B2M | log10(B2M + 0.001); |
| IGFBP2 | log10(IGFBP2 + 1); |
| IL6 | log10(IL6 + 1); |
| HE4 | log10(log10(HE4) + 0.001)); |
| FSH | log10(FSH + 1); |

As shown in Figure 16B, the transformations provided reasonable symmetric distribution patterns for the selected biomarkers. After transformation, the results were further converted to z-scores using each biomarker's population mean and standard deviation (Figure 16C). X-score conversion was performed to ensure that the biomarkers have comparable ranges in algorithm/model development.

### Selection and construction of biomarker panel

Selection and construction of the panel of biomarkers were done using a randomly selected training subset of the samples, involving extensive use of statistical resampling - bootstrap and leave-k-out cross-validation. Univariate assessment was performed, including ROC curve analysis, AUCs, and standard deviation of AUCs, estimated by bootstrap analysis. Examples of bootstrap estimated ROC/AUC of IGFBP2, IL6, FSH, and HE4 are provided at Figures 17A-17D.

Multivariate assessment was performed using linear and nonlinear multivariate models, including comparison of difference in AUC, and sensitivity at fixed specificity of 90% and 95%. The total number of biomarkers evaluated in the panels were ≤ 5. FSH was added to panels as a biomarker to eliminate the need for menopausal status-dependent cutoffs. An example of a single training/testing run is shown at (Figures 18A and 18B).

### Derivation of multivariate index assay models

Linear and non-linear multivariate index assay models were derived, using extensive statistical resampling. Performance of the models were compared to Transthyretin (TT or prealbumin), Apolipoprotein A-1 (Apo A-1), beta 2-Microglobulin (beta 2M), Transferrin (Tfr) and Cancer Antigen 125 (CA 125 II), CA125 alone, CA125+HE4, using criteria including AUC and sensitivity at fixed specificity of 90% and 95%.

## Claims

1. In vitro use of a panel for pre-operatively assessing the risk of a subject having an adnexal mass as having ovarian cancer, the panel consisting of markers:
Cancer Antigen 125 (CA125), Prealbumin, Transferrin, and Human Epididymis Protein 4 (HE4).

2. An in vitro method for reducing the rate of false positive pre-operative ovarian cancer assessment in a subject having an adnexal mass, the method comprising,
(a) measuring the level of a panel of markers consisting of CA125, Prealbumin, Transferrin, and HE4 in a biological sample derived from the subject; and
(b) comparing the level of said markers to a reference level.

3. An in vitro method for pre-operatively assessing the risk of a subject having an adnexal mass as having ovarian cancer, the method comprising
(a) measuring the level of a panel of markers consisting of CA125, Prealbumin, Transferrin, and HE4 in a biological sample derived from the subject; and
(b) comparing the level of said markers to a reference level.

4. The method of claim 2 or 3, wherein:
(i) the method further involves assessing clinical markers of ovarian cancer risk comprising age, pre-menopausal status, post-menopausal status, family history, physical examination, imaging results, and history of smoking;
(ii) the comparing comprises using a linear model or a non- linear model;
(iii) the method further involves detecting follicle-stimulating hormone (FSH); and/or
(iv) the detecting step is by immunoassay or affinity capture.

5. The method of claim 2 or 3, wherein the method is carried out in a plate, chip, beads, microfluidic platform, membrane, planar microarray, or suspension array.

## Patentansprüche

1. In vitro Verwendung eines Panels zum prä-operativen Bestimmen des Risikos eines Subjekts mit einer adnexalen Masse, an Eierstockkrebs erkrankt zu sein, wobei das Panel aus den Markern besteht:
Cancer Antigen 125 (CA125), Prealbumin, Transferrin und Human Epididymis Protein 4 (HE4).

2. In vitro Verfahren zum Verringern der Rate an falsch positiven prä-operativen Einschätzungen eines Subjekts mit einer adnexalen Masse, an Eierstockkrebs erkrankt zu sein, wobei das Verfahren umfasst:
(a) Erfassen des Niveaus eines Panels von Markern bestehend aus CA125, Prealbumin, Transferrin und HE4 in einer biologischen Probe, die von dem Subjekt stammt; und
(b) Vergleichen des Niveaus der Marker mit einem Referenzniveau.

3. In vitro Verfahren zum prä-operativen Bestimmen des Risikos eines Subjekts mit einer adnexalen Masse, an Eierstockkrebs erkrankt zu sein, wobei das Verfahren umfasst
(a) Erfassen des Niveaus eines Panels von Markern bestehend aus CA125, Prealbumin, Transferrin und HE4 in einer biologischen Probe, die von dem Subjekt stammt; und
(b) Vergleichen des Niveaus der Marker mit einem Referenzniveau.

4. Verfahren nach Anspruch 2 oder 3, wobei:
(i) das Verfahren ferner ein Bestimmen von klinischen Markern für Eierstockkrebsrisiko beinhaltet, umfassend Alter, prä-menopausalen Status, post-menopausalen Status, Familiengeschichte, physische Untersuchung, Bildauswertungen und Raucherhistorie;
(ii) das Vergleichen die Verwendung eines linearen Modells oder eines nicht-linearen Modells umfasst;
(iii) das Verfahren ferner ein Erfassen des Follikel-stimulierenden Hormons (FSH) umfasst; und/oder
(iv) der Erfassungsschritt durch ein Immunassay oder eine Affinitätsbindung erfolgt.

5. Verfahren nach Anspruch 2 oder 3, wobei das Verfahren auf einer Platte, einem Chip, in Beads, einer mikrofluiden Plattform, einer Membran, einem planaren Mikroarray oder in einem Suspensionsarray durchgeführt wird.

## Revendications

1. Utilisation *in vitro* d'un panel pour évaluer, avant opération, le risque qu'un sujet présentant une masse annexielle ait un cancer des ovaires, lequel panel est constitué des marqueurs suivants : antigène tumoral 125 (CA125), préalbumine, transferrine, et protéine épididy-mienne humaine 4 (HE4).

2. Procédé *in vitro* visant à réduire le taux de faux positifs lors de l'évaluation préopératoire du cancer des ovaires chez un sujet présentant une masse annexielle, lequel procédé comporte :
a) le fait de mesurer les niveaux des marqueurs d'un panel constitué des CA125, préalbumine, transferrine et HE4 dans un échantillon biologique dérivé du sujet,
b) et le fait de comparer les niveaux de ces marqueurs à des niveaux de référence.

3. Procédé *in vitro* visant à évaluer, avant opération, le risque qu'un sujet présentant une masse annexielle ait un cancer des ovaires, lequel procédé comporte :
a) le fait de mesurer les niveaux des marqueurs d'un panel constitué des CA125, préalbumine, transferrine et HE4 dans un échantillon biologique dérivé du sujet,
b) et le fait de comparer les niveaux de ces marqueurs à des niveaux de référence.

4. Procédé conforme à la revendication 2 ou 3, étant entendu que :
i) le procédé comporte en outre le fait d'évaluer des marqueurs cliniques du risque de cancer des ovaires, y compris l'âge, le statut de préménopause, le statut de postménopause, l'historique familial, l'examen physique, les résultats d'imagerie et l'historique de tabagisme ;
ii) la comparaison comporte le recours à un modèle linéaire ou à un modèle non-linéaire ;
iii) le procédé implique en outre la détection de la folliculostimuline (FSH) ;
iv) et/ou l'étape de détection est réalisée par immuno-dosage ou par capture par affinité.

5. Procédé conforme à la revendication 2 ou 3, lequel procédé est mis en oeuvre sur une plaque, une puce, des perles, une plateforme microfluidique, une membrane, un microréseau plan, ou un réseau en suspension.
